(19) Europäisches Patentamt
European Patent Office
Office européen des brevets

(11) EP 4 455 130 A1

(12) # EUROPEAN PATENT APPLICATION
published in accordance with Art. 153(4) EPC

(43) Date of publication:
**30.10.2024 Bulletin 2024/44**

(21) Application number: **23742914.7**

(22) Date of filing: **18.01.2023**

(51) International Patent Classification (IPC):
***C07D 401/04*** (2006.01) ***C07D 401/12*** (2006.01)
***C07D 401/14*** (2006.01) ***C07D 471/00*** (2006.01)
***A61K 31/47*** (2006.01) ***A61K 31/498*** (2006.01)
***A61P 25/04*** (2006.01) ***A61P 25/06*** (2006.01)
***A61P 29/00*** (2006.01)

(52) Cooperative Patent Classification (CPC):
**A61K 31/47; A61K 31/4709; A61K 31/498; A61K 31/55; A61P 1/00; A61P 9/06; A61P 13/00; A61P 19/00; A61P 21/00; A61P 25/00; A61P 25/04; A61P 25/06; A61P 25/28; A61P 29/00; A61P 35/00;** (Cont.)

(86) International application number:
**PCT/CN2023/072791**

(87) International publication number:
**WO 2023/138599 (27.07.2023 Gazette 2023/30)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **18.01.2022 CN 202210055432**
**07.06.2022 CN 202210634203**

(71) Applicant: **Chengdu Kanghong Pharmaceutical Co., Ltd.**
**Chengdu, Sichuan 610036 (CN)**

(72) Inventors:
- **LIU, Ting**
**Chengdu, Sichuan 610036 (CN)**
- **FANG, Qun**
**Chengdu, Sichuan 610036 (CN)**
- **WU, Fan**
**Chengdu, Sichuan 610036 (CN)**
- **KE, Zunhong**
**Chengdu, Sichuan 610036 (CN)**

(74) Representative: **Huang, Liwei**
**Cäcilienstraße 12**
**40597 Düsseldorf (DE)**

(54) **AROMATIC FUSED RING NAV1.8 INHIBITOR, AND USE THEREOF**

(57) An aromatic fused ring compound which acts as a sodium channel blocker, and a use thereof. The aromatic fused ring compound has inhibitory activity on sodium ion channel Nav1.8, and may be used as a drug for a wide range of pain treatment.

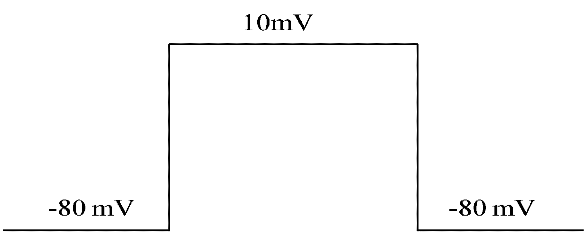


Fig. 1

EP 4 455 130 A1

(52) Cooperative Patent Classification (CPC): (Cont.)
**C07D 401/04; C07D 401/12; C07D 401/14;**
**C07D 403/04; C07D 471/00; C07D 471/04;**
**C07D 495/04; C07D 513/04**

## Description

### TECHNICAL FIELD

The present invention relates to a aromatic fused ring compound with inhibitory activity to the voltage-gated sodium channel 1.8 (Nav1.8) and their use.

### BACKGROUND

Pain is a complex physiological and psychological activity and one of the most common clinical symptoms. Pain is originally a protective mechanism of the human body to alert people to potential dangers, but abnormal pain can cause physiological disorders, especially chronic pain, which seriously affects people's quality of life. According to a report of 2019 (http://news.medlive.cn/anes/info-progress/show-153086_201.html), the global prevalence of chronic pain reaches 12-30%. In the United States, the number of patients with pain has exceeded that of diabetics, cardiac patients and cancer patients, and the economic loss caused by pain is up to 560-635 billion dollars every year (https://www.physio-pedia.com/Epidemiology_of_Pain); and according to the statistics from China in 2015, the pain market also reached 20.8 billion yuan (https://paindoctor.com/resources/chronic-pain-statisticsn/). At present, the commonly used analgesics such as opioid receptor agonists, cyclooxygenase inhibitors, GABA receptor agonists, etc., may have some side effects such as addiction, respiratory depression, gastrointestinal reaction, or cardiovascular events, central inhibition, etc., so the clinical needs are far from being met. Thus it can be seen that the pain market has great potential. (Nora D. Volkow, A. Thomas McLellan. Opioid Abuse in Chronic Pain-Misconceptions and Mitigation Strategies. N Engl J Med, 2016, 374(13): 1253-63. Sheng H G, Shao J Y, Kir Kland S C, et al. Inhibition of human colon Cancer cell growth by selective inhibition of cyclooxygenase-2. J Chm Invest, 1997, 99: 2254. Janette Brohan, Basavana G. Goudra. The Role of GABA Receptor Agonists in Anesthesia and Sedation. CNS Drugs, 2017.)

Pain can be classified into various types based on the stimulus properties, including mechanical pain, thermal pain, and chemical pain; according to the etiology of inflammation, it can be classified into inflammatory pain and non-inflammatory pain; according to the nerve location, it can be classified into central nerve pain, peripheral nerve pain, autonomic nerve pain; and according to the course of diseases, it can be classified into acute pain and chronic pain. No matter what kind of pain, it all needs the involvement of voltage-gated sodium channels (Navs).

Human algesthesia originates from the algesiroreceptors located at peripheral nerve endings throughout the body, which can convert mechanical, thermal, cold and chemical stimuli into nerve impulses. These impulses are transmitted to the dorsal root ganglia (DRG) via the afferent nerves and then to the nerve center through the efferent nerves, thus enabling the perception of pain (Bennett DL, Clark AJ, Huang J, et al. The Role of Voltage-Gated Sodium Channels in Pain Signaling. Physiol Rev, 2019, 99: 1079-1151.). Navs play the roles of triggering and transmitting signals during the transmission of nerve impulse signals, and are the main medium of the ascending branch of action potential (i.e., nerve impulse) (Mark D. Baker, John N.Wood. Involvement of Na+ channels in pain pathways. TRENDS in Pharmacological Sciences, 2001, 22(1): 27-31. Alan L Goldin. RESURGENCE OF SODIUM CHANNEL RESEARCH. Annu. Rev. Physiol. 2001. 63:871-894.). Therefore, inhibition of Navs helps in pain relief and treatment. However, the existing Navs inhibitors such as lidocaine, carbamazepine, lamotrigine, etc., have the defects of narrow treatment window and large side effects due to non-selectivity for Navs. Therefore, people have turned to the study of selective Nav inhibitors.

Navs are a class of transmembrane ion channel proteins composed of $\alpha$ subunits with molecular weight 260 kDa and $\beta$ subunits with molecular weight 30 ~ 40 kDa (Bennett DL, Clark AJ, Huang J, et al. The Role of Voltage-Gated Sodium Channels in Pain Signaling. Physiol Rev, 2019, 99: 1079-1151.) According to the inhibitory activity of tetrodotoxin (TTX), the subtypes of Navs can be divided into two categories: TTX-sensitive type (TTX-S), including Nav1.1, Nav1.2, Nav1.3, Nav1.4, Nav1.6 and Nav1.7; TTX-resistant type (TTX-R), including Nav1.5, Nav1.8, Nav1.9. According to the available physiological and pharmacological studies (Bennett DL, Clark AJ, Huang J, et al. The Role of Voltage-Gated Sodium Channels in Pain Signaling. Physiol Rev, 2019, 99: 1079-1151. Alan L Goldin. RESURGENCE OF SODIUM CHANNEL RESEARCH. Annu. Rev. Physiol. 2001. 63:871-94. Laura Sole, Michael M. Tamkun. Trafficking mechanisms underlying Nav channel subcellular localization in neurons. Channels, 2020, 14(1), 1-17. Manuel de Lera Ruiz, Richard L. Kraus. Voltage-Gated Sodium Channels: Structure, Function, Pharmacology and Clinical Indications. J. Med. Chem., 2015, 58(18): 7093-7118.), as shown in Table 1, Nav1.1, Nav1.2 and Nav1.3 are mainly distributed in the CNS region and are related to epilepsy, local anesthesia and other CNS diseases. Nav1.4 is mainly distributed in skeletal muscle, and its inhibitors are used as local anesthetics for myotonia. Nav1.5 is mainly distributed in cardiomyocytes, and its inhibitors are used to treat arrhythmias. Nav1.6 is involved in movement disorders; and currently, the main pain-related targets are Nav1.7, Nav1.8 and Nav1.9. Among them, the application in pain of Nav1.7 inhibitors is most widely studied, but there have been no successful clinical trials so far. There are few researches on Nav1.9, and its mechanism of action in pain is not very clear, and there are no pharmacodynamic models of pain for related inhibitors having been reported as evidences. Regarding the action mechanism of Nav1.8, Dib-Hajj et al., (Bennett DL, Clark AJ, Huang J, et al. The

Role of Voltage-Gated Sodium Channels in Pain Signaling. Physiol Rev, 2019, 99: 1079 -- 1151.) summarized existing researches in their 2019 review and concluded that Nav1.8 is the main contributor to the ascending branch of action potential. Its rapid initiation supports high-frequency discharge, and it has a high activation threshold, and a slow dynamic process. Blocking Nav1.8 can block the generation of action potential and the transmission of electrical signals. Blair and Bean's research (Blair NT, Bean BP.Roles of tetrodotoxin (TTX)-sensitive Na+ current, TTX-resistant Na+ current, and Ca2+ current in the action potentials of nociceptive sensory neurons. J Neurosci 2002, 22: 10277-10290.) suggests that although Nav1.8 and Nav1.9 are both expressed in DRG, Nav1.8's contribution to TTX-R current is the most significant. In the acute stage of nerve injury, Nav1.8 is down-regulated in the injured neurons, but up-regulated in the adjacent undamaged neurons, thus increasing spontaneous discharge. In chronic phase, crosstalk is formed between injured neurons and undamaged neurons, resulting in the upregulation of Nav1.8 in injured neurons, thus further increasing and maintaining the idiopathic discharge. In addition to mechanistic studies, the efficacy of Nav1.8 inhibitors in animal models of pain has also been validated: For example, A-803467 developed by Abbott, had a pain relief effect exceeding 50% in carrageenan model, complete Freund's adjuvant (CFA) model, sciatic nerve chronic compression pain (CCI) model, spinal nerve ligation pain (SNL) model and acute mechanical pain model, when compared with the model group ( Michael F. Jarvis, Prisca Honore, et al. A-803467, a potent and selective Nav1.8 sodium channel blocker, attenuates neuropathic and inflammatory pain in the rat. PNAS, 2007, 104(20): 8520-8525.); Moreover, A-803467 by systemic administration was superior to lidocaine in streptozotocin (STZ)-induced diabetic neuropathic heat pain model, and the two were equivalent when dosing by local injection of plantar, but the efficacy of A-803467 lasted longer (Mert, Gunes Y. Antinociceptive activities of lidocaine and the nav1.8 blocker a803467 in diabetic rats. J Am Assoc Lab Anim Sci. 2012; 51(5):579-585.); Pfizer's PF-01247324 also demonstrated significant pain relief on CFA and SNL models (Payne CE, Brown AR, Theile JW, et al. A novel selective and orally bioavailable Nav 1.8 channel blocker, PF-01247324, attenuates nociception and sensory neuron excitability. Br J Pharmacol. 2015; 172(10):2654-2670.). Most importantly, VX150, a highly selective Nav1.8 inhibitor developed by Vertex, has been clinically successful in three pain-related phase II trials. Taken together, Nav1.8 is a promising target for the treatment of pain or pain-related disorders.

Table 1. Overview of Navs subtypes

| Targets | Genes | Type | Major tissue distribution | Relationship to pain | Indications |
|---|---|---|---|---|---|
| Nav1.1 | SCN1A | TTX-s | CNS, PNS | / | Seizures, potential local anesthetics |
| Nav1.2 | SCN2A | TTX-s | CNS | / | Epilepsy |
| Nav1.3 | SCN3A | TTX-s | CNS(embryonic period), DRG (when injured) | Expressed rapidly when PNS damaged, overexcitating the damaged neurons | Epilepsy, local anesthesia |
| Nav1.4 | SCN4A | TTX-s | Skeletal muscle | / | Local anesthetics for myotonia |
| Nav1.5 | SCN5A | TTX-r | Cardiomyocytes | / | Arrhythmia |
| Nav1.6 | SCN8A | TTX-s | CNS, PNS | / | Dyskinesia |
| Nav1.7 | SCN9A | TTX-s | PNS | Multiple mutants, lower pain threshold | Hereditary erythromelalgia (>20 mutations), paroxysmal extreme pain disorder (>10 mutations), small fiber neuropathy, diabetic neuropathic pain |
| Nav1.9 | SCN11A | TTX-r | PNS | Lower pain threshold | Inflammatory pain, familial sporadic pain, erythromelalgia, megacolon disease |
| Nav1.8 | SCN10A | TTX-r | PNS | Lower pain threshold and discharge repeatedly | Neuropathic pain, inflammatory pain, postoperative pain (Vertex Phase II clinical) |

Currenttly, not many companies are reported to be developing Nav1.8 inhibitors. The main foreign companies are Abbott, Pfizer (WO2013114250A1), Gilead (AU2015224425A1), Sumitomo (WO2015008861A1), AbbVie

(WO2016149169A1), Raqualia (WO2020138271A1), Merck Sharp & Dohme (WO2020092187A1), Lieber (WO2020014243A1) and Vertex (WO2019014352A1). In China, there are mainly Hengrui (WO2020151728A1) and Shanghai Jiyu Pharmaceutical (CN111808019A). Among them, the specific inhibitory activity of Nav1.8 was not disclosed in their patents, or the activity was not satisfactory. Only Pfizer's PF-04531083 and Vertex's VX-150 and VX-548 entered phase II clinical trials, and the development of PF-04531083 was terminated because it did not show better activity than placebo in postoperative toothache. VX-150 was clinically successful in three phase II clinical trials, namely inflammatory pain, postoperative acute pain and neuropathic pain, providing preliminary validation of the role of Nav1.8 in pain . However, according to Vertex's official report, the development of VX150 has been terminated due to its unsatisfactory pharmacokinetics (PK). VX-548 is currently undergoing phase III clinical trials. Therefore, the development of new Nav1.8 inhibitors remains promising and necessary.

## SUMMARY

The object of the present invention is to provide aromatic fused ring compounds with inhibitory activity to the voltage-gated sodium channels, especially Nav1.8, and their use.

To achieve the above objects, the present invention provides a compound of formula I, or a solvate, a tautomer or a pharmaceutically acceptable salt thereof:

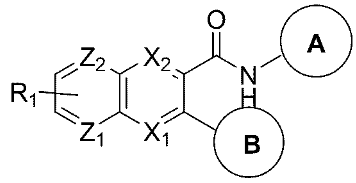

Formula I

wherein $X_1$, $X_2$, $Z_1$, $Z_2$ are independently selected from substituted or unsubstituted C or N;

$R_1$ is independently selected from hydrogen, halogen, $-NH_2$, -CN, OH, $-C_1$-$C_6$ alkyl, $-C_1$-$C_6$ haloalkyl, $-C_1$-$C_6$ alkoxy, $-C_1$-$C_6$ haloalkoxy, $-C_1$-$C_6$ alkylamino, $-SO_2R_2$, $-S(O)(NH)R_2$, $-COR_2$, $-CONR_2R_3$;

wherein $R_2$ and $R_3$ are independently selected from hydrogen, $-NH_2$, and $-C_1$-$C_3$ alkyl;

ring A is substituted or unsubstituted benzene ring or six -membered aromatic heteroaryl;

ring B is the substituted or unsubstituted three- to ten- membered aliphatic ring or aliphatic heterocyclic ring.

In some embodiments, at least one of $X_1$ and $X_2$ is N.

In some embodiments, $X_1$ and $X_2$ are both N.

In some embodiments, $X_1$ is N.

In some embodiments, $X_1$ and $X_2$ are simultaneously N, and $Z_1$ and $Z_2$ are C. In some embodiments, $X_1$ is N, and $X_2$, $Z_1$, $Z_2$ are C.

In some embodiments, $R_1$ is selected from H, halogen. Preferably, $R_1$ is H.

In some embodiments, $R_1$ is H.

In some embodiments, ring A is selected from a six-membered aryl or heteroaryl containing 0-3 nitrogen atoms, wherein the aryl or heteroaryl is optionally substituted by hydrogen, halogen, $-NH_2$, -CN, -OH, $-C_1$-$C_6$ alkyl, carbonyl, $-C_1$-$C_6$ haloalkyl, $-C_1$-$C_6$ alkoxy, $-C_1$-$C_6$ haloalkoxy, $-C_1$-$C_6$ alkylamino, $-SO_2R_2$, $-S(O)(NH)R_2$, $-COR_2$, $-CONR_2R_3$ or $-POR_2R_3$; where $R_2$ and $R_3$ are independently selected from hydrogen, $-NH_2$, $-NHCH_3$ and $-C_1$-$C_3$ alkyl.

In some embodiments, ring A is

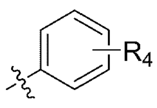

,

wherein $R_4$ is hydrogen, halogen, $-NH_2$, -CN, -OH, $-C_1$-$C_6$ alkyl, $-C_1$-$C_6$ haloalkyl, $-C_1$-$C_6$ alkoxy, $-C_1$-$C_6$ haloalkoxy,

$-C_1-C_6$ alkylamino, $-SO_2R_2$, $-S(O)(NH)R_2$, $-COR_2$, $-CONR_2R_3$, or $-POR_2R_3$; $R_2$ and $R_3$ are selected independently from hydrogen, $-NH_2$, $-NHCH_3$, $-C_1-C_3$ alkyl, or $R_2$ and $R_3$ together with phosphorus to form a three- to eight-membered ring.

In some embodiments, ring A is

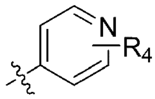

,

wherein $R_4$ is hydrogen, halogen, $-NH_2$, -CN, -OH, $-C_1-C_6$ alkyl, $-C_1-C_6$ haloalkyl, $-C_1-C_6$ alkoxy, $-C_1-C_6$ haloalkoxy, $-C_1-C_6$ alkylamino, $-SO_2R_2$, $-S(O)(NH)R_2$, $-COR_2$, $-CONR_2R_3$, or $-POR_2R_3$; $R_2$ and $R_3$ are selected independently from hydrogen, $-NH_2$, $-NHCH_3$, $-C_1-C_3$ alkyl, or $R_2$ and $R_3$ together with phosphorus to form a three- to eight-membered ring.

In some embodiments, ring A is

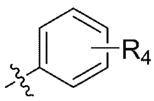

,

wherein $R_4$ is hydrogen, halogen, $-SO_2R_2$, $-S(O)(NH)R_2$, $-COR_2$, $-CONR_2R_3$; wherein $R_2$ and $R_3$ are independently selected from hydrogen, $-NH_2$, $-NHCH_3$, $-C_1-C_3$ alkyl.

In some embodiments, the ring A is

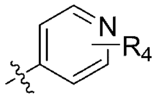

,

wherein $R_4$ is hydrogen, halogen, $-SO_2R_2$, $-S(O)(NH)R_2$, $-COR_2$, $-CONR_2R_3$; wherein $R_2$ and $R_3$ are independently selected from hydrogen, $-NH_2$, $-NHCH_3$, $-C_1-C_3$ alkyl.

In some embodiments, ring A is selected from:

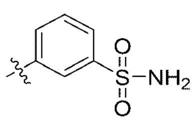 , 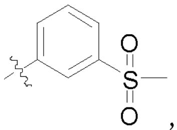 , 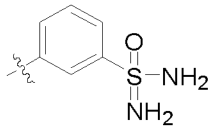 , 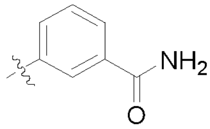 , 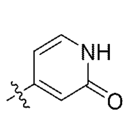 , 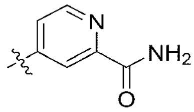

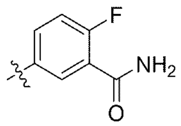 , 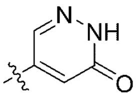 , 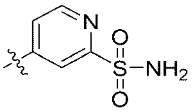 , 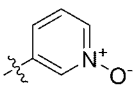 , 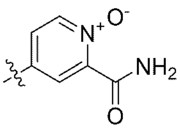 .

In some embodiments, ring B is selected from a three- to ten-membered aliphatic ring or aliphatic heterocyclic ring containing 0-3 heteroatoms which are selected from N, O, and S, and optionally, the aliphatic ring or aliphatic heterocyclic ring is substituted by a halogen, carbonyl group, $-NH_2$, -CN, -OH, $-C_1-C_6$ alkyl, $-C_1-C_6$ haloalkyl, $-C_1-C_6$ alkoxy, $-C_1-C_6$ haloalkoxy, $-C_1-C_6$ alkylamino, $-C_3-C_6$ cycloalkyl and $-C_3-C_6$ cycloheteroalkyl.

In some embodiments, ring B is

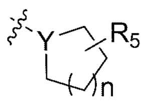

,

wherein Y is selected from CH or N; Ring B is substituted by m $R_5$, wherein $R_5$ is hydrogen, halogen, $-NH_2$, -CN, -OH, $-C_1-C_6$ alkyl, $-C_1-C_6$ haloalkyl, $-C_1-C_6$ alkoxy, $-C_1-C_6$ haloalkoxy, $-C_1-C_6$ alkylamino; m is an integer of 0-2 (n+3) and n is an integer of 0-6.

In some embodiments, n is an integer from 1 to 4.

In some embodiments, Y is N; n is 3; $R_5$ is hydrogen or halogen.

In some embodiments, ring B is:

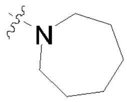 , 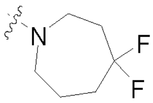 or 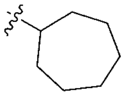 .

In some embodiments, the compounds of the present invention are described as Formula II:

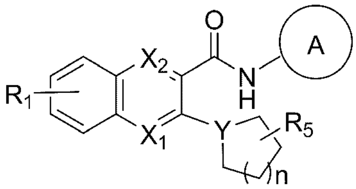

Formula II

wherein $X_1$, $X_2$ are independently selected from C or N, and at least $X_1$ is N;

$R_1$ is independently selected from hydrogen, halogen, $-NH_2$, -CN, -OH, $-C_1$-$C_6$ alkyl, $-C_1$-$C_6$ haloalkyl, $-C_1$-$C_6$ alkoxy, $-C_1$-$C_6$ haloalkoxy, $-C_1$-$C_6$ alkylamino, $-SO_2R_2$, $-S(O)(NH)R_2$, $-COR_2$, $-CONR_2R_3$; wherein $R_2$ and $R_3$ are independently selected from hydrogen, $-NH_2$, and $-C_1$-$C_3$ alkyl;

Y is selected from CH or N;

$R_5$ is selected from hydrogen, halogen, $-NH_2$, -CN, -OH, $-C_1$-$C_6$ alkyl, $-C_1$-$C_6$ haloalkyl, $-C_1$-$C_6$ alkoxy, $-C_1$-$C_6$ haloalkoxy, $-C_1$-$C_6$ alkylamino;

n is an integer from 0 to 6;

ring A is selected from a six-membered aryl or heteroaryl containing 0-3 nitrogen atoms, wherein the aryl or heteroaryl is optionally substituted by hydrogen, halogen, $-NH_2$, -CN, -OH, $-C_1$-$C_6$ alkyl, carbonyl, $-C_1$-$C_6$ haloalkyl, $-C_1$-$C_6$ alkoxy, $-C_1$-$C_6$ haloalkoxy, $-C_1$-$C_6$ alkylamino, $-SO_2R_2$, $-S(O)(NH)R_2$, $-COR_2$, $-CONR_2R_3$ or $-POR_2R_3$; wherein $R_2$ and $R_3$ are independently selected from hydrogen, $-NH_2$, $-NHCH_3$, and $-C_1$-$C_3$ alkyl.

In some preferred embodiments, in compound of Formula II of the present invention, ring A is

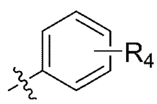

or

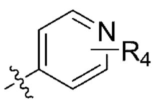

;

wherein $R_4$ is hydrogen, halogen, $-NH_2$, -CN, -OH, $-C_1$-$C_6$ haloalkyl, $-C_1$-$C_6$ alkoxy, $-C_1$-$C_6$ haloalkoxy, $-C_1$-$C_6$ alkylamino, $-SO_2R_2$, $-S(O)(NH)R_2$, $-COR_2$, $-CONR_2R_3$, or $-POR_2R_3$; $R_2$ and $R_3$ are selected independently from hydrogen, $-NH_2$, $-NHCH_3$, $-C_1$-$C_3$ alkyl, or $R_2$ and $R_3$ together with phosphorus to form a three- to eight-membered ring.

In some embodiments, the compounds of the present invention are shown as Formula III:

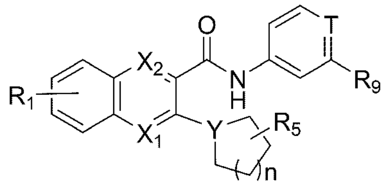

Formula III

wherein $X_1$ and $X_2$ are independently selected from C or N, and at least $X_1$ is N;

$R_1$ is independently selected from hydrogen, halogen, $-NH_2$, -CN, -OH, $-C_1-C_6$ alkyl, $-C_1-C_6$ haloalkyl, $-C_1-C_6$ alkoxy, $-C_1-C_6$ haloalkoxy, $-C_1-C_6$ alkylamino, $-SO_2R_2$, $-S(O)(NH)R_2$, $-COR_2$, $-CONR_2R_3$; wherein $R_2$ and $R_3$ are independently selected from hydrogen, $-NH_2$, and $-C_1-C_3$ alkyl;

T is $CR_6$ or N;

$R_6$ is hydrogen, halogen or $-C_1-C_6$ alkyl;

$R_9$ is hydrogen, halogen, $-NH_2$, -CN, -OH, $-C_1-C_6$ alkyl, $-C_1-C_6$ haloalkyl, $-C_1-C_6$ alkoxy, $-C_1-C_6$ haloalkoxy, $-C_1-C_6$ alkylamino, $-SO_2R_2$, $-S(O)(NH)R_2$, $-COR_2$, $-CONR_2R_3$, or $-POR_2R_3$;

$R_2$ and $R_3$ are independently selected from hydrogen, $-NH_2$, $-NHCH_3$, $-C_1-C_3$ alkyl, or $R_2$ and $R_3$ together with phosphorus to form a three- to eight-membered ring;

$R_5$ is selected from hydrogen, halogen, $-NH_2$, -CN, -OH, $-C_1-C_6$ alkyl, $-C_1-C_6$ haloalkyl, $-C_1-C_6$ alkoxy, $-C_1-C_6$ haloalkoxy, $-C_1-C_6$ alkylamino;

n is an integer from 0 to 6.

In some preferred embodiments, in compound of Formula III of the present invention, Y is N and n is an integer of 1-4.

In some preferred embodiments, in compound of Formula III of the present invention, n is 3.

In some embodiments, the compounds of the present invention are described as formula IV:

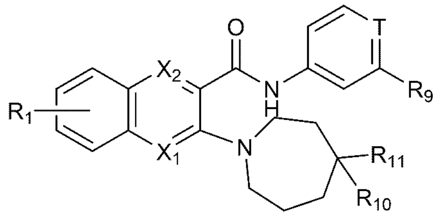

Formula IV

wherein $R_{10}$ and $R_{11}$ are independently selected from hydrogen or halogen;

other substituents are defined as described above.

In some preferred embodiments, in compound of Formula IV of the present invention, $R_1$ is independently selected from hydrogen, halogen, $-C_1-C_6$ alkyl, $-C_1-C_6$ haloalkyl, $-C_1-C_6$ alkoxy, $-C_1-C_6$ haloalkoxy.

In some preferred embodiments, in compound of Formula IV of the present invention, $R_1$ is independently selected from hydrogen, halogen, $-CH_3$ or $-OCH_3$.

In some preferred embodiments, in compound of Formula IV of the present invention, $R_1$ is selected from hydrogen or halogen.

In some preferred embodiments, in compound of Formula IV of the present invention, $X_1$ is N and $X_2$ is C.

In some preferred embodiments, in compound of Formula IV of the present invention, $X_1$ is N and $X_2$ is N.

In some preferred embodiments, in compound of Formula IV of the present invention, $R_6$ is hydrogen or halogen.

In some preferred embodiments, in compound of Formula IV of the invention, $R_9$ is $-SO_2R_2$, $-S(O)(NH)R_2$, $-COR_2$ or $-CONR_2R_3$, wherein $R_2$ and $R_3$ are independently selected from hydrogen, $-NH_2$, $-NHCH_3$, and $-C_1$-$C_3$ alkyl.

In some embodiments, the compounds of the present invention are shown as formula V:

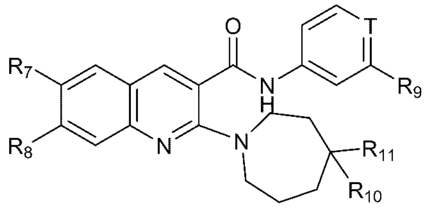

Formula V

wherein $R_7$, $R_8$ are independently selected from hydrogen, halogen, $-CH_3$, $-OCH_3$;

$R_9$ is selected from $-SO_2R_2$, $-S(O)(NH)R_2$, $-COR_2$, $-CONR_2R_3$;

$R_2$ and $R_3$ are independently selected from hydrogen, $-NH_2$, $-NHCH_3$, $-C_1$-$C_3$ alkyl;

T is $CR_6$ or N;

$R_{10}$ and $R_{11}$ are independently selected from hydrogen or halogen.

In some preferred embodiments, in compound of Formula V of the present invention, $R_9$ is selected from $-SO_2NH_2$, $-CONH_2$.

In some preferred embodiments, in compound of Formula V of the present invention, $R_9$ is $-SO_2NH_2$.

In some preferred embodiments, in compound of Formula V of the present invention, $R_7$ and $R_8$ are independently selected from hydrogen or halogen.

In some preferred embodiments, in compound of Formula V of the present invention, $R_{10}$ and $R_{11}$ are both halogen.

In some embodiments, the compounds of the present invention having the following general structure VI:

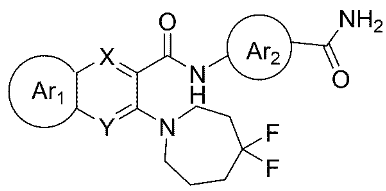

Formula VI

wherein $Ar_1$ is a substituted or unsubstituted five- or six-membered aryl or heteroaryl;

$Ar_2$ is a substituted or unsubstituted six-membered aryl or benzene ring;

X and Y are CorN.

In some preferred embodiments, the $Ar_1$ is selected from a five or six-membered aryl or heteroaryl containing 0-3 N, O, S atoms, and the aryl or heteroaryl is optionally substituted by hydrogen, halogen, $-NH_2$, -CN, -OH, $-C_1$-$C_6$ alkyl, carbonyl, $-C_1$-$C_6$ haloalkyl, $-C_1$-$C_6$ alkoxy, $-C_1$-$C_6$ haloalkoxy, $-C_1$-$C_6$ alkylamino, $-SO_2R_2$, $-S(O)(NH)R_2$, $-COR_2$, $-CONR_2R_3$, or $-POR_2R_3$; wherein $R_2$ and $R_3$ are independently selected from hydrogen, $-NH_2$, $-NHCH_3$, and $-C_1$-$C_3$ alkyl.

In some preferred embodiments, the $Ar_2$ is selected from a six-membered aryl containing 0-3 N atoms or benzene ring, and the aryl or benzene ring is optionally substituted by halogen, $-NH_2$, -CN, -OH, $-C_1$-$C_6$ alkyl, carbonyl, $-C_1$-$C_6$ haloalkyl, $-C_1$-$C_6$ alkoxy, $-C_1$-$C_6$ haloalkoxy, $-C_1$-$C_6$ alkylamino.

In some embodiments, the compounds of the present invention have the following general structure VII:

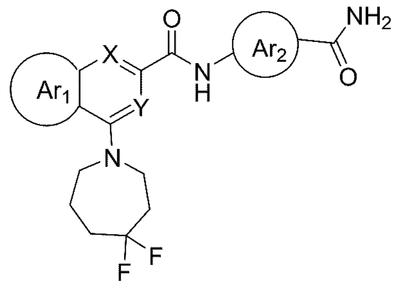

Formula VII

wherein $Ar_1$ is a substituted or unsubstituted five- or six-membered aryl or heteroaryl;

$Ar_2$ is a substituted or unsubstituted six-membered aryl or benzene ring;

X and Y are CorN.

In some preferred embodiments, the $Ar_1$ is selected from a five or six-membered aryl or heteroaryl containing 0-3 N, O, S atoms, and the aryl or heteroaryl is optionally substituted by hydrogen, halogen, $-NH_2$, -CN, -OH, $-C_1-C_6$ alkyl, carbonyl, $-C_1-C_6$ haloalkyl, $-C_1-C_6$ alkoxy, $-C_1-C_6$ haloalkoxy, $-C_1-C_6$ alkylamino, $-SO_2R_2$, $-S(O)(NH)R_2$, $-COR_2$, $-CONR_2R_3$, or $-POR_2R_3$; wherein $R_2$ and $R_3$ are independently selected from hydrogen, $-NH_2$, $-NHCH_3$, and -C $alkyl_{1-3}$.

In some preferred embodiments, the $Ar_2$ is selected from a six-membered aryl containing 0-3 N atoms or benzene ring, and the aryl or benzene ring is optionally substituted by halogen, $-NH_2$, -CN, -OH, $-C_1-C_6$ alkyl, carbonyl, $-C_1-C_6$ haloalkyl, $-C_1-C_6$ alkoxy, $-C_1-C_6$ haloalkoxy, $-C_1-C_6$ alkylamino.

In some embodiments, the compounds of the present invention have the following general structure VIII:

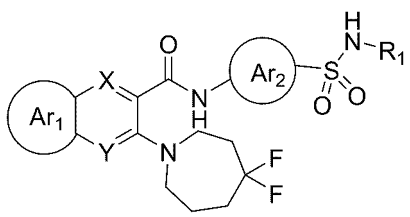

Formula VIII

wherein $Ar_1$ is a substituted or unsubstituted five- or six-membered aryl or heteroaryl;

$Ar_2$ is a substituted or unsubstituted six-membered aryl or benzene ring;

X and Y are C or N;

$R_1$ is an aliphatic chain, aliphatic ring or aryl.

In some preferred embodiments, the $Ar_1$ is selected from a five- or six-membered aryl or heteroaryl containing 0-3 atoms of N, O, S, and the aryl or heteroaryl is optionally substituted by hydrogen, halogen, $-NH_2$, -CN, -OH, $-C_1-C_6$ alkyl, carbonyl, $-C_1-C_6$ haloalkyl, $-C_1-C_6$ alkoxy, $-C_1-C_6$ haloalkoxy, $-C_1-C_6$ alkylamino .

In some preferred embodiments, the $Ar_2$ is selected from a six-membered aryl containing 0-3 N atoms or benzene ring, and the aryl or benzene ring is optionally substituted by halogen, $-NH_2$, -CN, -OH, $-C_1-C_6$ alkyl, carbonyl, $-C_1-C_6$ haloalkyl, $-C_1-C_6$ alkoxy, $-C_1-C_6$ haloalkoxy, $-C_1-C_6$ alkylamino.

In some preferred embodiments, the $R_1$ is hydrogen, halogen, $-NH_2$, -CN, -OH, $-C_1-C_6$ alkyl, carbonyl, $-C_1-C_6$ haloalkyl, $-C_1-C_6$ alkoxy, $-C_1-C_6$ haloalkoxy or $-C_1-C_6$ alkylamino.

In some embodiments, the compounds of the present invention have the following general structure IX:

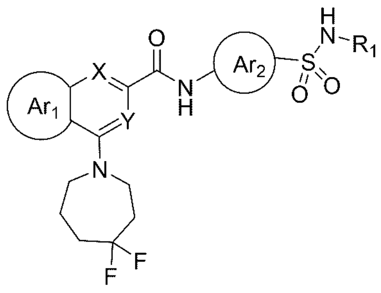

Formula IX

wherein $Ar_1$ is a substituted or unsubstituted five- or six-membered aryl or heteroaryl;

$Ar_2$ is a substituted or unsubstituted six-membered aryl or benzene ring;

X and Y are C or N;

$R_1$ is an aliphatic chain, aliphatic ring or aryl.

In some preferred embodiments, the $Ar_1$ is selected from a five- or six-membered aryl or heteroaryl containing 0-3 atoms of N, O, S, and the aryl or heteroaryl is optionally substituted by hydrogen, halogen, $-NH_2$, -CN, -OH, $-C_1-C_6$ alkyl, carbonyl, $-C_1-C_6$ haloalkyl, $-C_1-C_6$ alkoxy, $-C_1-C_6$ haloalkoxy, $-C_1-C_6$ alkylamino.

In some preferred embodiments, the $Ar_2$ is selected from a six-membered aryl containing 0-3 N atoms or benzene ring, and the aryl or benzene ring is optionally substituted by halogen, $-NH_2$, -CN, -OH, $-C_1-C_6$ alkyl, carbonyl, $-C_1-C_6$ haloalkyl, $-C_1-C_6$ alkoxy, $-C_1-C_6$ haloalkoxy, $-C_1-C_6$ alkylamino.

In some preferred embodiments, the $R_1$ is hydrogen, halogen, $-NH_2$, -CN, -OH, $-C_1-C_6$ alkyl, $-C_1-C_6$ haloalkyl, $-C_1-C_6$ alkoxy, $-C_1-C_6$ haloalkoxy or $-C_1-C_6$ alkylamino.

In some embodiments, the compounds of the present invention have the following general structure X:

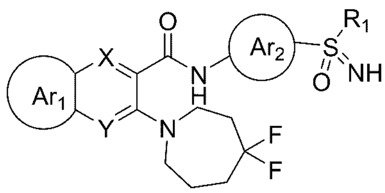

Formula X

wherein $Ar_1$ is a substituted or unsubstituted five- or six-membered aryl or heteroaryl;

$Ar_2$ is a substituted or unsubstituted six-membered aryl or benzene ring;

X and Y are CorN;

$R_1$ is an aliphatic chain, aliphatic ring or aryl.

In some preferred embodiments, the $Ar_1$ is selected from a five - or six-membered aryl or heteroaryl containing 0-3 atoms of N, O, S, and the aryl or heteroaryl is optionally substituted by hydrogen, halogen, $-NH_2$, -CN, -OH, $-C_1-C_6$ alkyl, carbonyl, $-C_1-C_6$ haloalkyl, $-C_1-C_6$ alkoxy, $-C_1-C_6$ haloalkoxy, $-C_1-C_6$ alkylamino.

In some preferred embodiments, the $Ar_2$ is selected from a six-membered aryl containing 0-3 N atoms or benzene ring, and the aryl or benzene ring is optionally substituted by halogen, $-NH_2$, -CN, -OH, $-C_1-C_6$ alkyl, carbonyl, $-C_1-C_6$ haloalkyl, $-C_1-C_6$ alkoxy, $-C_1-C_6$ haloalkoxy, $-C_1-C_6$ alkylamino.

In some preferred embodiments, the $R_1$ is hydrogen, $-NH_2$, $-C_1-C_6$ alkyl, $-C_1-C_6$ haloalkyl, $-C_1-C_6$ alkoxy, $-C_1-C_6$ haloalkoxy or $-C_1-C_6$ alkylamino.

In some embodiments, the compounds of the present invention have the following general structure XI:

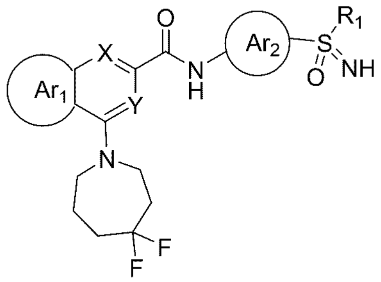

Formula XI

wherein $Ar_1$ is a substituted or unsubstituted five- or six-membered aryl or heteroaryl;

$Ar_2$ is a substituted or unsubstituted six-membered aryl or benzene ring;

X and Y are C or N;

$R_1$ is an aliphatic chain, aliphatic ring or aryl.

In some preferred embodiments, the $Ar_1$ is selected from a five- or six-membered aryl or heteroaryl containing 0-3 atoms of N, O, S, and the aryl or heteroaryl is optionally substituted by hydrogen, halogen, $-NH_2$, -CN, -OH, $-C_1-C_6$ alkyl, carbonyl, $-C_1-C_6$ haloalkyl, $-C_1-C_6$ alkoxy, $-C_1-C_6$ haloalkoxy, $-C_1-C_6$ alkylamino.

In some preferred embodiments, the $Ar_2$ is selected from a six-membered aryl containing 0-3 N atoms or benzene ring, and the aryl or benzene ring is optionally substituted by halogen, $-NH_2$, -CN, -OH, $-C_1-C_6$ alkyl, carbonyl, $-C_1-C_6$ haloalkyl, $-C_1-C_6$ alkoxy, $-C_1-C_6$ haloalkoxy, $-C_1-C_6$ alkylamino.

In some preferred embodiments, the $R_1$ is hydrogen, $-NH_2$, $-C_1-C_6$ alkyl, $-C_1-C_6$ haloalkyl, $-C_1-C_6$ alkoxy, $-C_1-C_6$ haloalkoxy or $-C_1-C_6$ alkylamino.

In some embodiments, the compounds of the present invention are selected from:

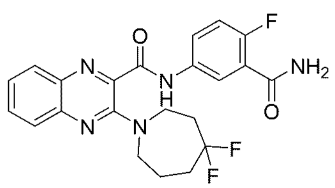
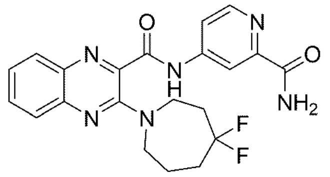
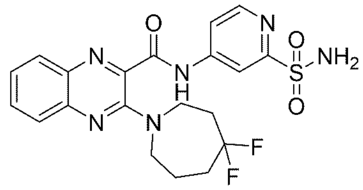

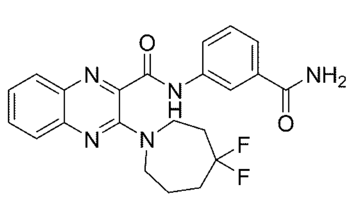
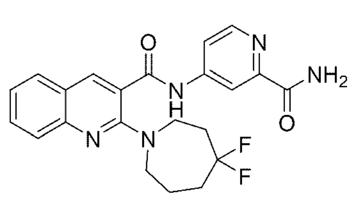
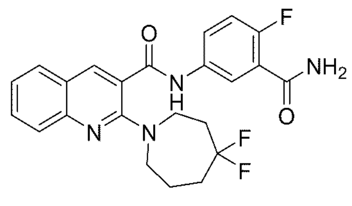

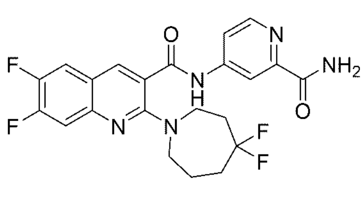
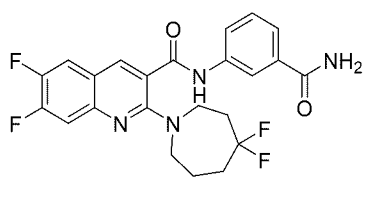
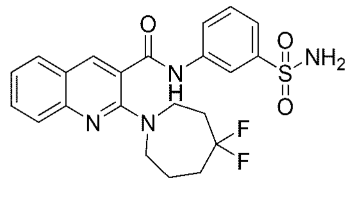

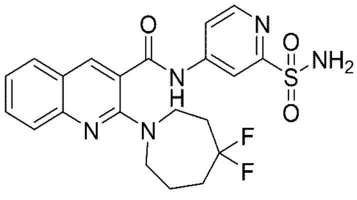
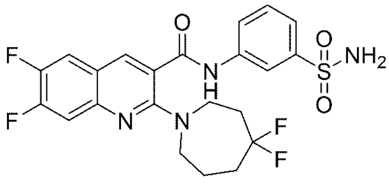
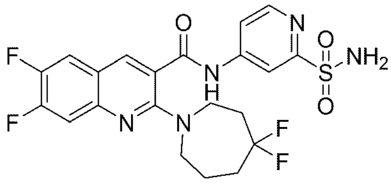
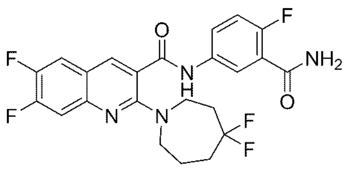
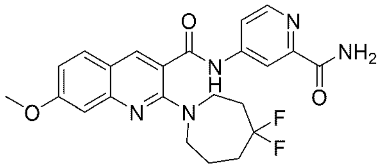
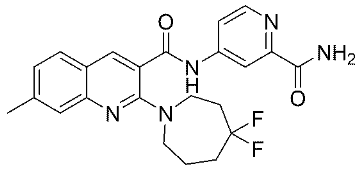
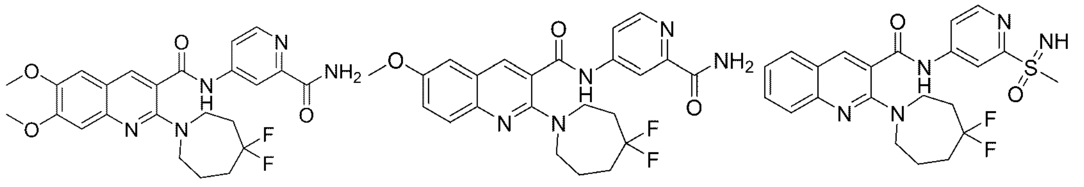
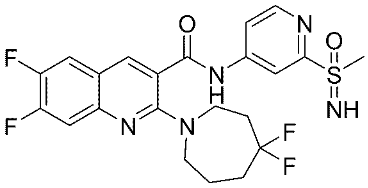
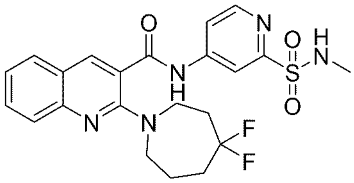
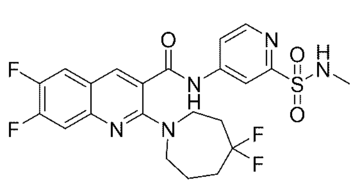
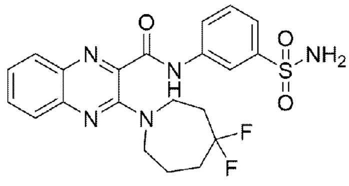
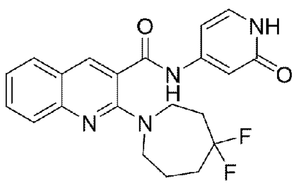
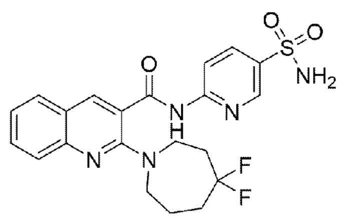
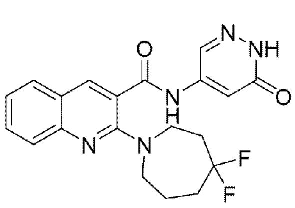
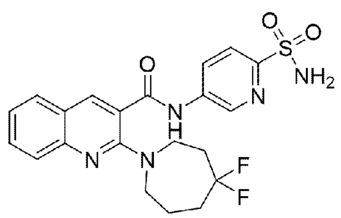
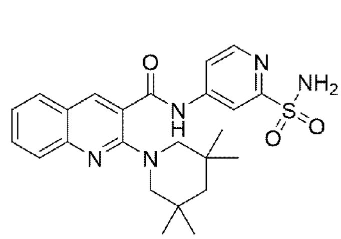
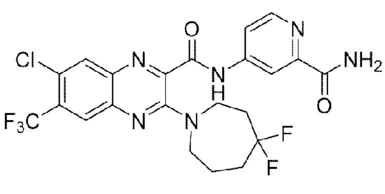
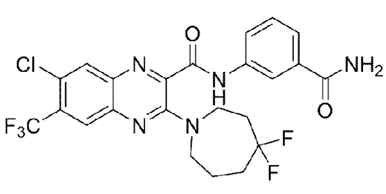
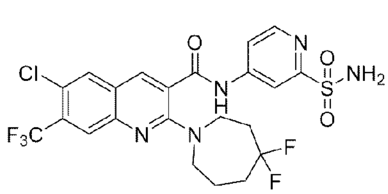

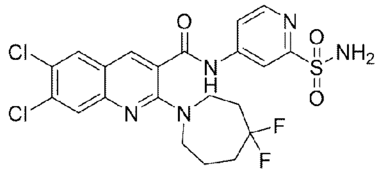
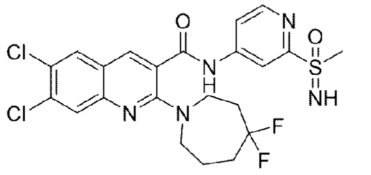
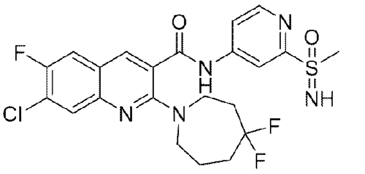

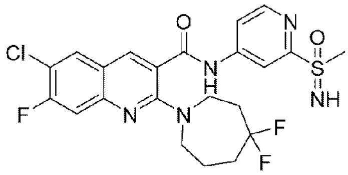
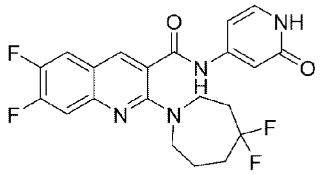
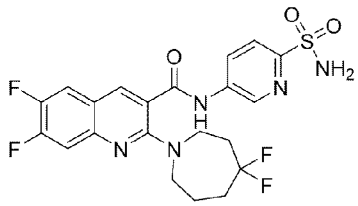

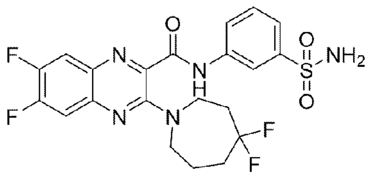
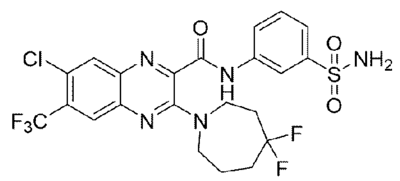
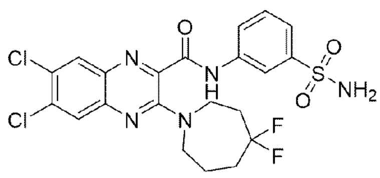

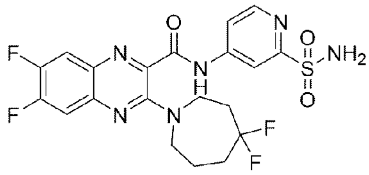
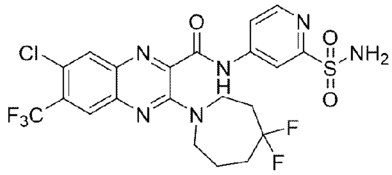
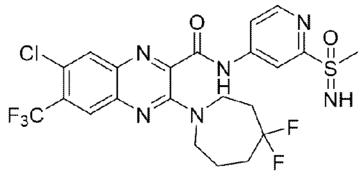

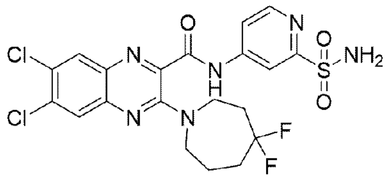
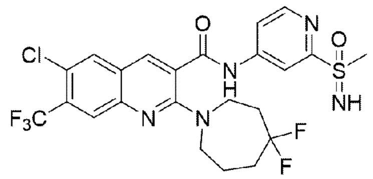
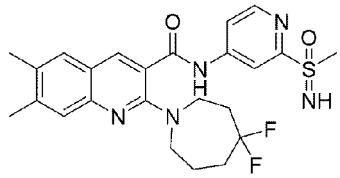

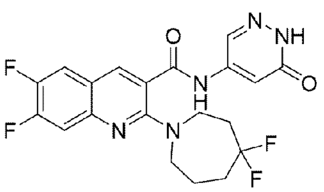
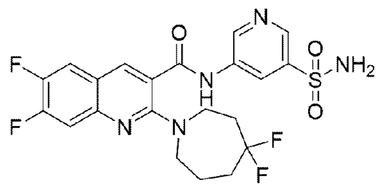
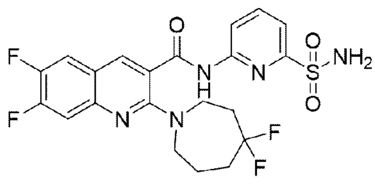

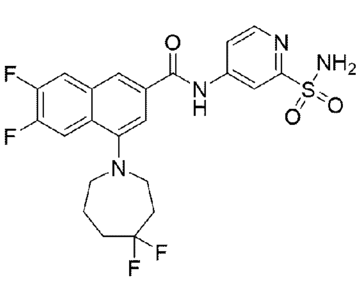
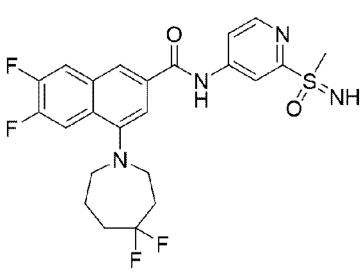
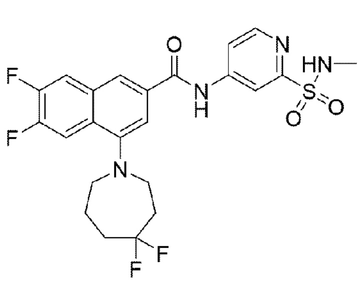

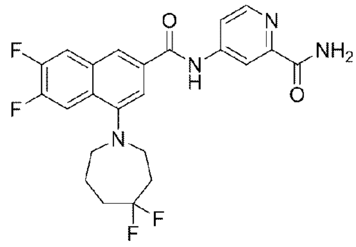
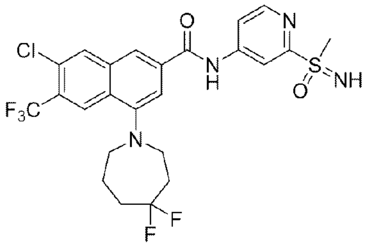
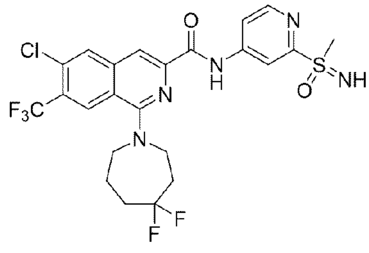

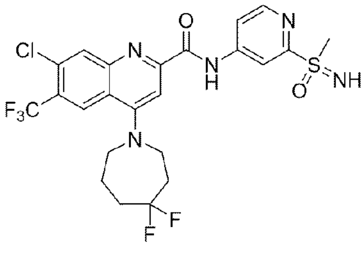
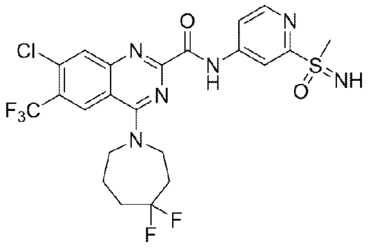
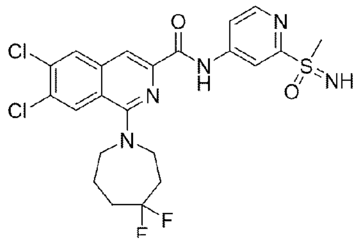

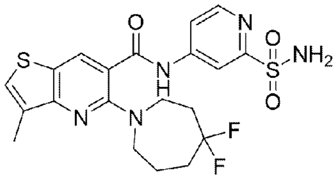
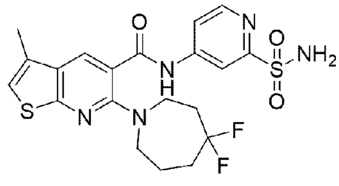
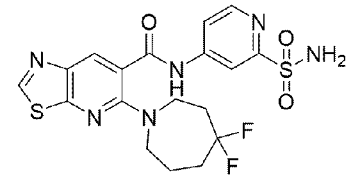

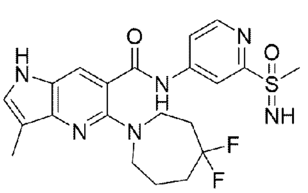
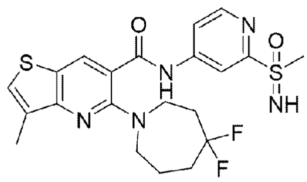
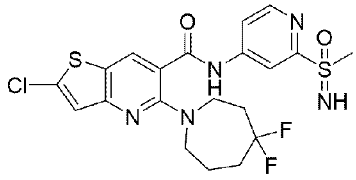

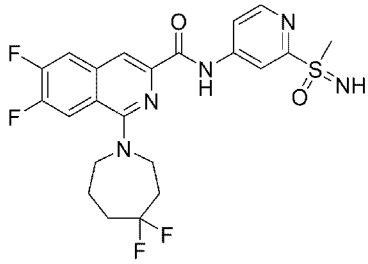
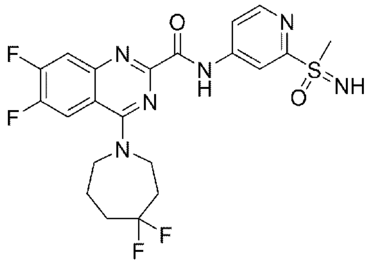
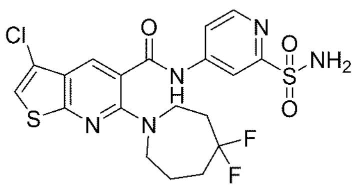.

In some preferred embodiments, the compounds described in the present invention are selected from:

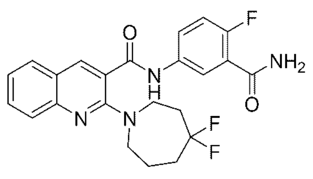
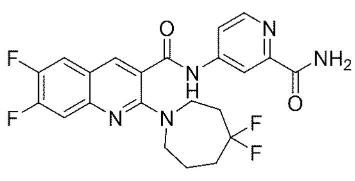
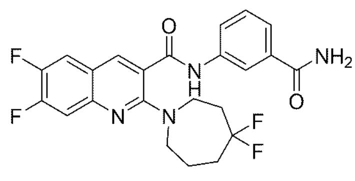

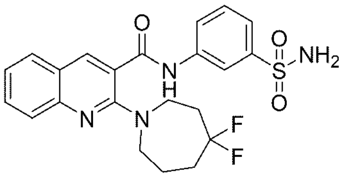 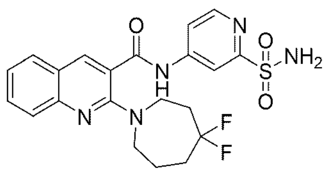 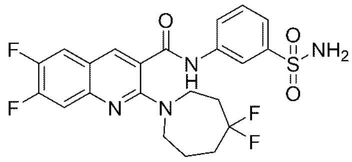

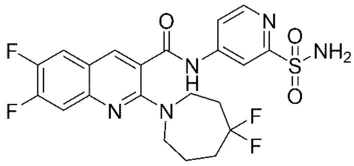 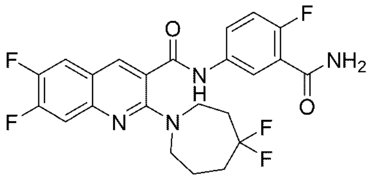 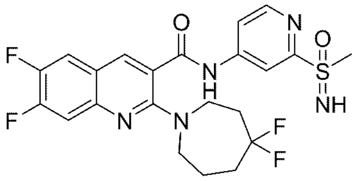

The second object of the present invention is to provide a pharmaceutical composition comprising the compound described in the first purpose of the present invention or a pharmaceutically acceptable salt thereof, and a pharmaceutically acceptable carrier.

The third object of the invention is to provide a use of the compound or the pharmaceutically acceptable salt in the preparation of a medicament for the treatment of pain.

The fourth object of the invention is to provide a method for preventing or treating pain, comprising the administration of the compound or the pharmaceutically acceptable salt of the first object of the invention to a patient.

In some embodiments, the pain is selected from chronic pain, enteric pain, neuropathic pain, musculoskeletal pain, acute pain, inflammatory pain, cancer pain, primary pain, post-operative pain, visceral pain, multiple sclerosis, Charcot-Marie-Tooth syndrome, incontinence, or arrhythmia.

## BRIEF DESCRIPTION OF THE DRAWINGS

FIG. 1 shows the experimental voltage variation in method 2 of Example 33, wherein -80mV is the clamping voltage, and Nav1.8 channels of the cell are in the resting state under this voltage; 10mV is the activation voltage, and Nav1.8 channels of the cell are in the activated state under this voltage.

FIG. 2 shows mechanical allodynia test results of Example 36.

## Definitions

As used herein, the term "alkyl" by itself or as part of another substituent refers to (unless otherwise indicated) a straight or branched alkyl group having a specified number of carbon atoms (i.e., $C_{1-8}$ means one to eight carbons). Examples of alkyl include methyl, ethyl, n-propyl, isopropyl, n-butyl, tert-butyl, isobutyl, sec-butyl, n-amyl, n-hexyl, n-heptyl, n-octyl, etc.

As used herein, the term "heteroaryl" refers to a group having 5 to 10 heterocyclic atoms, preferably five- or six-membered monocyclic aromatic heterocycle or eight- to ten-membered bicyclic aromatic heterocycle; and a group with one to three heteroatoms in addition to carbon. A "heteroatom" is nitrogen, oxygen, or sulfur.

The term "aliphatic ring" refers to monocyclic or polycyclic alkanes with saturated or unsaturated bonds, preferably saturated monocyclic alkanes, such as cyclopropane, cyclobutane, cyclopentane, cyclohexane, cycloheptane. Polycyclic alkanes can be divided into spirals and bridged rings, depending on how they are bound.

The term "aliphatic heterocycle" refers to monocyclic or polycyclic alkanes that have saturated or unsaturated bonds of one or more heteroatoms in addition to carbon atoms. Heteroatoms such as nitrogen, oxygen, or sulfur.

The terms "substituted", "substituted... by" means that any one or more hydrogen atoms bound to a particular atom are substituted by a substituent, which can include heavy hydrogen and variants of hydrogen, provided that the valence state of the particular atom is normal and the substituted compound is stable. The term "optionally substituted" means that a substituent may or may not be substituted, unless otherwise specified, the kind and number of substituents may be arbitrary on the basis of what is chemically achievable.

In addition, although the term "optionally substituted" does not appear, if any substitution occurs in a ring (such as an aliphatic ring or aryl), it should be understood that the position and number of substituents are arbitrary, as long as they are chemically achievable.

As used herein, the term "halogen" by itself or as part of another substituent means (unless otherwise stated)

an atom of fluorine, chlorine, bromine or iodine. In addition, the term "haloalkyl" is intended to include both monohaloalkyl and polyhaloalkyl groups. For example, the term "$C_{1-4}$ haloalkyl" is intended to include trifluoromethyl, 2,2,2-trifluoroethyl, 4-chlorobutyl, 3-bromopropyl, difluoromethyl, etc.

As used herein, "alkoxy" stands for the above alkyl groups having a specific number of carbon atoms connected by an oxygen bridge, and unless otherwise specified, -$C_{1-}$ $C_6$alkoxy groups include the alkoxy groups of $C_1$, $C_2$, $C_3$, $C_4$, $C_5$ and $C_6$. Examples of alkoxy groups include, but are not limited to: methoxy, ethoxy, n-propoxy, isopropoxy, n-butoxy, sec-butoxy, tert-butoxy, n-amoxy, and S-amoxy.

Unless otherwise specified, structures described herein are also meant to include all isomers (such as enantiomers, diastereoisomers, and geometric (or conformational) isomers) of the structure; Such as the R and S configurations of each asymmetric center, the (Z) and (E) double bond isomers, and the (Z) and (E) conformational isomers. Thus, single stereochemical isomers of these compounds and enantiomers, diastereoisomers, and geometric isomers (or conformational isomers) mixtures fall within the scope of the present invention. Unless otherwise specified, all tautomerism forms of the compounds of the invention fall within the scope of the invention. In addition, unless otherwise specified, the structures described herein are also meant to include compounds that differ only in the presence of one or more isotopically enriched atoms. For example, compounds with structures other than hydrogen being substituted by deuterium or tritium or carbon being substituted by $^{13}C$- or $^{14}C$-enriched carbon fall within the scope of the present invention.

Unless otherwise specified, a structure described herein is also meant to include a solvate of that structure, which refers to the physical association of the applied compound with one or more solvent molecules; The physical association involves various degrees of ionic and covalent bonding, including hydrogen bonding; In some cases, such as when one or more solvent molecules are introduced into the crystal lattice of a crystalline solid, the solvate will be able to be separated; "Solvate" covers both the solution phase and the separable solvate; Non-restrictive examples of suitable solvents include but are not limited to isopropyl alcohol, ethanol, methanol, DMSO, ethyl acetate, acetic acid and ethanolamine, etc.; "Hydrate" is a solvate in which the solvent molecule is $H_2O$.

As used herein, the term "composition" is intended to cover a product containing specified ingredients with specified quantity, and any product derived directly or indirectly from a combination of specified ingredients with specified quantity. The phrase "pharmaceutically acceptable" means that the carrier, diluent or excipient must be compatible with other ingredients in the formulation and not harmful to its recipients of the drug.

As used herein, the term "pharmaceutically acceptable salts" is intended to include salts that are prepared with active compounds from relatively non-toxic acids or bases. When the compounds of the present invention contain relatively acidic functional groups, alkali addition salts may be obtained by contacting the neutral form of such compounds with sufficient quantities of the desired base, either pure or in a suitable inert solvent. Examples of salts derived from pharmaceutically acceptable inorganic bases include aluminum, ammonium, calcium, copper, iron, ferrous, lithium, magnesium, manganese salts, manganous, potassium, sodium, zinc, etc. Salts derived from pharmaceutically acceptable organic bases include salts of primary, secondary, and tertiary amines (including substituted amines, cyclic amines, naturally occurring amines, etc.), Such as arginine, betaine, caffeine, choline, N,N'-dibenzylethylenediamine, diethylamine, 2-diethylaminoethanol, 2-dimethylaminoethanol, ethanolamine, ethylenediamine, N-ethylmorpholine, N-ethylpiperidine, glucosamine, histidine, isopropylamine, lysine, methylglucosamine, morpholine, piperazine, piperidine, polyamine resin, procaine, purine, theobromine, triethylamine, trimethylamine, tripropylamine, tromethamine, etc. When the compounds of the invention contain relatively basic functional groups, acid addition salts may be obtained by contacting the neutral form of such compounds with sufficient quantities of pure or desired acids in a suitable inert solvent. Pharmaceutically acceptable examples of acid addition salts include those derived from inorganic acids such as hydrochloric acid, hydrobromic acid, nitric acid, carbonic acid, monohydrocarbonic acid, phosphoric acid, monohydrophosphoric acid, dihydrophosphoric acid, sulfuric acid, monohydrosulfuric acid, hydroiodic acid or phosphite, etc., as well as salts derived from relatively non-toxic organic acids such as acetic acid, propionic acid, isobutyric acid, malonic acid, benzoic acid, succinic acid, suberic acid, fumaric acid, mandelic acid, phthalic acid, benzene sulfonic acid, p-toluenesulfonic acid, citric acid, tartaric acid, methanesulfonic acid, etc. Salts of amino acids such as arginine and organic acids such as glucuronic acid or galacturonic acid are also included.

The term "pharmaceutically acceptable carrier" means a representative carrier of any preparation or carrier medium with the capability of delivering an effective amount of the active substance of the present invention, which does not interfere with the biological activity of the active substance and has no toxic side effects to the host or patient, including water, oil, vegetable and mineral, paste base, lotion base, ointment base, etc. These substrates include suspension agents, viscosifiers, transdermal promoters, etc. Their formulations are well known to technicians in the field of cosmetics or topical drugs.

Compositions containing compounds of formula I are usually formulated according to standard pharmaceutical practice of pharmaceutical compositions. Typical preparations are prepared by mixing the compounds of the invention with diluents, carriers, or excipients. Preparations may also include buffering agents, stabilizers, surfactants, wetting agents, lubricants, emulsifiers, suspension agents, preservatives, antioxidants, opaque agents, glidants, processing

aids, colorants, sweeteners, aromatics, flavorings, thinners, and one or more of the other known additives.

The compounds of the present invention may be applied in any convenient use form, such as tablets, powders, capsules, solutions, dispersions, suspensions, syrups, sprays, suppositories, gels, emulsions, patches, etc. Such compositions may comprise conventional components of pharmaceutical preparations, such as diluents, carriers, pH regulators, sweeteners, fillers, and additional active agents.

The compounds of the present invention may be administered by any suitable means, including oral, topical (including buccal and sublingual), rectum, vagina, transdermal, parenteral, subcutaneous, intraperitoneal, pulmonary, intradermal, intrathecal and epidural, and intranasal, and intralesional if required for topical treatment. Parenteral infusion includes intramuscular, intravenous, intra-arterial, intraperitoneal, intracerebral, intraocular, intrafocal, or subcutaneous administration.

"Pain" in the present invention means the pain associated with abnormalities in the voltage-gated sodium channels, in particular, the voltage-gated sodium channel is Nav1.8. Pain of the present invention includes, but is not limited to, chronic pain, intestinal pain, neuropathic pain, musculoskeletal pain, acute pain, inflammatory pain, cancer pain, primary pain, post-operative pain, visceral pain, multiple sclerosis, Charcot-Marie-Tooth syndrome, incontinence, and arrhythmia.

Among them, intestinal pain such as inflammatory bowel disease pain, Crohn's disease pain or interstitial cystitis pain. Musculoskeletal pain such as osteoarthritis pain, back pain, cold pain, burning pain or toothache. Inflammatory pain such as rheumatoid arthritis pain or vulvodynia, idiopathic pain including fibromyalgia. Neuropathic pain such as postherpetic neuralgia, diabetic neuralgia, painful HIV-associated sensory neuropathy, trigeminal neuralgia, mouth burn syndrome, amputative pain, hallucinatory pain, painful neuroma; Traumatic neuroma; Morton neuroma; Nerve entrapment injury, spinal stenosis, carpal tunnel syndrome, radiculalgia, sciatica; Nerve avulsion injury, brachial plexus avulsion injury; Complex local pain syndrome, medication-induced neuralgia, cancer chemotherapy-induced neuralgia, antiretroviral therapy-induced neuralgia; Pain after spinal cord injury, idiopathic small fiber neuropathy, idiopathic sensory neuropathy or trigeminal.

The compound of the invention is named manually or by Chemdraw software, and the commercially available compound adopts the supplier catalog name.

Compared with the prior art, the invention has the following advantages:

provides a series of structurally novel aromatic fused ring compounds that have inhibitory activity against Nav1.8 and can be used as drugs for the treatment of a wide range of pain.

## DETAIL DESCRIPTION OF THE EMBODIMENTS

The invention is further elaborated in combination with specific examples. It should be understood that these examples are used only to illustrate the invention and not to limit the scope of the invention. Experimental methods for which specific conditions are not indicated in the following examples are usually in accordance with conventional conditions or those recommended by the manufacturer. The chemical reactions described in the examples (preparation) can be readily modified to prepare many other compounds of the present invention, and alternative methods for preparing compounds of the present invention are considered to be within the scope of the present invention. Unless otherwise defined, the terms used herein have the same meaning as those familiar to those skilled in the art. In addition, any method or material similar or equivalent to the described contents may be applied in the present invention.

The structure of the compound is determined by nuclear magnetic resonance (NMR) and/or mass spectrometry (MS). The NMR shift ($\delta$) is given in units of $10^{-6}$ (ppm). NMR was determined by a Bruker AVANCE III HD (400 MHz) or Bruker NEO (400 MHz) nuclear magnetometer with deuterated dimethyl sulphone (DMSO-$d_6$) or deuterated chloroform ($CDCl_3$) as solvent and tetramethylsilane (TMS) as internal standard. MS was determined by liquid-mass combination (LC-MS): Shimadzu's LC-20AD/LCMS-2020 or Agilent's Agilent 1260/6125. High performance liquid phase (HPLC) analysis was performed by Shimadzu's LC-20AD or LC-2030C, or Agilent's Agilent 1100/1200. Preparation and purification were performed by waters2767 or gilson GX281.

### Example 1

Preparation of 3-(3-(4,4-difluoroazepan-1-yl) quinoxaline-2-formamido-pyridine-1-oxide (KH01)

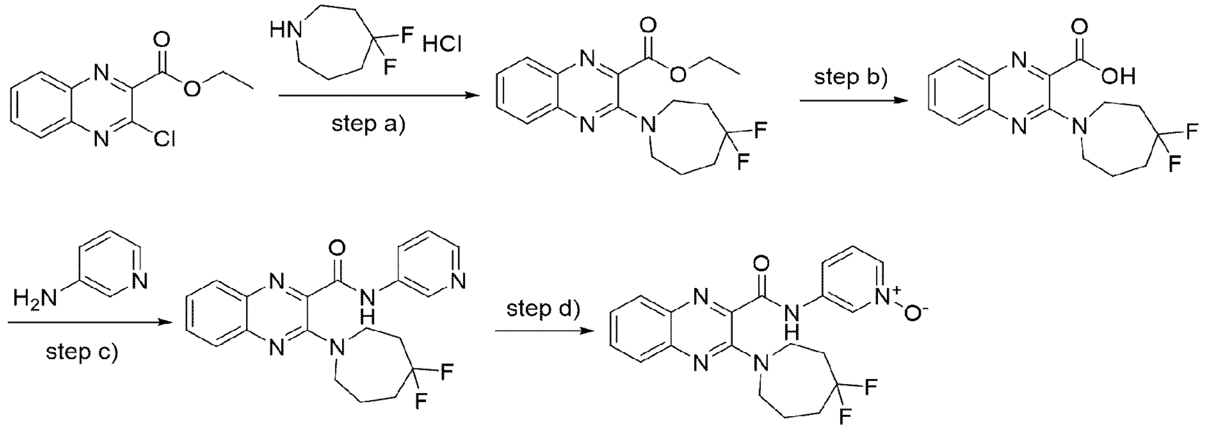


KH01

Step a): Preparation of ethyl 3-(4,4-difluoroazepan-1-yl) quinoxaline-2-carboxylate

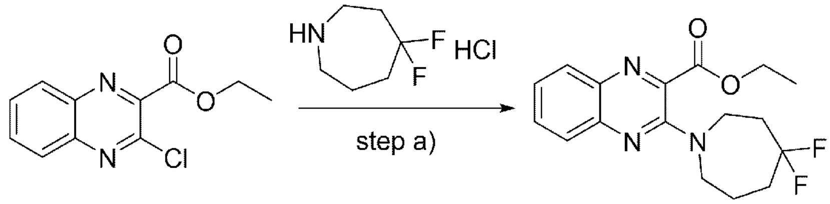


4,4-difluoroazepane hydrochloride (4.3 g, 25.4 mmol) and DIEA (8.2 g, 63.5 mmol) were added to the isopropyl alcohol (150 mL) solution of ethyl 3-quinoxaline-2-carboxylate (5g, 21.2 mmol) and the reaction mixture was stirred at 50 °C for 16 h. After the reaction was cooled to room temperature, ethyl acetate was added to dilute it, and water was added to separate the two phases, then the organic phase was washed twice with saturated brine, dried over anhydrous sodium sulfate, filtered and concentrated under reduced pressure. The resulting crude product was purified by silica gel chromatography to obtain light yellow solid ethyl 3-(4,4-difluoroazepan-1-yl) quinoxaline-2-carboxylate (6.2 g, yield 73%). ESI-MS(m/z): 336.0[M+H]$^+$.

Step b) : Preparation of 3-(4,4-difluoroazepan-1-yl) quinoxaline-2-carboxylic acid

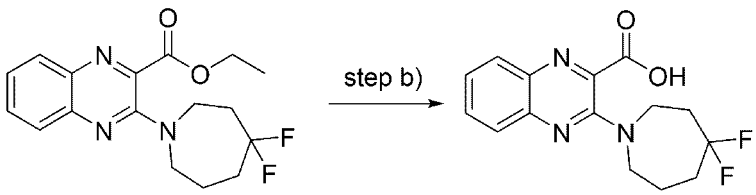


Lithium hydroxide (3.7 g, 89.2 mmol) was slowly added to a mixture of ethyl 3-(4,4-difluoroazepan-1-yl) quinoxaline-2-carboxylate (6 g, 17.8 mmol) in water (20 mL)/methanol (60 mL)/tetrahydrofuran (5 mL), and reacted at 50 °C for 2.5 h. After the reaction was completed, it was quenched with ice water, and added 1 M of dilute hydrochloric acid to adjust the pH about 7, then organic phase was extracted with ethyl acetate, washed in saturated salt water, dried with anhydrous sodium sulfate, and filtered. After the filtrate was rotated dry, yellow solid 3-(4,4-difluoroazepan-1-yl) quinoxaline-2-carboxylic acid was obtained (4 g, yield 74%). $^1$H NMR (400 MHz, DMSO-*d*$_6$) δ 7.81 (d, *J* = 8.2 Hz, 1H), 7.67 -7.55 (m, 2H), 7.47-7.34 (m, 1H), 3.78-3.72 (m, 2H), 3.64 (t, *J* = 6.0 Hz, 2H), 2.36-2.34 (m, 2H), 2.13-1.97 (m, 2H), 1.94-1.83 (m, 2H). ESI-MS(m/z): 308.1[M+H]$^+$.

Step c): Preparation of 3-(4,4-difluoroazepan-1-yl)-*N*-(pyridine-3-yl) quinoxaline-2-carboxamide

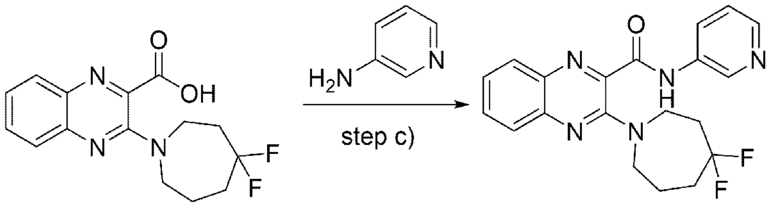


3-(4,4-difluoroazepan-1-yl) quinoxaline-2-carboxylic acid (500 mg, 1.6 mmol) was dissolved in DMF (5 mL), then DIEA (420.5 mg, 3.2 mmol) and HATU (928 mg, 2.4 mmol) were added and stirred at room temperature for 1 h, then pyridine-3-amine (183.7 mg, 1.9 mmol) was added and the reaction was continued for 2 h. 10 mL saturated $NH_4Cl$ aqueous solution was added to quench the reaction, and the resulting mixture was extracted with dichloromethane (20 mL*3), then the organic phase was separated and washed with saturated salt water, dried over anhydrous sodium sulfate, filtered, and concentrated under reduced pressure. The resulting crude product was purified by normal phase preparative chromatography to obtain yellow solid 3-(4,4-difluoroazepan-1-yl)-*N*-(pyridine-3-yl) quinoxaline-2-carboxamide (405 mg, yield 64.9%). ESI-MS(m/z): 382.1 $[M-H]^-$.

Step d): Preparation of 3-(3-(4,4-difluoroazepan-1-yl)) quinoxaline-2-carboxamido) pyridine-1-oxide(KH01)

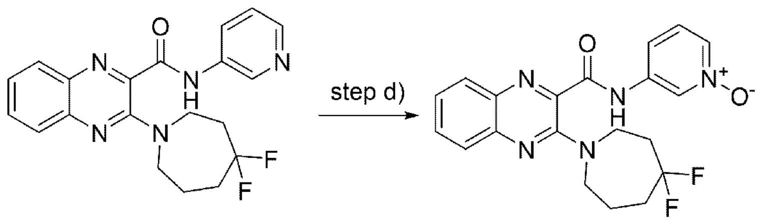


KH01

m-Chloroperbenzoic acid (180.0 mg, 1.0 mmol) was added to a solution of 3-(4,4-difluoroazepan-1-yl)-N-(pyridine-3-yl) quinoxaline-2-carboxamide (200 mg, 0.5 mmol) in dichloromethane (3 mL), and the reaction mixture was stirred at room temperature for 16 h. After the reaction was completed, 10 mL of saturated NaHCOs aqueous solution was added, and the resulting mixture was extracted with dichloromethane (20 mL×3), then the organic phase was separated and washed in saturated salt water, dried over anhydrous sodium sulfate, filtered, and concentrated under reduced pressure. The resulting crude product was purified by reverse preparative chromatography to obtain yellow solid 3-(3-(4,4-difluoroazepan-1-yl) quinoxaline-2-carboxamido) pyridine-1-oxide (56.42 mg, yield 27.0%). $^1H$ NMR (400 MHz, DMSO-$d_6$) $\delta$ 11.40 (s, 1H), 8.82 (t, $J$ = 1.6 Hz, 1H), 8.06 (d, $J$ = 6.4 Hz, 1H), 7.96 (d, $J$ = 8.2 Hz, 1H), 7.77-7.69 (m, 2H), 7.63 (d, $J$ = 9.4 Hz, 1H), 7.55-7.50(m, 1H), 7.45 (dd, $J$ = 8.4, 6.4 Hz, 1H), 3.83-3.73 (m, 2H), 3.61 (t, $J$ = 5.8 Hz, 2H), 2.40 (br s, 2H), 2.03 (t, $J$ = 16.6 Hz, 2H), 1.93 (d, $J$ = 5.4 Hz, 2H). ESI-MS(m/z): 400.0 $[M+H]^+$.

**Example 2**

Preparation of N-(3-carbamoyl-4-fluorophenyl)-3-(4,4-difluoroazepan-1-yl) quinoxaline-2-carboxamide (KH02)

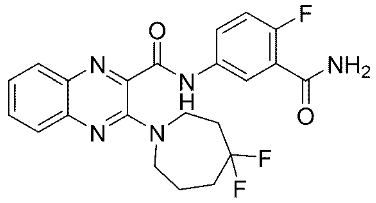

KH02

*N*-(3-carbamoyl-4-fluorophenyl)-3-(4,4-difluoroazepan-1-yl) quinoxaline-2-carboxamide is synthesized in the same way as step a-c in Example 1, except that the pyridine-3-amine in step c was replaced with 5-amino-2-fluorobenzamide, then white solid N-(3-carbamoyl-4-fluorophenyl)-3-(4,4-difluoroazepan-1-yl) quinoxaline-2-carboxamide was obtained(67.41 mg, 35.9% yield). $^{1}H$ NMR (400 MHz, $CDCl_3$) δ 9.58 (s, 1H), 8.36-8.27 (m, 1H), 8.14 (dd, *J* = 6.6, 2.9 Hz, 1H), 7.92 (dd, *J* = 8.3, 0.8 Hz, 1H), 7.74 (d, *J* = 7.7 Hz, 1H), 7.71-7.63 (m, 1H), 7.47-7.30 (m, 1H), 7.21 (dd, *J* = 11.3, 9.0 Hz, 1H), 6.76 (d, *J* = 11.3 Hz, 1H), 5.95 (s, 1H), 3.96-3.84 (m, 2H), 3.63 (d, *J* = 5.1 Hz, 2H), 2.55-2.38 (m, 2H), 2.02 (dd, *J* = 12.3, 8.2 Hz, 4H). ESI-MS(m/z): 444.1 $[M+H]^+$.

**Example 3**

Preparation of 3-(4,4-difluoroazepan-1-yl)-N-(2-carbonyl-1,2-dihydropyridin-4-yl) quinoxaline-2-carboxamide (KH03)

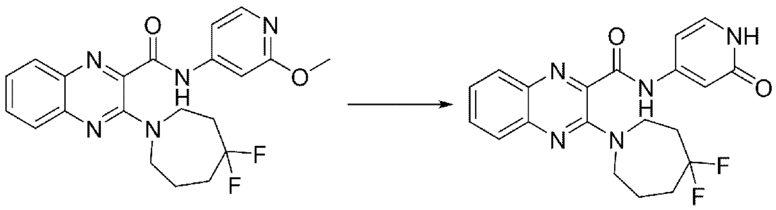

KH03

3-(4,4-difluoroazepan-1-yl)-*N*-(2-methoxypyridin-4-yl) quinoxaline-2-carboxamide is synthesized in the same way as step a-c in Example 1, except that the pyridine-3-amine in step c is replaced with 2-methoxypyridin-4-amine.

Sodium iodide (181.5 mg, 1.21mmol) and trimethylchlorosilane (131 mg, 1.21 mmol) were added to a solution of 3-(4,4-difluoroazepan-1-yl)-N-(2-methoxypyridin-4-yl) quinoxaline-2-carboxamide (190 mg, 0.605 mmol) in tetrahydrofuran (4 mL), and the resulting mixture was reacted at 50 °C for 16 h. After the reaction was completed, it was quenched with water and extracted with ethyl acetate. The organic phase obtained was washed with saturated salt water, dried over anhydrous sodium sulfate, filtered, and concentrated under reduced pressure. The resulting crude product was prepared by C18 reversed columnar under the condition of formic acid to obtain light yellow solid powder 3-(4,4-difluoroazepan-1-yl)-*N*-(2-carbonyl-1,2-dihydropyridine-4-yl) quinoxaline-2-carboxamide (67.1 mg, yield 37%). $^{1}H$ NMR (400 MHz, DMSO-$d_6$) δ 11.38 (br s, 1H), 11.07 (s, 1H), 7.95 (d, *J* = 8.2 Hz, 1H), 7.71 (d, *J* = 3.5 Hz, 2H), 7.56-7.45 (m, 1H), 7.38 (d, *J* = 7.1 Hz, 1H), 6.87 (s, 1H), 6.50 (d, *J* = 7.1 Hz, 1H), 3.77 (d, *J* = 2.5 Hz, 2H), 3.62 (br s, 2H), 2.39 (br s, 2H), 2.03 (br s, 2H), 1.93 (d, *J* = 5.1 Hz, 2H). ESI-MS(m/z): 400.1 $[M+H]^+$.

**Example 4**

Preparation of 3-(4,4-difluoroazepan-1-yl)-N-(6-carbonyl-1,6-dihydropyridazin-4-yl) quinoxaline-2-carboxamide (KH04)

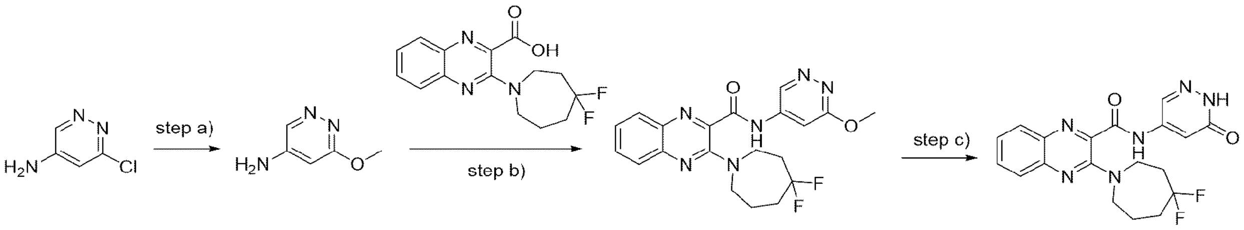


KH04

Step a): Preparation of 6-methoxypyridazin-4-amine

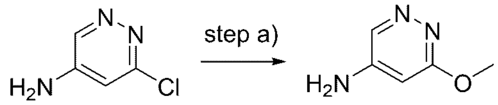


Sodium methylate (1.78g, 32.9mmol) was added to a solution of 6-chloro-pyridazin-4-amine (850 mg, 6.589 mmol) in methanol (20 mL) at 0 °C and stirred at 75 °C for 16 h. After the reaction mixture was cooled to room temperature, it was filtered and the filtrate was extracted and concentrated under reduced pressure. The resulting crude product was purified by silica gel column chromatography to obtain white solid 6-methoxypyridazin-4-amine (780 mg, yield 94%). $^1H$ NMR (400 MHz, DMSO-$d_6$) δ 8.26 (d, *J* = 1.5 Hz, 1H), 6.33 (s, 2H), 5.97 (d, *J* = 1.4 Hz, 1H), 3.87 (s, 3H). ESI-MS(m/z): 126.1 $[M+H]^+$.

Step b): Preparation of 3-(4,4-difluoroazepan-1-yl)-N-(6-methoxydrazin-4-yl) quinoxaline-2- carboxamide

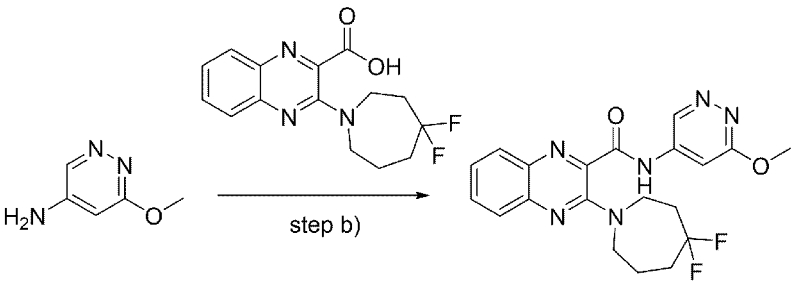


For the synthesis of 3-(4,4-difluoroazepan-1-yl) quinoxaline-2-carboxylic acid, see step a-b of Example 1.

HATU (297 mg, 0.781 mmol) and DIEA (210 mg, 1.628 mmol) were added to a solution of 3-(4,4-difluoroazepan-1-yl) quinoxaline-2-carboxylic acid (200 mg, 0.651 mmol) in DMF (5 mL). After it was reacted at room temperature for 0.5 h, 6-methoxy-pyridazin-4-amine (163 mg, 1.302 mmol) was added and reacted at room temperature for 16 h. After the reaction was completed, water was added to quench the reaction, and the organic phase (20 mL) was extracted with ethyl acetate, washed with saturated salt water, dried over anhydrous sodium sulfate, and filtered. The filtrate was concentrated under reduced pressure, and the resulting crude product was purified by silica gel plate (ethyl acetate/petroleum ether (v/v) = 1/1) to obtain a light yellow oil 3-(4, 4-difluoroazepan-1-yl)-*N*-(6-methoxy-pyridazin-4-yl) quinoxaline-2-carboxamide (55 mg, 20% yield). $^1H$ NMR (400 MHz, DMSO-d6) δ 11.65 (s, 1H), 9.13 (s, 1H), 7.95 (s, 1H), 7.74 (d, *J* = 13.5 Hz, 2H), 7.63 (s, 1H), 7.52 (s, 1H), 4.05 (s, 3H), 3.77 (br s, 2H), 3.58 (t, *J* = 5.6 Hz, 2H), 2.39 (br s, 2H), 2.00 (d, *J* = 12.4 Hz, 2H), 1.92 (d, *J* = 4.7 Hz, 2H). ESI-MS(m/z): 415.1 $[M+H]^+$.

Step c): Preparation of 3-(4,4-difluoroazepan-1-yl)-N-(6-carbonyl-1,6-dihydropyridazin-4-yl) quinoxaline-2-carboxamide (KH04)

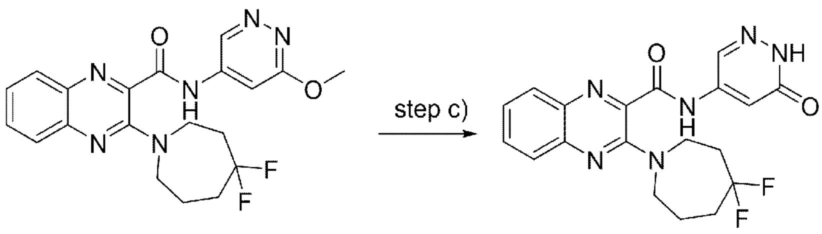


Sodium iodide (50 mg, 0.332 mmol) and trimethylchlorosilane (36 mg, 0.332 mmol) were added to a solution of 3-(4,4-difluoroazepan-1-yl)-N-(6-methoxydiazin-4-yl) quinoxaline-2-carboxamide (55 mg, 0.133 mmol) in tetrahydrofuran (4 mL), and that was reacted at 50 °C for 16 hours. After the reaction was completed, water was added to quench the reaction. The organic phase was extracted with ethyl acetate (20 mL), washed with saturated salt water, dried with anhydrous sodium sulfate, and filtered. The filtrate was concentrated under reduced pressure, and the resulting crude product was prepared by C18 reverse column under the condition of formic acid to obtain the light yellow solid powder 3-(4,4-difluoroazepan-1-yl)-*N*-(6-carbonyl-1,6-dihydropyridazin-4-yl) quinoxaline-2- carboxamide (13.2 mg, yield 25%). $^1H$ NMR (400 MHz, DMSO-$d_6$) δ 12.89 (s, 1H), 11.45 (s, 1H), 8.09 (d, *J* = 2.3 Hz, 1H), 7.96 (d, *J* = 8.1 Hz, 1H), 7.73-7.72 (m, 2H), 7.52 (ddd, *J* = 8.3, 5.6, 2.8 Hz, 1H), 7.33 (d, *J* = 2.0 Hz, 1H), 3.79-3.74 (m, 2H), 3.57 (br s, 2H), 2.40 (br s, 2H), 2.05-2.00 (m, 2H), 1.93 (br s, 2H). ESI-MS(m/z): 401.1 $[M+H]^+$.

**Example 5**

Preparation of N-(2-carbamoylpyridin-4-yl)-3-(4,4-difluoroazepan-1-yl) quinoxaline-2- carboxamide (KH05)

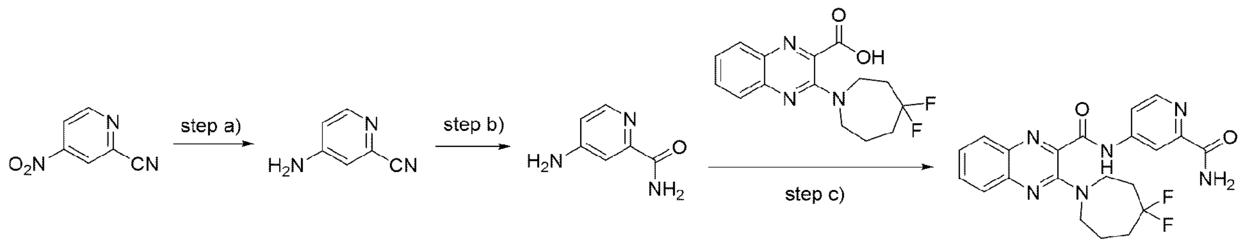


KH05

Step a) : Preparation of 4-aminopyridine-2-carbonitrile

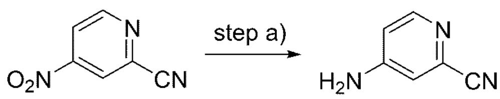


Iron powder (1.88 g, 33.5 mmol) and ammonium chloride (1.78 g, 33.5 mmol) were added to a mixture of 2-cyano-4-nitropyridine (1 g, 6.7 mmol) in ethanol (100 mL)/ water (10 mL) and stirred at 60 °C for 2 h. After the reaction mixture was cooled to room temperature, it was filtered, and the filtrate was quenched with water, then the organic phase was extracted with ethyl acetate, washed with saturated brine, dried with anhydrous sodium sulfate, filtered, and concentrated under reduced pressure. The resulting crude product was purified by silica gel column chromatography (methanol/dichloromethane (v/v) = 1/40) to obtain white solid 4-aminopyridine-2-carbonitrile (400 mg, yield 51%). $^1H$ NMR (400 MHz, DMSO-$d_6$) δ 8.07 (d, *J* = 5.7 Hz, 1H), 6.95 (d, *J* = 2.3 Hz, 1H), 6.69 (dd, *J* = 5.7, 2.3 Hz, 1H), 6.60 (s, 2H). ESI-MS(m/z): 120.2 $[M+H]^+$.

Step b): Preparation of 4-aminopyridine-2-carboxamide

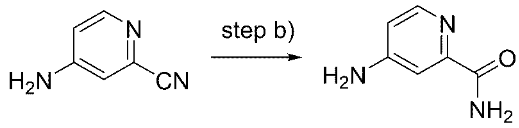


Cesium carbonate (1.65 g, 10.08 mmol) was added to a solution of 4-aminopyridine-2-carbonitrile (400 mg, 3.36 mmol) in dimethyl sulfoxide (10 mL), and 30% hydrogen peroxide (0.5mL) was slowly added at 0 °C. The reaction was carried out at room temperature for 4 h. After the reaction was completed, it was quenched with ice water, and the organic phase was extracted with ethyl acetate, washed with saturated salt water, dried with anhydrous sodium sulfate, and filtered. The filtrate was concentrated under reduced pressure, and the resulting crude product was purified by reverse phase preparative chromatography to obtain 4-aminopyridine-2-carboxamide (300 mg, yield 65%). $^1$H NMR (400 MHz, DMSO-$d_6$) δ 8.01 (d, *J* = 5.5 Hz, 1H), 7.89 (s, 1H), 7.39 (s, 1H), 7.21 (d, *J* = 2.3 Hz, 1H), 6.58 (dd, *J* = 5.5, 2.4 Hz, 1H), 6.28 (s, 2H). ESI-MS(m/z): 308.1 $[M+H]^{+}$.

Step c): Preparation of N-(2-carbamoylpyridin-4-yl)-3-(4,4-difluoroazepan-1-yl) quinoxaline-2-carboxamide (KH05)

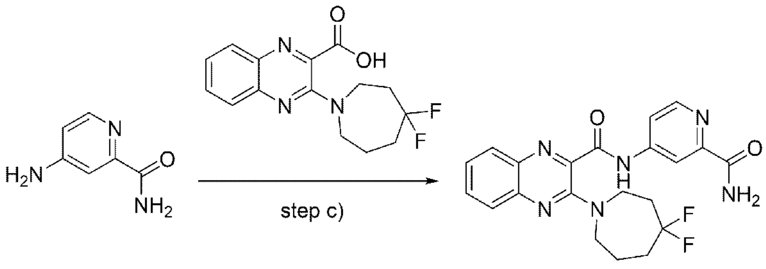


KH05

For the synthesis of 3-(4,4-difluoroazepan-1-yl) quinoxaline-2-carboxylic acid see step a-b of Example 1.

2-(7-Azobenzotriazole-1-yl)-N,N,N',N'-tetramethyluronium hexafluorophosphate (HATU) (333 mg, 0.8795 mmol), N,N-diisopropylethylamine (188 mg, 1.459 mmol) were added to a solution of 3-(4,4-difluoroazepan-1-yl) quinoxaline-2-carboxylic acid (224 mg, 0.730 mmol) in N,N-dimethylformamide (5 mL), and the reaction mixture was reacted at room temperature for 0.5 h, and then 4-aminopyridine-2-carboxamide (110 mg, 0.803 mmol) was added, and the resulting mixture was reacted at room temperature for 16 h. After the reaction was completed, it was quenched with water, and the organic phase was extracted with ethyl acetate, washed with saturated salt water, dried with anhydrous sodium sulfate, filtered, and concentrated under reduced pressure. The resulting crude product was purified by preparative chromatography to a light yellow solid powder N-(2-carbamoylpyridin-4-yl)-3-(4,4-difluoroazepan-1-yl) quinoxaline-2-carboxamide (25.6 mg, yield 8.3%). $^1$H NMR (400 MHz, DMSO-$d_6$) δ 11.55 (s, 1H), 8.58 (d, *J* = 5.4 Hz, 1H), 8.45 (s, 1H), 8.13 (s, 1H), 8.03-7.91 (m, 2H), 7.77-7.71 (m, 2H), 7.68 (s, 1H), 7.55-7.48 (m, 1H), 3.88-3.76 (m, 2H), 3.63 (t, *J* = 5.7 Hz, 2H), 2.40 (br s, 2H), 2.10-2.01 (m, 2H), 1.93 (d, *J* = 5.4 Hz, 2H). ESI-MS(m/z): 427.1 $[M+H]^+$.

**Example 6**

Preparation of 3-(4,4-difluoroazepan-1-yl)-N-(2-sulfamoylpyridin-4-yl) quinoxaline-2-carboxamide (KH06)

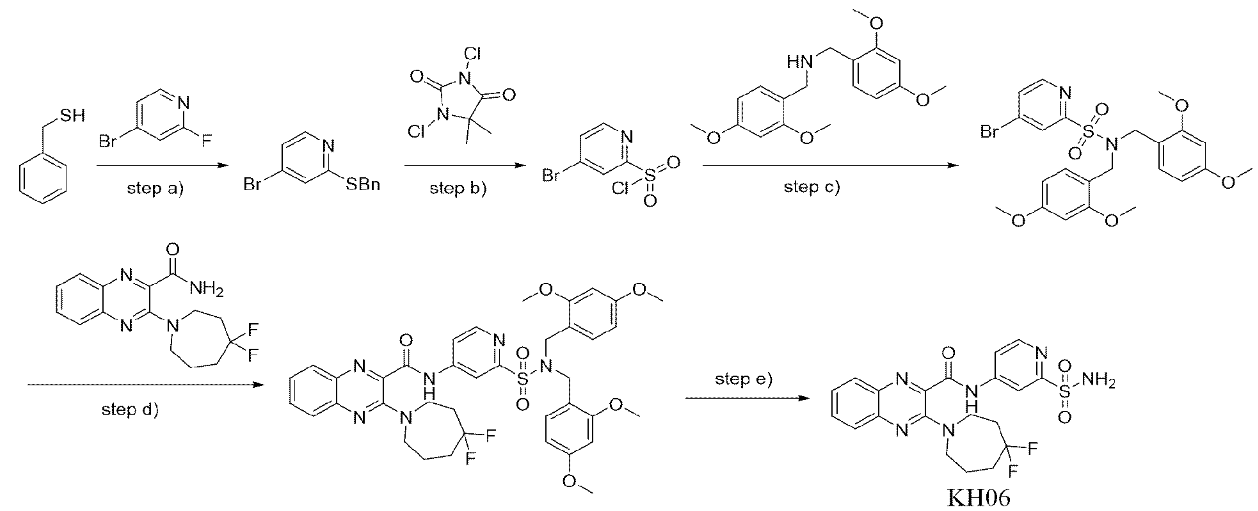


KH06

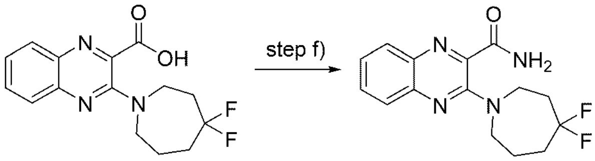


Step a): Preparation of 2-benzylthio-4-bromopyridine

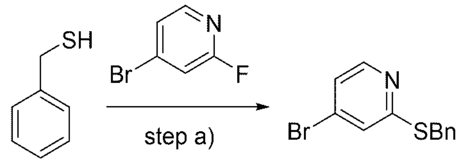


The solution of benzyl thiol (3 g, 24.1 mmol) in tetrahydrofuran (50 mL) was placed into an ice water bath, and NaH (1.45 g, 60.4 mmol, 60%) was added in batches, then the reaction system was returned to room temperature and stirred for 1 h. Then 4-bromo-2-fluoropyridine (4.5 g, 25.5 mmoL) was added and it was continued to react for 2 h. After the reaction was completed, the reaction mixture was concentrated under reduced pressure, and 20 mL saturated $NH_4Cl$ aqueous solution was added under the condition of ice water bath, organics were extracted with dichloromethane (20 mL×3), and the organic phase was separated and washed with saturated salt water, dried with anhydrous sodium sulfate, and filtered. The filtrate was concentrated under reduced pressure, and the resulting crude product was purified by normal phase chromatography (petroleum ether/ethyl acetate (v/v) = 4/1) to obtain colorless oil 2-benzylthioether-4-bromopyridine (5.8 g, yield 85.6%). ESI-MS(m/z) : 281.9 $[M+2]^+$.

Step b): Preparation of 4-bromopyridine-2-sulfonyl chloride

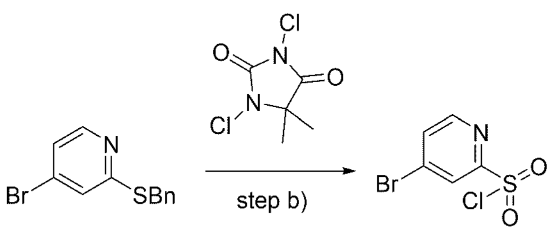

Water (8 mL) and acetic acid (4 mL) were added to a solution of 2-benzylthio-4-bromopyridine (2 g, 7.1 mmol) in dichloromethane (28 mL), and the reaction system was cooled to 0°C. 1,3-Dichloro-5,5-dimethylimidazolidine-2,4-dione (4.22 g, 21.4 mmol) was added in batches, and then the reaction system was returned to room temperature and stirred for 16 h. After the reaction was completed, the reaction system was placed into an ice water bath, and 20 mL saturated NaHCOs aqueous solution was added to quench the reaction. The organics was extracted with dichloromethane (20 mL×3), and the organic phase was separated and washed with saturated salt water, dried with anhydrous magnesium sulfate, and filtered. The cream-colored semi-oily 4-bromopyridine-2-sulfonyl chloride (4.78g, crude) was obtained after concentration under reduced pressure. ESI-MS(m/z): 253.9 $[M\text{-}Cl+MeO^{-}+2]^{+}$.

Step c): Preparation of 4-bromo-N,N-bis(2,4-dimethoxybenzyl)pyridine sulfonamide

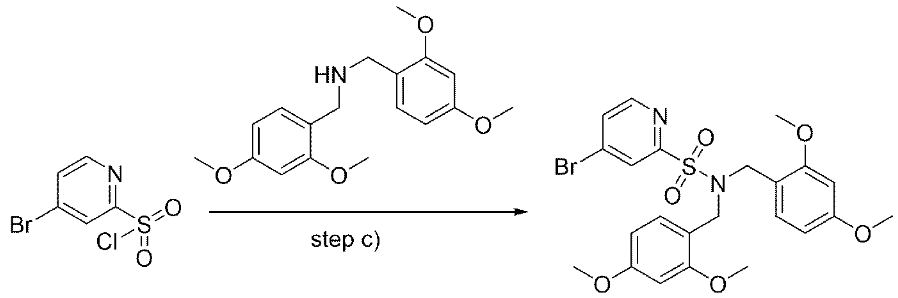


4-Bromopyridine-2-sulfonyl chloride (2 g, 7.8 mmol) was dissolved in dichloromethane (20 mL), and DIEA (5.04 g, 38.9 mmol) was added under an ice water bath, and then bis(2,4-dimethoxybenzyl) amine (3.71 g, 11.7 mmol) was added in batches. After that, the reaction system was returned to room temperature and stirred for 4 h. After the reaction was completed, 20 mL saturated $NH_4Cl$ aqueous solution was added to quench the reaction system, and the organic phase was extracted with dichloromethane (25 mL×3), separated and washed with saturated salt water, dried with anhydrous sodium sulfate, and filtered. The filtrate was concentrated under reduced pressure. The resulting crude product was purified by normal phase chromatography (petroleum ether/ethyl acetate (v/v) = 4/1), and the light brown sugar 4-bromo-N,N-bis(2,4-dimethoxybenzyl)pyridine sulfonamide was prepared (860 mg, yield 20.5%). LCMS (ESI, *m/z*) : 560.9 $[M+Na+2]^{+}$. $^{1}H$ NMR (400 MHz, DMSO-$d_6$) δ 8.51 (d, *J* = 5.2 Hz, 1H), 7.86-7.82 (m, 1H), 7.60 (d, *J* = 1.8 Hz, 1H), 7.08 (d, *J* = 8.4 Hz, 2H), 6.45 (dd, *J* = 8.4, 2.3 Hz, 2H), 6.35 (d, *J* = 2.3 Hz, 2H), 4.43 (s, 4H), 3.73 (s, 6H), 3.61 (s, 6H). ESI-MS(m/z): 560.9 $[M+Na^{+}+2]^{+}$.

Step d): Preparaton of N-(2-(N,N-bis(2,4-dimethoxybenzyl)sulfamoyl)pyridin-4-yl)-3-(4,4-difluoroazepan-1-yl) quinoxaline-2-carboxamide

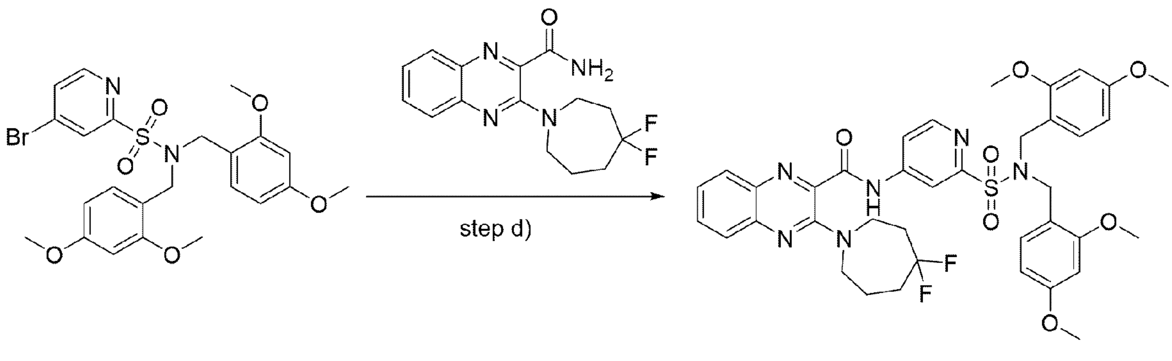


4-Bromo-N,N-bis (2,4-dimethoxybenzyl) pyridine sulfonamide (140 mg, 0.26 mmol) was dissolved in toluene (2 mL), then 3-(4,4-difluoroazepan-1-yl) quinoxaline-2-carboxamide (87.7 mg, 0.28 mmol) and $Cs_2CO_3$ (254.6 mg, 0.78 mmol) were added, followed by Ruphos Pd $G_2$ (20.2 mg, 0.026 mmol). After the reaction system was replaced with nitrogen, the reaction was carried out at 110°C for 16 h. After the reaction was completed, the reaction system was cooled to room temperature, and then concentrated under reduced pressure. The resulting residue was added with 10

mL saturated $NH_4Cl$ aqueous solution, and extracted with dichloromethane (25 mL×3). The organic phase was separated and washed with saturated salt water, dried with anhydrous sodium sulfate, and filtered. The filtrate was concentrated under reduced pressure. The resulting crude products was purified by the positive phase chromatography (petroleum ether/ethyl acetate (v/v) = 3/1) to obtain yellow solid N-(2-(N,N-bis(2,4-dimethoxybenzyl)sulfamoy)pyridin-4-yl)-3-(4,4-difluoroazepan-1-yl) quinoxaline-2-carboxamide (116.8 mg, Yield 58.7%). ESI-MS($^+$m/z): 763.1 [M+H].

Step e): Preparation of 3-(4,4-difluoroazepan-1-yl)-N-(2-sulfamoylpyridin-4-yl) quinoxaline-2- carboxamide (KH06)

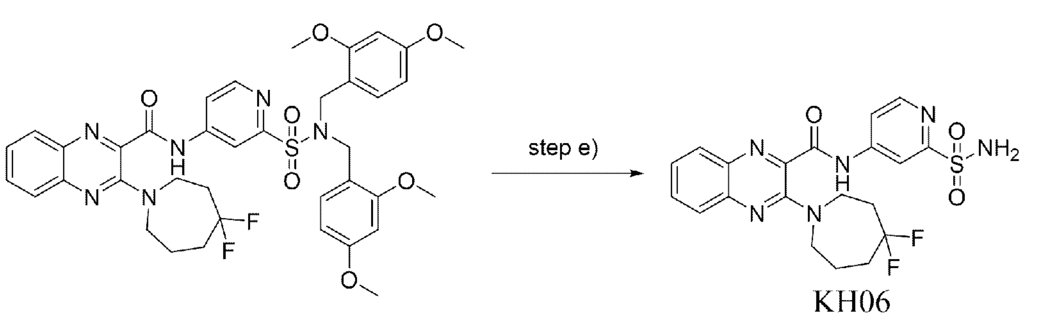


KH06

N-(2-(N,N-bis(2,4-dimethoxybenzyl)sulfamoyl)pyridin-4-yl)-3-(4,4-difluoroazepan-1-yl) quinoxaline-2-carboxamide (110 mg, 0.14 mmol) was dissolved in dichloromethane (2 mL), then trifluoroacetic acid (1 mL) was added and the reaction system was stirred at room temperature for 2 h after addition. After the reaction was completed, 10 mL saturated NaHCOs aqueous solution was added, and the reaction mixture was extracted with dichloromethane (25 mL×3). The organic phase was separated and washed with saturated salt water, dried with anhydrous sodium sulfate, and filtered, and the filtrate was concentrated under reduced pressure. The resulting crude product was purified by reverse phase chromatography to obtain yellow solid N-(2-aminosulfonylpyridine-4-yl)-3-(4,4-difluoroazepan-1-yl)quinoline-2-carboxamide(31.17 mg, yield 46.7%). $^1$H NMR (400 MHz, DMSO-$d_6$) δ 11.71 (s, 1H), 8.66 (d, *J* = 5.4 Hz, 1H), 8.40 (d, *J* = 1.7 Hz, 1H), 7.96 (d, *J* = 8.1 Hz, 1H), 7.93 (dd, *J* = 5.5, 2.0 Hz, 1 H), 7.77-7.71 (m, 2 H), 7.55-7.51 (m, 1 H), 7.50 (d, *J* = 4.2 Hz, 2 H), 3.86-3.74 (m, 2 H), 3.61 (t, *J* = 5.7 Hz, 2H), 2.40 (brs, 2H), 2.05 (d, *J* = 19.4 Hz, 2H), 1.93 (d, *J* = 5.4 Hz, 2H). ESI-MS(m/z): 463.0 [M+H]$^+$.

Step f): Preparation of 3-(4, 4-difluoroazepan-1-yl) quinoxaline-2-carboxamide

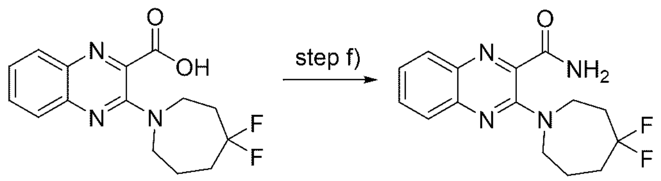


For the synthesis of 3-(4,4-difluoroazepan-1-yl) quinoxaline-2-carboxylic acid, see step a-b of Example 1.
HATU (371.2 mg, 0.9 mmol) and DIEA (420.5 mg, 3.2 mmol) were added to a solution of 3-(4,4-difluoroazepan-1-yl) quinoxaline-2-carboxylic acid (200 mg, 0.6 mmol) in DMF (5 mL). After half an hour, $NH_4Cl$ (139.2 mg, 2.6 mmol) was added and stirred at room temperature for 2 h. Water (15 mL) was added to quench the reaction, then dichloromethane (20 mL×3) was used to extract the reaction system, and the organic phase was separated and washed with saturated salt water, dried with anhydrous sodium sulfate, and filtered, and the filtrate was concentrated under reduced pressure. The resulting crude product was purified by normal phase chromatography (dichloromethane/methanol (v/v) = 20/1) to obtain yellow solid 3-(4,4-difluoroazepan-1-yl) quinoxaline-2-carboxamide (160 mg, yield 80.2%). $^1$H NMR (400 MHz, DMSO-$d_6$) δ 8.25 (brs, 1H), 7.85 (d, *J* = 7.2 Hz, 2H), 7.65 (d, *J* = 3.9 Hz, 2H), 7.45-7.40 (m, 1H), 3.84 3.73 (m, 2 H), 3.68 (2 H, t, *J* = 5.8 Hz), 2.39 (d, *J* = 10.3 Hz, 2 H), 2.11-1.88 (m, 4H). ESI-MS(m/z): 306.9 [M+H]$^+$.

**Example 7**

Preparation of N-(3-carbamoylphenyl)-3-(4,4-difluoroazepan-1-yl) quinoxaline-2-carboxamide (KH07)

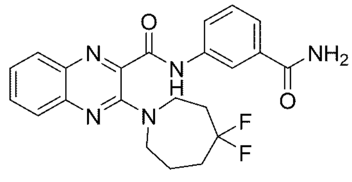

KH07

The procedure was the same as in step a-c of Example 1, except that pyridine-3-amine was replaced with 3-aminobenzamide, and after the reaction, it was extracted with ethyl acetate to obtain white solid N-(3-carbamoylphenyl)-3-(4,4-difluoroazepan-1-yl) quinoxaline-2-carboxamide (50 mg, yield 12.1%). $^1$H NMR (400 MHz, $CDCl_3$) δ 11.10 (s, 1H), 8.24 (s, 1H), 8.02 (brs, 1H), 7.94 (dd, *J* = 16.2, 8.1 Hz, 2H), 7.73 (d, *J* = 4.0 Hz, 2H), 7.66 (d, *J* = 7.8 Hz, 1H), 7.51-7.48 (m, 2H), 7.42 (br s, 1H), 3.89-3.76 (m, 2H), 3.71 (t, *J* = 5.8 Hz, 2H), 2.41-2.38 (m, 2H), 2.13 2.04 (m, 2H), 1.95-1.92 (m, 2H). ESI-MS(m/z): 425.9 $[M+H]^+$.

**Example 8**

Preparation of N-(2-carbamoylpyridin-4-yl)-2-(4,4-difluoroazepan-1-yl)quinoline-3- carboxamide (KH08)

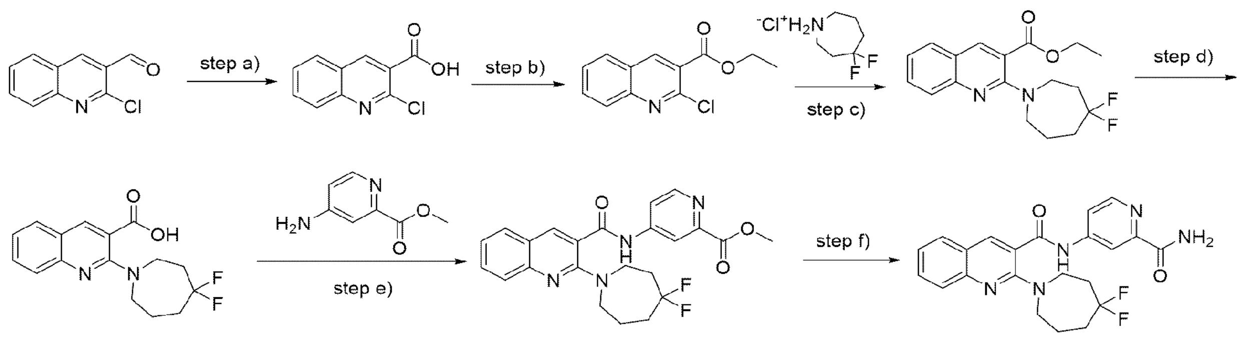


KH08

Step a): Preparation of 2-chloroquinoline-3-carboxylic acid

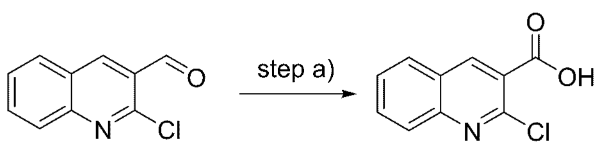


$NaClO_2$ (11.8 g, 130.9 mmol) and $Na_2H_2PO_4$ (18.5 g, 130.9 mmol) were dissolved in water (10 mL) and a solution of 2-chloroquinolin-3-formaldehyde (5 g, 26.1 mmol) in tert-butanol was added at 0°C and stirred at room temperature for 16 h. After the reaction was completed, water was added to dilute the mixture, and the pH of the reaction mixture was adjusted to 3-4 with 1M HCl solution, and it was filtered. The residue was washed to obtain light yellow solid 2-chloroquinoline-3-carboxylic acid (2.8 g, yield 52%). $^1$ H NMR (400MHz, DMSO-$d_6$) δ 8.86 (s, 1H), 8.16 (d, *J* = 8.0 Hz, *1H*), 8.00 (d, *J* = 8.4 Hz, *1H*), 7.92 (dd, *J* = 11.2, 4.1 Hz, 1H), 7.72 (t, *J* = 7.5 Hz, 1H). ESI-MS(m/z): 208.1 $[M+H]^+$.

Step b): Preparation of ethyl 2-chloroquinoline-3-carboxylate

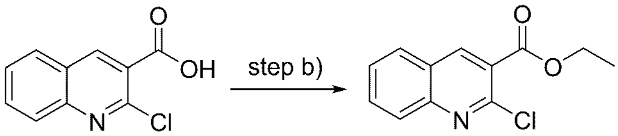


$K_2CO_3$ (3.7 g, 27 mmol) and ethane iodide (4.2 g, 27 mmol) were added to a solution of 2-chloroquinolin-3-carboxylic acid (2.8 g, 13.5 mmol) in DMF (20 mL) and stirred at room temperature for 16 h. After the reaction was completed, water was added to dilute the reaction mixture, and reaction system was extracted with ethyl acetate (100 mL×3). The combined organic phase was washed with saturated salt water for 3 times, and the solvent was removed by vacuum rotary evaporation. The crude product was purified by silica gel column chromatography (petroleum ether/ethyl acetate (v/v) = 3/1) to obtain light yellow solid ethyl 2-chloroquinoline-3-carboxylate (2.3 g, yield 72%). $^1H$ NMR (400 MHz, $CDCl_3$) δ 8.95-8.50 (m, 1H), 8.07 (t, *J* = 13.4 Hz, 1H), 7.90 (d, *J* = 8.2 Hz, 1H), 7.86-7.80 (m, 1H), 7.68-7.55 (m, 1H), 4.48 (q, *J* = 7.1 Hz, 2H), 1.46 (t, *J* = 7.1 Hz, 3H). ESI-MS(m/z): 236.1 $[M+H]^+$.

Step c): Preparation of ethyl 2-(4,4-difluoroazepan-1-yl) quinoline-3-carboxylate

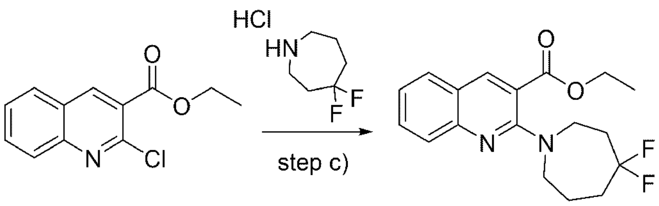


4-Difluoroazepane hydrochloride (1.9 g, 11.64 mmol) and DIEA (3.75 g, 29.1 mmol) were added to a solution of 2-chloroquinolin-3-carboxylic acid (2.3 g, 9.7 mmol) in NMP (15 mL), respectively. And the reaction mixture was stirred at 140°C for 16 h. After the reaction was completed, it was cooled to room temperature, and diluted with 100 mL water, then the reaction mixture was extracted with ethyl acetate (100 mL×3), and the combined organic phase was washed with saturated salt water, dried with anhydrous sodium sulfate, and filtered. The filtrate was concentrated under reduced pressure. The crude product was purified by silica gel column chromatography (petroleum ether/ethyl acetate (v/v) = 5/1) to obtain light yellow oil ethyl 2-(4,4-difluoroazepan-1-yl)quinoline-3-carboxylate (2 g, yield 61%). $^1H$ NMR (400 MHz, $CDCl_3$) δ 8.31 (s, 1H), 7.68 (dd, *J = 10.6,* 8.7 Hz, 2H), 7.60-7.56 (m, 1H), 7.25-7.21 (m, 1H), 4.40 (q, *J* = 7.1 Hz, 2H), 3.88-3.74 (m, 2H), 3.48 (2H, t, *J* = 5.6 Hz), 2.58-2.40 (m, 2H), 1.98 (dd, *J* = 8.7, 4.1 Hz, 4H), 1.42 (t, *J* = 7.1 Hz, 3H). ESI-MS(m/z): 335.0 [M+H].$^+$

Step d) : Preparation of 2-(4,4-difluoroazepan-1-yl)quinoline-3-carboxylic acid

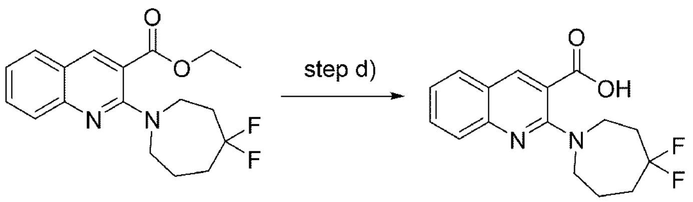


Lithium hydroxide (288 mg, 12 mmol) was added to a solution of 2-(4,4-difluoroazepan-1-yl)quinoline-3-ethylcarboxylate(2 g, 6.0 mmol) in methanol/water (10 mL/2 mL), and reacted at room temperature for 16 h. After the reaction was completed, the pH of the reaction system was adjusted to 3-4 with 1M HCl solution, then it was extracted with ethyl acetate (50 mL×3), and the combined organic phase was washed with saturated salt water (50 mL), dried with anhydrous

sodium sulfate, and filtered. The filtrate was concentrated under reduced pressure into yellow solid 2-(4,4-difluoroazepan-1-yl) quinoline-3-carboxylic acid (1.4 g, yield 70%), which was directly used in the next step. ESI-MS($^+$m/z): 306.9 [M+H].

Step e): Preparation of N-(2-methylformate pyridin-4-yl)-2-(4,4-difluoroazepan-1-yl) quinoline-3-carboxamide

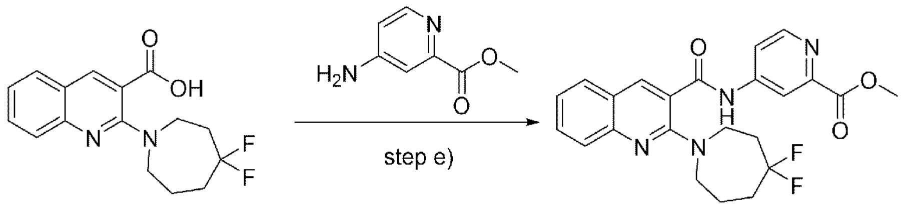


Oxalyl chloride (621 mg, 4.89 mmol) and a drop of DMF were added to 2-(4,4-difluoroazepan-1-yl) quinoline-3-carboxylic acid (500 mg, 1.63 mmol) at 0 °C and reacted at room temperature for 1h. The reaction mixture was concentrated under reduced pressure, and the obtained crude product was dissolved in 4 mL dichloromethane. Then triethylamine (823 mg, 8.15 mmol) and methyl 4-aminopyridine-2-carboxylate (300 mg, 1.95 mmol) were added, and reacted at room temperature for 16 h. After the reaction was completed, it was diluted with water and then extracted with ethyl acetate (30 mL×3). The combined organic phase was washed with saturated salt water (50 mL), dried with anhydrous sodium sulfate and filtered. Filtrate was concentrated under reduced pressure to obtain yellow solid N-(2-methylformate pyridin-4-yl)-2-(4,4-difluoroazepan-1-yl) quinoline-3-carboxamide (300 mg, yield 41%), which was directly used for the next step. ESI-MS(m/z): 441.2 $[M+H]^+$.

step f): Preparation of N-(2-carbamoylpyridin-4-yl)-2-(4,4-difluoroazepan-1-yl)quinoline-3-carboxamide (KH08)

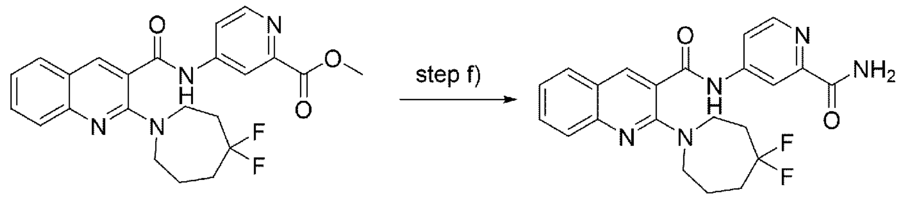


N-(2-methylformate pyridin-4-yl)-2-(4,4-difluoroazepan-1-yl)quinoline-3-carboxamide (300 mg, 0.68 mmol) was dissolved in 3 mL acetonitrile. Ammonium chloride (6.3 mg, 0.12 mmol) and 1.8 mL ammonia water were added and the resulting mixture was reacted at room temperature for 3 h. After the reaction was completed, the reaction system was concentrated and the crude product was purified by Prep-HPLC to obtain white solid N-(2-carbamoylpyridine-4-yl)-2-(4,4-difluoroazepan-1-yl)quinoline-3-carboxamide (50 mg, yield 17.3%). $^1$H NMR (400 MHz, DMSO-$d_6$) δ 11.20 (s, 1H), 8.56 (d, *J* = 5.5 Hz, 1H), 8.48-8.33 (m, 2H), 8.11 (brs, 1H), 7.95-7.80 (m, 2H), 7.65 (d, *J* = 3.6 Hz, 3H), 7.32-7.30 (m, 1H), 3.80-3.67 (m, 2H), 3.57 (2H, t, *J* = 5.8 Hz), 2.43-2.31 (m, 2H), 2.01-1.98 (m, 2H), 1.89 (d, *J* = 5.4 Hz, 2H). ESI-MS(m/z): 425.9 $[M+H]^+$.

**Example 9**

Preparation of N-(3-carbamoyl-4-fluorophenyl)-2-(4,4-difluoroazepan-1-yl)quinoline-3- carboxamide (KH09)

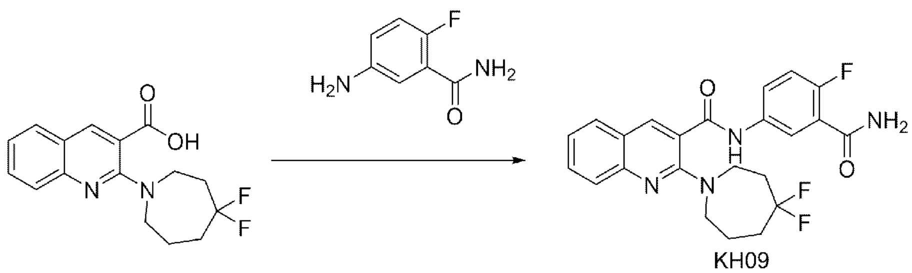

KH09

For the synthesis of 2-(4,4-difluoroazepan-1-yl)quinoline-3-carboxylic acid, see step a-d in Example 8.

HATU (296 mg, 0.778 mmol), 5-amino-2-fluorobenzamide (92.2 mg, 0.598mmol) and DIEA (231 mg, 1.796 mmol) were added to a solution of (4,4-difluoroazepan-1-yl)quinoline-3-carboxylic acid (200 mg, 0.598 mmol) in DMF (5 mL), and reacted at room temperature for 16 h. After the reaction was completed, water was added to quench the reaction, and the organic phase was extracted with ethyl acetate (20 mL×3), washed with saturated salt water, dried with anhydrous sodium sulfate, and the filtrate was concentrated under reduced pressure. The resulting crude product was prepared by Prep-HPLC to obtain white solid powder of N-(3-carbamoyl-4-fluorophenyl)-2-(4,4-difluoroazepan-1-yl)quinoline-3-carboxamide (108.7 mg, yield 41%). $^1$H NMR (400 MHz, DMSO-$d_6$) δ 10.79 (s, 1H), 8.32 (s, 1H), 8.03 (dd, *J* = 6.4, 2.7 Hz, 1H), 7.86 (d, *J* = 4.4 Hz, 1H), 7.84 (d, *J* = 3.6 Hz, 1H), 7.68 (br s, 2H), 7.64 (br s, 2H), 7.30 (dd, *J* = 11.9, 7.2 Hz, 2H), 3.76 (d, *J* = 4.8 Hz, 2H), 3.62 (t, *J* = 5.8 Hz, 2H), 2.40 (brs, 2H), 2.00 (brs, 2H), 1.89 (d, *J* = 4.9 Hz, 2H). ESI-MS(m/z): 443.2 $[M+H]^+$.

**Example 10**

Preparation of N-(2-carbamoylpyridin-4-yl)-2-(4,4-difluoroazepan-1-yl)-6,7-difluoroquinoline-3-carboxamide (KH10)

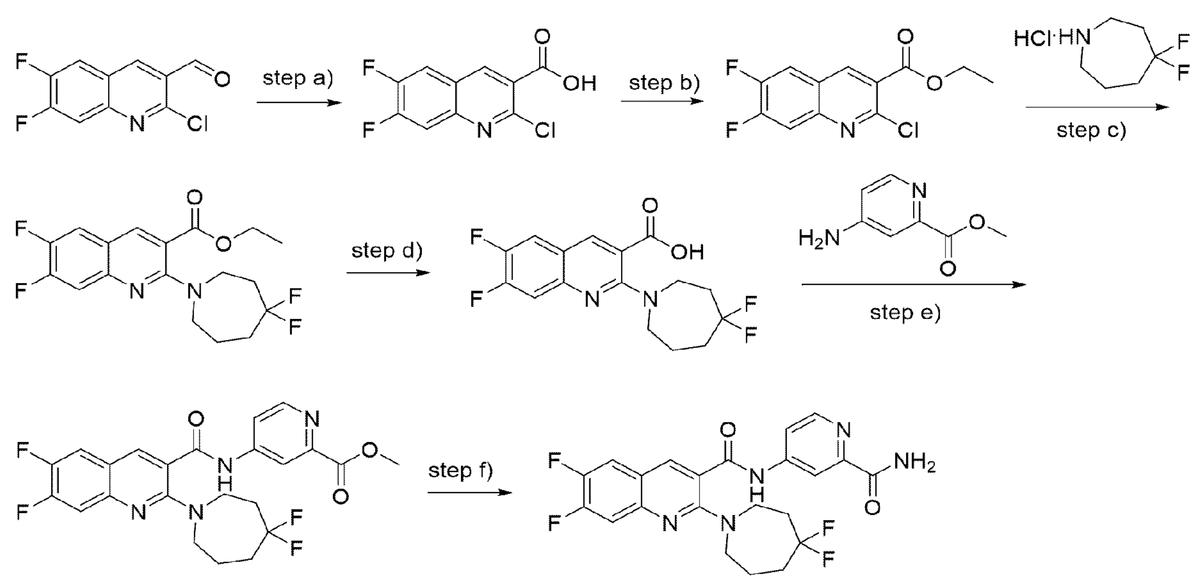


KH10

Step a) : Preparation of 2-chloro-6,7-difluoroquinolin-3-carboxylic acid

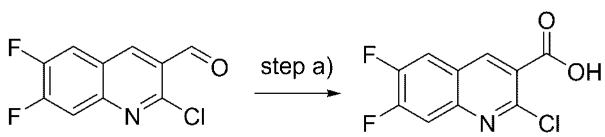

Silver nitrate (1.2 g, 7.02 mmol) was dissolved in a pre-configured solution of NaOH (0.88 g, 22 mmol) in ethanol/water (12 mL/3 mL) under an ice water bath, and the solution of 2-chloro-6,7-difluoroquinolin-3-carbaldehyde (1 g, 4.39 mmol) in ethanol (10 mL) was slowly dripped into it at room temperature and stirred for 16 h. After the reaction was completed, the reaction mixture was filtered, and the pH of the filtrate was adjusted to 6 with 1 M hydrochloric acid, then organic phase was extracted with ethyl acetate (50 mL×3), washed with saturated salt water (30 mL×2), dried with anhydrous sodium sulfate, and filtered, and the filtrate was concentrated under reduced pressure. The white solid 2-chloro-6,7-difluoroquinolin-3-carboxylic acid was obtained (1.0 g, yield 93.46%).

ESI-MS(m/z): 244.0 $[M+H]^+$.

Step b) : Preparation of ethyl 2-chloro-6,7-difluoroquinolin-3-carboxylate

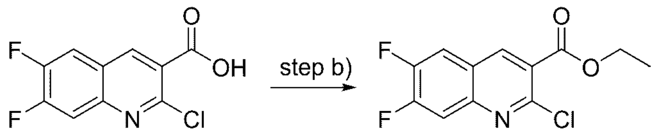


2-chloro-6,7-difluoroquinolin-3-carboxylic acid (1.0 g, 4.10 mmol) was dissolved in DMF (20 mL), then ethane iodide (1.28g, 8.21 mmol) and potassium carbonate (1.13 g, 8.21 mmol) were added and stirred at room temperature for 16 h. Water (100 mL) was added to dilute the reaction mixture, which was extracted with ethyl acetate (50 mL×3), and the combined organic phase was washed with saturated salt water (50 mL×2), dried with anhydrous sodium sulfate, and filtered. The filtrate was concentrated under reduced pressure, and the crude product was purified by normal silica gel column (petroleum ether/ethyl acetate (v/v) = 0% ~ 30%) to obtain white solid ethyl 2-chloro-6,7-difluoroquinolin-3-carboxylate (1.0 g, yield 89.29%).

ESI-MS(m/z): 281.2 $[M+H]^+$.

Step c) : Preparation of ethyl 2-(4,4-difluoroazepan-1-yl)-6,7-difluoroquinolin-3-carboxylate

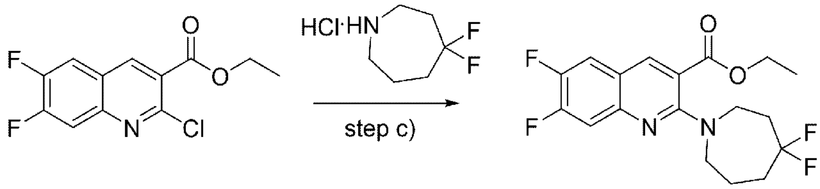


4-Difluoroazepane hydrochloride (0.81 g, 4.79 mmol) and DIEA (1.42 g, 11.04 mmol) were added to a solution of ethyl 2-chloro-6,7-difluoroquinolin-3-carboxylate (1.0 g, 3.68 mmol) in NMP (13 mL), and stirred at 140 °C for 16 h. After the reaction was completed, the reaction system was diluted with water (30 mL), extracted with ethyl acetate (20 mL×3), and the combined organic phase was washed with saturated salt water, dried with anhydrous sodium sulfate, and filtered, and the filtrate was concentrated under reduced pressure. The crude product was purified by normal silica gel column (petroleum ether/ethyl acetate (v/v) = 0 %~25 %) to obtain yellow oil ethyl 2-(4,4-difluoroazepan-1-yl)-6,7-difluoroquinolin-3-carboxylate (900 mg, yield 65.93 %). $^1H$ NMR (400 MHz, $CDCl_3$) delta 8.21 (s, 1H), 7.49-7.32 (m, 2H), 4.40 (q, $J$ = 7.1 Hz, 2H), 3.83-3.74 (m, 2H), 3.45 (t, $J$ = 5.6 Hz, 2H), 2.53-2.37 (m, 2H), 2.04-1.92 (m, 4H), 1.42 (t, $J$ = 7.1 Hz, 3H). ESI-MS(m/z): 371.2 $[M+H]^+$.

Step d) : Preparation of 2-(4,4-difluoroazepan-1-yl)-6,7-difluoroquinolin-3-carboxylic acid

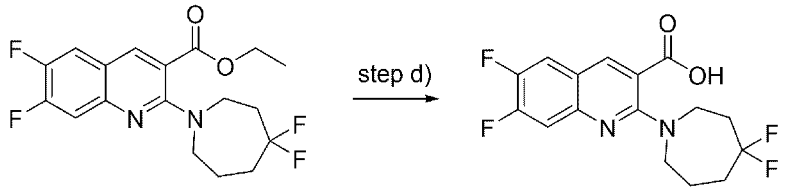


A mixture of methanol (10 mL), water (10 mL) and THF (10 mL) was first prepared, and ethyl 2-(4,4-difluoroazepan-1-yl)-6,7-difluoroquinolin-3-carboxylate (900 mg, 2.43 mmol) was added to the prepared mixture. Then lithium hydroxide (612.36 mg, 14.58 mmol) was added and stirred at 50 °C for 3 h. The pH of reaction mixture was adjusted to 6 with 1 M hydrochloric acid, and it was extracted with ethyl acetate (15 mL×3), then the combined organic phase was washed with saturated salt water (20 mL×2), dried with anhydrous sodium sulfate, and filtered. The filtrate was concentrated under reduced pressure to obtain light yellow solid 2-(4,4-difluoroazepan-1-yl)-6,7-difluoroquinolin-3-carboxylic acid(702.03 mg, yield 84.38%). $^1$H NMR (400 MHz, DMSO-$d_6$) δ13.42 (brs, 1H), 8.44 (s, 1H), 7.93 (dd, $J$ = 10.9, 9.2 Hz, 1H), 7.53 (dd, $J$ = 12.3, 7.6 Hz, 1H), 3.75-3.67 (m, 2H), 3.49 (2H, t, $J$ = 5.8 Hz), 2.46-2.33 (m, 2H), 2.08-1.86 (m, 4H). ESI-MS(m/z): 343.2 $[M+H]^+$.

step e): Preparation of methyl 4-(2-(4,4-difluoroazepan-1-yl)-6,7-difluoroquinoline-3- carboxamido) pyridine carboxylate

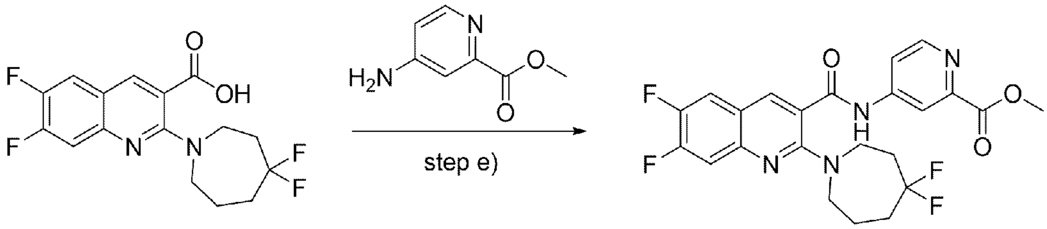


At room temperature, 2-(4,4-difluoroazepan-1-yl)-6,7-difluoroquinolin-3-carboxylic acid (300 mg, 0.88 mmol) was dissolved in dichloromethane (10 mL), then DMF (0.05 mL) was added, and oxalyl chloride (335.09 mg, 2.64 mmol) was slowly added. The resulting mixture was stirred at room temperature for 1 h. The solvent was removed by vacuum rotary evaporation and the resulting residue was dissolved in dichloromethane (5 mL). Then methyl 4-aminopyridine-2-carboxylate (401.68 mg, 2.64 mmol) and triethylamine (445.23 mg, 4.40 mmol) were added. The reaction mixture was stirred at room temperature for 16 h. After the reaction was completed, the reaction system was quenched with saturated sodium bicarbonate solution, extracted with dichloromethane (30 mL×3), and the combined organic phase was washed with saturated salt water, dried with anhydrous sodium sulfate, and filtered, and the filtrate was concentrated under reduced pressure. The resulting crude product was purified by normal silica gel column (petroleum ether/ethyl acetate (v/v) = 0 %~35 %) to obtain light yellow solid methyl 4-(2-(4,4-difluoroazepan-1-yl)-6,7-difluoroquinoline-3-carboxamido) pyridine carboxylate(152 mg, yield 36.4%). ESI-MS(m/z): 476.8 $[M+H]^+$.

step f): Preparation of N-(2-carbamoylpyridine-4-yl)-2-(4,4-difluoroazepan-1-yl)-6,7-difluoroquinoline-3-carboxamide (KH10)

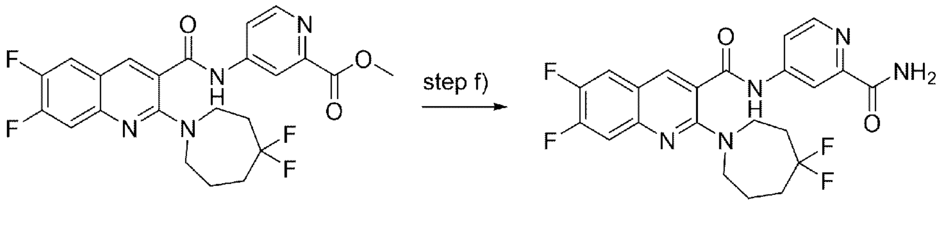


Ammonium chloride solid (5 mg, 0.09 mmol) and ammonia water (5 mL) were added to a solution of methyl 4-(2-(4,4-difluoroazepan-1-yl)-6,7-difluoroquinolin-3-carboxamido)pyridine carboxylate(150 mg, 0.315 mmol) in acetonitrile (5 mL) and react at 40 °C for 4 h, and water (20 mL) was added to dilute the reaction solution. The organic phase was extracted with ethyl acetate (35 mL×3), washed with saturated salt water, dried with anhydrous sodium sulfate, and filtered. The filtrate was concentrated under reduced pressure. The resulting crude product was purified by Prep-HPLC to obtain white solid N-(2-carbamoylpyridine-4-yl)-2-(4,4-difluoroazepan-1-yl)-6,7-difluoroquinoline-3-carboxamide (53.77 mg, yield 37.0%). $^1H$ NMR (400 MHz, DMSO-$d_6$) δ 11.26 (s, 1H), 8.56 (d, *J* = 5.5 Hz, 1H), 8.45-8.32 (m, 2H), 8.11 (d, *J* = 2.2 Hz, 1H), 7.97 7.85 (m, 2H), 7.66 (d, *J* = 2.2 Hz, 1H), 7.60 (dd, *J* = 12.2, 7.6 Hz, 1H), 3.74-3.71 (m, 2H), 3.55 (t, *J* = 5.8 Hz, 2H), 2.39-2.38 (m, 2H), 1.97-1.95 (m, 2H), 1.88-1.86 (m, 2H). ESI-MS(m/z): 462.3 [M+H]$^+$.

**Example 11**

Preparation of N-(3-carbamoyl-4-fluorophenyl)-3-(4,4-difluoroazepan-1-yl) quinoline-2-carboxamide (KH11)

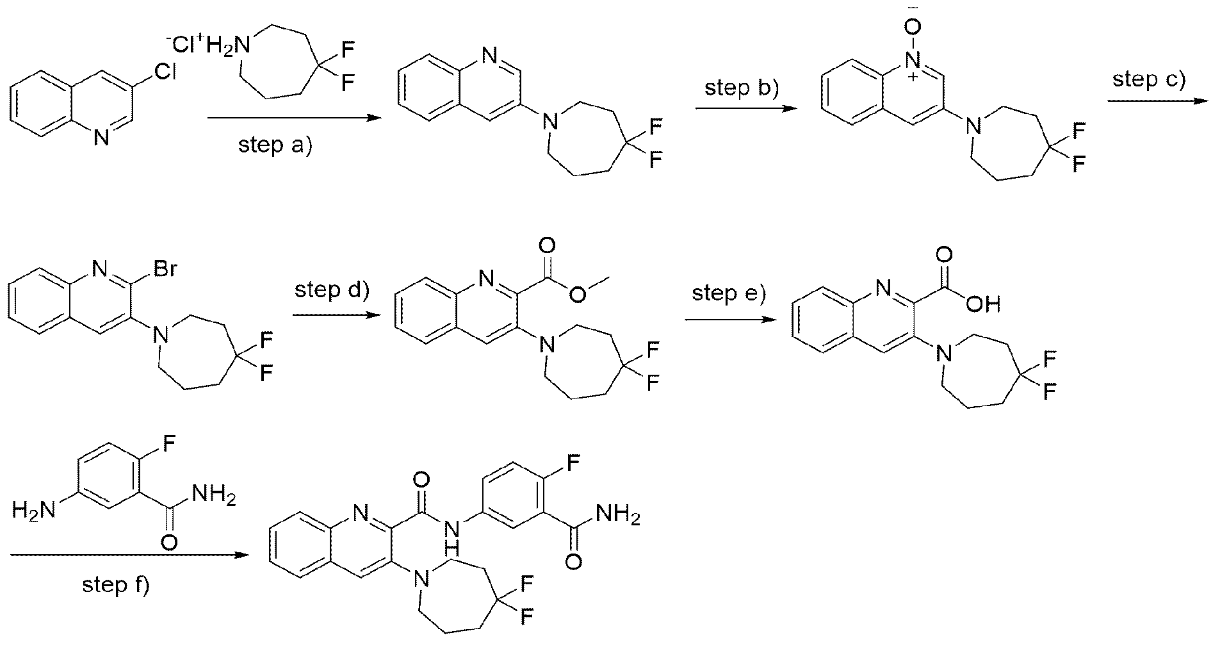


KH11

Step a): Preparation of 3-(4,4-difluoroazepan-1-yl) quinoline

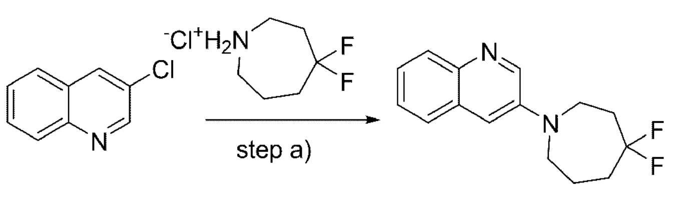


4,4-Difluoroazepane hydrochloride(4.49 g, 26.4 mmol), Ruphos-Pd-G2(0.513 g, 0.66 mmol) and potassium tert-butanol (7.4 g, 66 mmol) were added to a solution of 3-chloroquinoline (3.6 g, 22 mmol) in 1,4-dioxane (30 mL) and stirred at 105 °C for 16 h under the protection of nitrogen. After the reaction was completed, the reaction system was quenched with water (100 mL), extracted with ethyl acetate (50 mL × 3), and the combined organic phase was washed with saturated salt water (50 mL × 3), dried with anhydrous sodium sulfate, and filtered, and the filtrate was concentrated under reduced pressure. The crude product was purified by silica gel column chromatography (ethyl acetate/petroleum ether (v/v) = 1/20) to obtain yellow solid 3-(4,4-difluoroazepan-1-yl) quinoline (3 g, yield 51.7%). $^1H$ NMR (400 MHz, DMSO-$d_6$) δ 8.73 (d, *J* = 2.5 Hz, 1H), 7.82 (d, *J* = 8.1 Hz, 1H), 7.72 (d, *J* = 7.9 Hz, 1H), 7.45-7.28 (m, 3H), 3.68 (d, *J* = 4.9 Hz, 2H), 3.60 (2H, t, *J* = 5.8 Hz), 2.39-2.24 (m, 2H), 2.17-2.02 (m, 2H), 1.97-1.85 (m, 2H). ESI-MS(m/z): 263.2 [M+H]$^+$.

Step b): Preparation of 3-(4,4-difluoroazepan-1-yl) quinoline-oxide

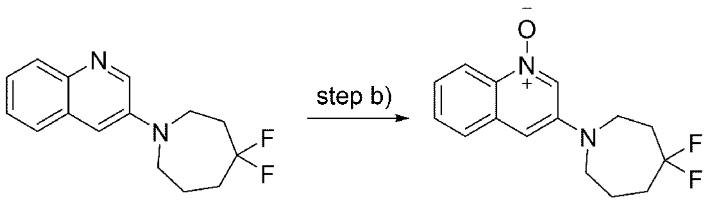


m-Chloroperoxybenzoic acid (3.4 g, 19.7 mmol) was slowly added to the solution of 3-(4,4-difluoroazepan-1-yl)quinoline(2.6 g, 9.88 mmol) in dichloromethane (40 mL) and reacted at 40 °C for 48 h. After the reaction was completed, the reaction system was quenched with water (50 mL), extracted with ethyl acetate (50 mL × 3), and the combined organic phase was washed twice with 10% sodium thiosulfate aqueous solution, and then dried with anhydrous sodium sulfate, filtered and the filtrate was concentrated under reduced pressure. The crude product was purified by silica gel column chromatography (ethyl acetate/petroleum ether (v/v) = 1/5) to obtain white solid 3-(4,4-difluoroazepan-1-1) quinoline-1-oxide (1.5 g, yield 55.5%). ESI-MS(m/z): 279.3 $[M+H]^+$.

Step c): Preparation of 2-bromo-3-(4,4-difluoroazepan-1-yl) quinoline

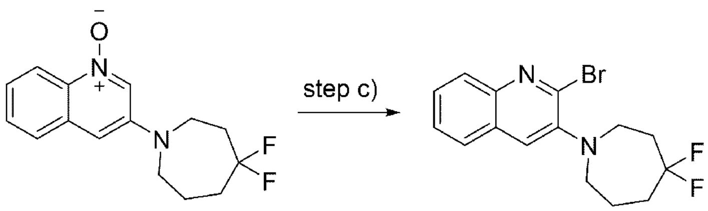


N-bromosuccinimide (1.9 g, 10.7 mmol) and triphenylphosphine (2.83 g, 10.7 mmol) were added to a solution of 3-(4,4-difluoroazepan-1-yl) quinoline-1-oxide (1.5 g, 5.37 mmol) in toluene (20 mL) and reacted at 115 °C for 2 h. After the reaction was completed, the reaction system was quenched with water (40 mL), extracted with ethyl acetate (30 mL× 3), and the combined organic phase was washed with saturated salt water (30 mL× 3), dried with anhydrous sodium sulfate, filtered, and the filtrate was concentrated under reduced pressure. The crude product was purified by silica gel column chromatography (ethyl acetate/petroleum ether (v/v) = 1/7) to obtain white solid 2-bromo-3-(4,4-difluoroazepan-1-1) quinoline (100 mg, yield 5.4%). $^1H$ NMR (400 MHz, DMSO - $d_6$) delta 7.95-7.85 (m, 2H), 7.61-7.55 (m, 3H), 3.29 (br s, 4H), 2.36-2.30 (m, 4H), 1.86 (br s, 2H). ESI-MS(m/z): 341.2 $[M+H]^+$.

Step d): Preparation of methyl 3-(4,4-difluoroazepan-1-yl) quinoline-2-carboxylate

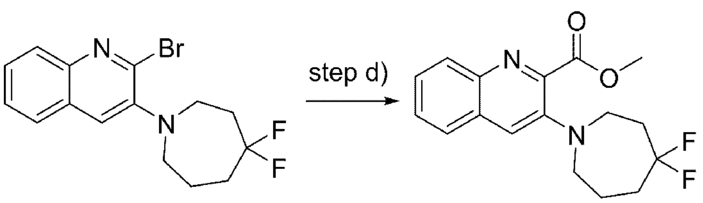


Triethylamine (88.8 mg, 0.879 mmol) and Pd(dppf)$Cl_2$ (23 mg, 0.0293 mmol) were added to a solution of 2-bromo-3-(4,4-difluoroazepan-1-yl)quinoline(100 mg, 0.293 mmol) in methanol (20 mL), and replaced gas with carbon monoxide three times, and the reaction system was reacted at 110 ° C in an autoclave(4 atm) for 16 hours. After the reaction was completed, the reaction system was quenched with water (40 mL), extracted with ethyl acetate (20 mL × 3), and the combined organic phase was washed with saturated salt water (30 mL), dried with anhydrous sodium sulfate,

filtered, and the filtrate was concentrated under reduced pressure. The crude product was purified by silica gel column chromatography (ethyl acetate/petroleum ether (v/v) = 1/5) to obtain yellow solid methyl 3-(4,4-difluoroazepan-1-yl) quinoline-2-carboxylate (55 mg, yield 58%). $^1$H NMR (400 MHz, DMSO-$d_6$) δ 7.98-7.84 (m, 3H), 7.63-7.45 (m, 2H), 3.939 (s, 3H), 3.40-3.34 (m, 4H), 2.33-2.23 (m, 2H), 2.21-2.14 (m, 2H), 1.91-1.83 (m, 2H). ESI-MS(m/z): 321.3 [M+H]$^+$.

Step e): Preparation of 3-(4,4-difluoroazepan-1-yl) quinoline-2-carboxylic acid

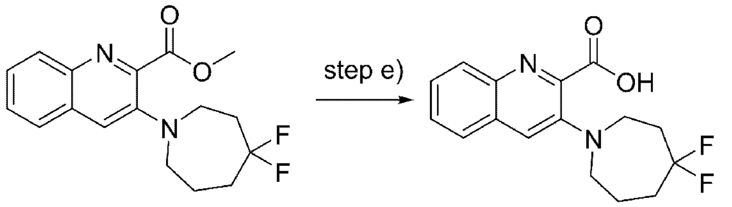


Lithium hydroxide (25 mg, 0.601 mmol) was slowly added to a mixture of methyl 3-(4,4-difluoroazepan-1-yl) quinoline-2-carboxylate (55 mg, 0.178 mmol) in water (0.5 mL) and tetrahydrofuran (2 mL), and reacted at room temperature for 1 h. After the reaction was completed, the system was was quenched with ice water, and 1 M of diluted hydrochloric acid was added to adjust the pH to 7. Then it was extracted with ethyl acetate (10 mL × 3), and the combined organic phase was washed with saturated salt water, dried with anhydrous sodium sulfate, filtered, and the filtrate was concentrated under reduced pressure to obtain yellow solid 3-(4,4-difluoroazepan-1-yl) quinoline-2-carboxylic acid(40 mg, yield 76%). ESI-MS(m/z): 307.1 [M+H]$^+$.

step f): Preparation of N-(3-carbamoyl-4-fluorophenyl)-3-(4,4-difluoroazepan-1-yl) quinoline-2-carboxamide (KH11)

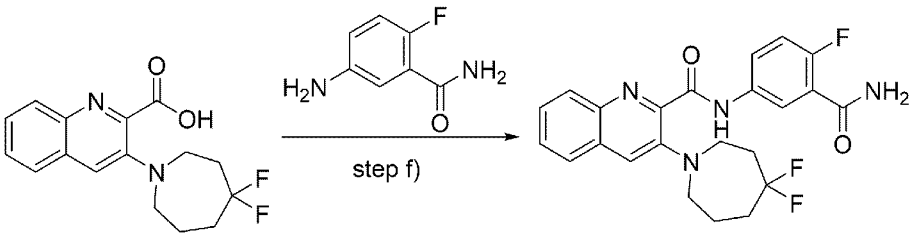


HATU (60 mg, 0.1302 mmol), 5-amino-2-fluorobenzamide(20 mg, 0.1302 mmol) and triethylamine (39 mg, 0.3908 mmol) were added to a solution of 3-(4,4-difluoroazepan-1-yl) quinoline-2-carboxylic acid (40 mg, 0.1302 mmol) in dichloromethane (2 mL) and reacted at room temperature for 2 h. After the reaction was completed, the reaction system was quenched with water (20 mL), extracted with ethyl acetate (10 mL × 3), and the combined organic phase was washed with saturated salt water (10 mL), dried with anhydrous sodium sulfate, filtered, and the filtrate was concentrated under reduced pressure. The crude product was purified by Prep-HPLC to obtain a solid powder N-(3-carbamoyl-4-fluorophenyl)-3-(4,4-difluoroazepan-1-yl) quinoline-2-carboxamide (3.10 mg, yield 5.3%). $^1$H NMR (400 MHz, MeOD) δ 8.16 (dd, *J* = 6.4, 2.7 Hz, 1H), 8.05-7.97 (m, 2H), 7.96 (s, 1H), 7.86 (d, *J* = 8.3 Hz, 1H), 7.65-7.55 (m, 2H), 7.32-7.24 (m, 1H), 3.49-3.43 (m, 4H), 2.35-2.24 (m, 2H), 2.17-2.01 (m, 2H), 1.98-1.90 (m, 2H). ESI-MS(m/z): 443.2 [M+H]$^+$.

**Example 12**

Preparation of N-(3-carbamoylphenyl)-2-(4,4-difluoroazepan-1-yl)-6,7-difluoroquinoline-3- carboxamide (KH12)

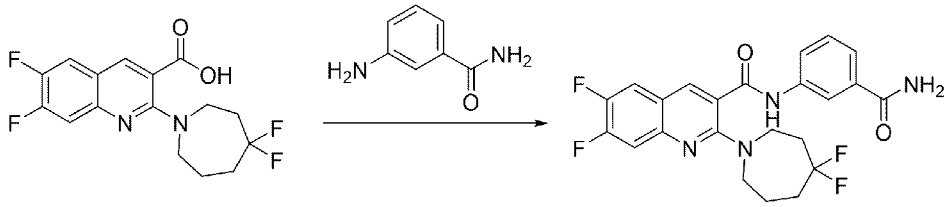

KH12

2-(4,4-difluoroazepan-1-yl)-6,7-difluoroquinolin-3-carboxylic acid was prepared the same as in step a-d of example 10.

(4,4-Difluoroazepan-1-yl)-6,7-difluoroquinolin-3-carboxylic acid(100 mg, 0.29 mmol) was dissolved in DMF (2 mL), then 3-aminobenzoamide (43.75 mg, 0.32 mmol), HATU (113.3 mg, 0.35 mmol) and DIEA (113.27 mg, 0.87 mmol) were added and stirred at room temperature for 4 h. Water (20 mL) was added to dilute the reaction system, then it was extracted with ethyl acetate (10 ml × 3), washed with saturated salt water (10 ml × 2), and the organic phase was dried with anhydrous sodium sulfate and concentrated. White solid N-(3-carbamoylphenyl)-2-(4,4-difluoroazepan-1-yl)-6,7-difluoroquinoline-3-carboxamide was prepared by Prep-HPLC (3.2 mg, yield 2.37%). $^1H$ NMR (400 MHz, DMSO-$d_6$) δ 10.80 (s, 1H), 8.31 (s, 1H), 8.19 (s, 1H), 8.01-7.88 (m, 2H), 7.85 (d, *J* = 8.0 Hz, 1H), 7.62 (d, *J* = 7.8 Hz, 2H), 7.43-7.40 (m, 2H), 3.82-3.67 (m, 2H), 3.61 (2H, t, *J* = 5.9 Hz), 2.36-2.32 (m, 2H), 2.00 (brs, 2H), 1.89 (brs, 2H). ESI-MS(m/z): 461.3 $[M+H]^+$.

**Example 13**

Preparation of 2-(4,4-difluoroazepan-1-yl)-N-(3-sulfamoylphenyl)quinoline-3-carboxamide (KH13)

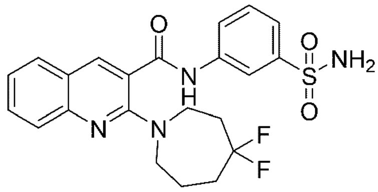

KH13

The preparation of 3-(4,4-difluoroazepan-1-yl)-N-(3-sulfamoylphenyl)quinoline-3-carboxamide is similar to step a-d of example 8 and example 6, except that the 3-(4,4- difluoroazepan-1-yl)quinoline-2-carboxylic acid in step f of Example 6 is substituted with the 2-(4,4- difluoroazepan-1-yl)quinoline-3-carboxylic acid synthesized through steps a-d of Example 8, and the 4-bromo-2-fluoropyridine in step a of Example 6 is replaced with 4-bromo-2-fluorobenzene, and change the room temperature of step e in Example 6 to 80 °C, resulting in a white solid 2-(4,4- difluoroazepan-1-yl)-N-(3-sulfamoylphenyl)quinoline-3-carboxamide (22.12 mg, yield 30.56%). $^1H$ NMR (400 MHz, DMSO-d6) δ 10.96 (s, 1H), 8.33 (s, 2H), 7.93-7.75 (m, 2H), 7.64 (d, *J* = 3.6 Hz, 2H), 7.57 (dd, *J* = 8.0, 5.5 Hz, 2H), 7.42 (s, 2H), 7.35-7.25 (m, 1H), 3.83-3.70 (m, 2H), 3.61 (t, *J* = 5.9 Hz, 2H), 2.43-2.40 (m, 2H), 2.00-1.95 (m, 2H), 1.90 (br s, 2H). ESI-MS(m/z): 461.1 [M+H]+.

**Example 14**

Preparation of 2-(4,4-difluoroazepan-1-yl)-N-(2-sulfamoylpyridine-4-yl) quinoline-3-carboxamide (KH14)

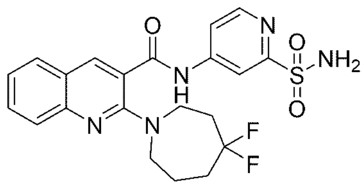

KH14

The preparation of 2-(4,4-difluoroazepan-1-yl)-N-(2-sulfamoylpyridine-4-yl)quinoline-3- carboxamide is similar to step a-d of Example 8 and Example 6, except that the 3-(4,4-difluoroazepan-1-yl)quinoline-2-carboxylic acid in step f of Example 6 is substituted with 2-(4,4-difluoroazepan-1-yl)quinoline-3-carboxylic acid synthesized through steps a-d of Example 8. The room temperature in step e of Example 6 is changed to 80 °C, resulting in the white solid 2-(4,4-difluoroazepan-1-yl)-N-(2-sulfamoylpyridine-4-yl) quinoline-3-carboxamide (7.49 mg, yield 11.4%). $^1$H NMR (400 MHz, DMSO-$d_6$) $\delta$ 11.34 (s, 1H), 8.63 (d, *J* = 5.4 Hz, 1H), 8.41 (s, 1H), 8.34 (d, *J* = 1.7 Hz, 1H), 7.90-7.81 (m, 2H), 7.65 (d, *J* = 3.6 Hz, 2H), 7.47 (s, 2H), 7.37-7.27 (m, 1H), 3.77-3.69 (m, 2H), 3.55 (t, *J* = 5.9 Hz, 2H), 2.43-2.40 (m, 2H), 1.98-1.95 (m, 2H), 1.89 (br s, 2H). ESI-MS(m/z): 462.1 $[M+H]^+$.

**Example 15**

Preparation of N-(2-carbamoylpyridin-4-yl)-3-(4,4-difluoroazepan-1-yl) quinoline-2-carboxamide (KH15)

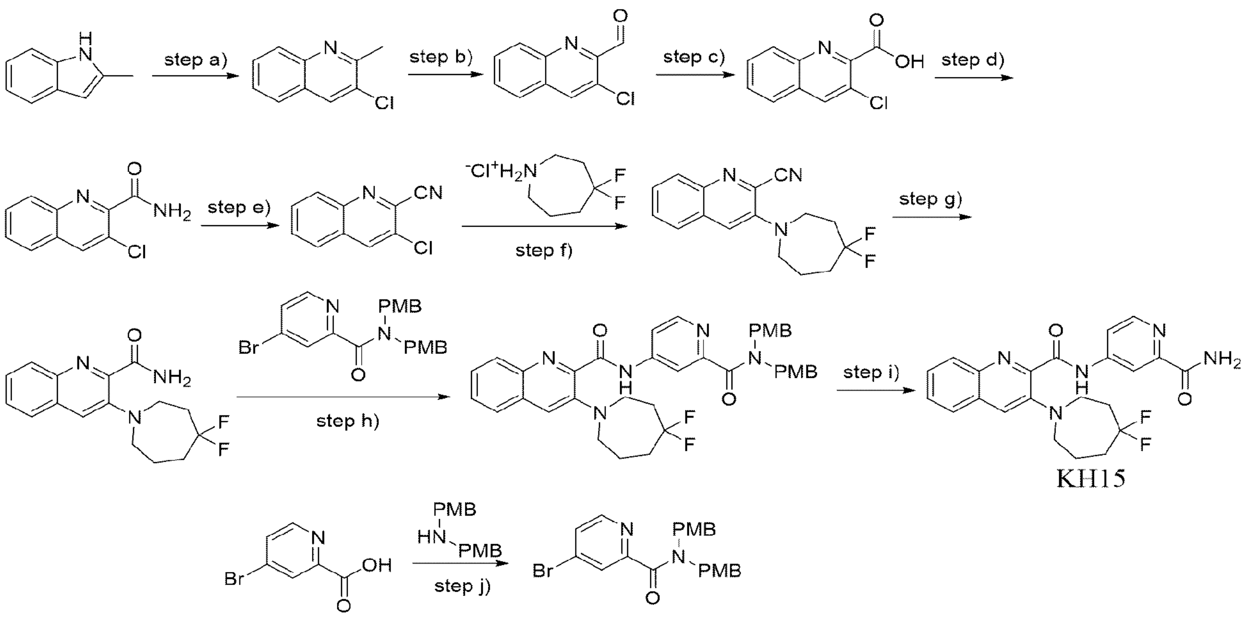


KH15

Step a): Preparation of 3-chloro-2-methylquinoline

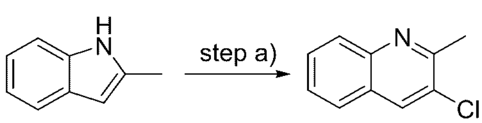


The solution of benzyltriethylammonium chloride (1.74 g, 7.63 mmol) in chloroform (300 mL) was slowly added to a solution of 2-methyl-1H-indole(10 g, 76.3 mmol) in 50% KOH (32 mL) in a 0 °C ice bath, and stirred at room temperature for 16 hours. After the reaction was completed, it was quenched with water (200 mL), extracted with dichloromethane (200 mL × 3), and the combined organic phase was washed with saturated salt water (80 mL × 3),

dried with anhydrous sodium sulfate, filtered, and concentrated under reduced pressure. The resulting crude product was purified by silica gel column chromatography (ethyl acetate/petroleum ether (v/v)=1/20) to obtain yellow solid 3-chloro-2-methylquinoline (2.1 g, yield 15%). $^1$H NMR (400 MHz, DMSO-$d_6$) δ 8.55 (s, 1H), 8.06-7.94 (m, 2H), 7.80-7.78 (m, 1H), 7.71-7.56 (m, 1H), 2.77 (s, 3H). ESI-MS(m/z): 178.1 [M+H]$^+$.

Step b): Preparation of 3-chloroquinoline-2-carbaldehyde

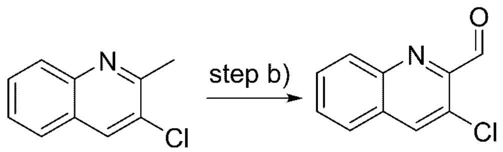


Selenium dioxide (3.76 g, 33.8 mmol) was slowly added to a solution of 3-chloro-2-methylquinoline (2 g, 11.2 mmol) in 1,4-dioxane (40 mL) and reacted at 100 °C for 2 hours. After the reaction was completed, it was cooled to room temperature, then filtered, and the filtrate was concentrated under reduced pressure to obtain a white solid 3-chloroquinoline-2-carbaldehyde (2.1 g, yield 97%). ESI-MS(m/z): 192.1 [M+H]$^+$.

Step c): Preparation of 3-chloroquinoline-2-carboxylic acid

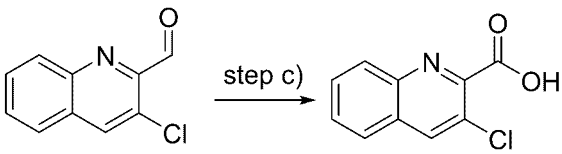


A mixed aqueous solution of sodium chlorite(4.7 g, 52.3 mmol) and disodium hydrogen phosphate (7.4 g, 52.3 mmol) were slowly added to a solution of 3-chloroquinoline-2-carbaldehyde (2 g, 10.47 mmol) in tert-butanol (20 mL) in a 0 °C ice bath (36 mL) and stirred at room temperature for 16 hours. After the reaction was completed, the pH was adjusted to neutral with 1 M hydrochloric acid aqueous solution, then the organic phase was extracted with ethyl acetate (100 mL × 3), washed with saturated salt water (80 mL), dried with anhydrous sodium sulfate, filtered, and the filtrate was concentrated under reduced pressure to obtain a white solid 3-chloroquinoline-2-carboxylic acid(1.9 g, yield 88%). $^1$H NMR (400 MHz, DMSO-$d_6$) δ 8.79 (s, 1H), 8.11 (dd, $J$ = 19.2, 8.3 Hz, 2H), 7.93 (dd, $J$ = 11.2, 4.1 Hz, 1H), 7.80 (t, $J$ = 7.5 Hz, 1H). ESI-MS(m/z): 208.1 [M+H]$^+$.

Step d): Preparation of 3-chloroquinoline-2-carboxamide

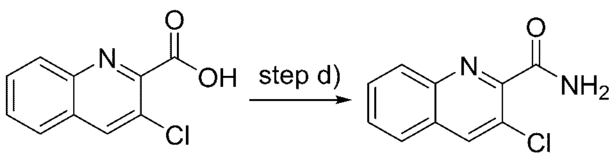


Ammonium chloride (231.5 mg, 4.33 mmol), HATU (657.7mg, 1.73 mmol) and N,N-diisopropylethylamine (558 mg, 4.33 mmol) were added to a solution of 3-chloroquinolin-2-carboxylic acid (300 mg, 1.44 mmol) in DMF (3 mL) and reacted at room temperature for 4 h. After the reaction was completed, the reaction system was quenched with water (20 mL), extracted with ethyl acetate (30 mL × 3), and the combined organic phase was washed with saturated salt water (30 mL × 2), dried with anhydrous sodium sulfate, filtered, and concentrated under reduced pressure. The crude product was purified by silica gel column chromatography (methanol/dichloromethane (v/v) = 1/10) to obtain white solid 3-chloroquinoline-2-carboxamide (180 mg, 60% yield). $^1$H NMR (400 MHz, DMSO-$d_6$) δ 8.68 (s, 1H), 8.17 (s, 1H), 8.07 (d, $J$ = 8.5 Hz, 1H), 8.02 (d, $J$ = 8.2 Hz, 1H), 7.86 (t, $J$ = 7.4 Hz, 2H), 7.73 (t, $J$ = 7.5 Hz, 1H). ESI-MS(m/z): 207.3 [M+H].$^+$

Step e): Preparation of 3-chloroquinoline-2-carbonitrile

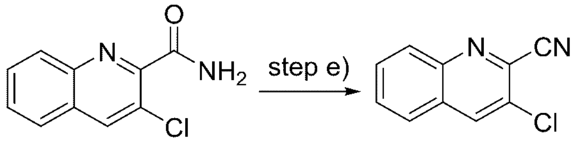


Triethylamine (220 mg, 2.17 mmol) and trifluoroacetic anhydride (220 mg, 1.04 mmol) were added to a solution of 3-chloroquinolin-2-carboxamide (180 mg, 0.87 mmol) in tetrahydrofuran (5 mL) under a 0 °C ice bath, and continually stirred under the ice bath for 1 h. After the reaction was completed, the reaction system was quenched with water (10 mL), extracted with ethyl acetate (20 mL × 3), and the combined organic phase was washed with saturated salt water (20 mL × 3), dried with anhydrous sodium sulfate and filtered, and the filtrate was concentrated under reduced pressure. The crude product was purified by silica gel column chromatography (ethyl acetate/petroleum ether (v/v) = 1/10) to obtain yellow solid 3-chloroquinoline-2-carbonitrile (100 mg, yield 61%). $^1$H NMR (400 MHz, DMSO-$d_6$) δ 8.93 (s, 1H), 8.16 (d, *J* = 8.4 Hz, 1H), 8.10 (d, *J* = 8.1 Hz, 1H), 7.97 (t, *J* = 7.6 Hz, 1H), 7.87 (t, *J* = 7.5 Hz, 1H). ESI-MS(m/z): 189.0 [M+H].$^+$

Step f): Preparation of 3-(4, 4-difluoroazepan-1-yl) quinoline-2-carbonitrile

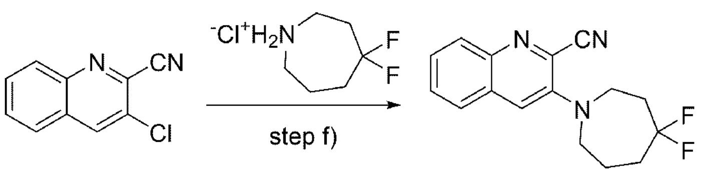


4,4-Difluoroazepane hydrochloride (400 mg, 2.34 mmol), Ruphos-Pd-G2 (140 mg, 0.179 mmol) and cesium carbonate (1750 mg, 5.4 mmol) were added to the solution of 3-chloroquinolin-2-carbonitrile (340 mg, 1.80 mmol) in toluene (8 mL) and stirred at 110 °C for 16 h under the protection of nitrogen. After the reaction was completed, the reaction system was quenched with water (20 mL), extracted with ethyl acetate (20 mL × 3), and the combined organic phase was washed with saturated salt water (20 mL), dried with anhydrous sodium sulfate, filtered, and the filtrate was concentrated under reduced pressure. The crude product was purified by silica gel column chromatography (ethyl acetate/petroleum ether (v/v) = 1/5) to obtain yellow oil 3-(4, 4-difluoroazepan-1-yl) quinoline-2-carbonitrile (200 mg, yield 48%). ESI-MS(m/z): 228.1 [M+H].$^+$

Step g): Preparation of 3-(4,4-difluoroazepan-1-yl) quinoline-2-carboxamide

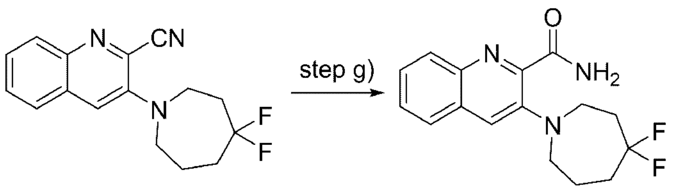


Cesium carbonate (437 mg, 1.315 mmol) was added to a solution of 3-(4,4-difluoroazepan-1-yl)quinoline-2-carbonitrile (200 mg, 0.877 mmol) in dimethyl sulfoxide (3 mL), and 30% hydrogen peroxide (0.5 mL) was slowly added at 0 °C. The reaction was carried out under ice bath for 2 h. After the reaction was completed, ice water (10 mL) was added to quench the reaction, which was extracted with ethyl acetate (10 mL × 3), and the combined organic phase was washed with saturated salt water (20 mL), dried with anhydrous sodium sulfate, and filtered. Filtrate was concentrated under reduced pressure to yellow solid 3-(4,4-difluoroazepan-1-yl) quinoline-2-carboxamide (120 mg, yield 45%). ESI-MS(m/z): 306.3 [M+H].$^+$

step h): Preparation of N-(2-(bis(4-methoxybenzyl)carbamoyl)pyridin-4-yl)-3-(4,4-difluoroazepan-1-yl)quinoline-2-carboxamide

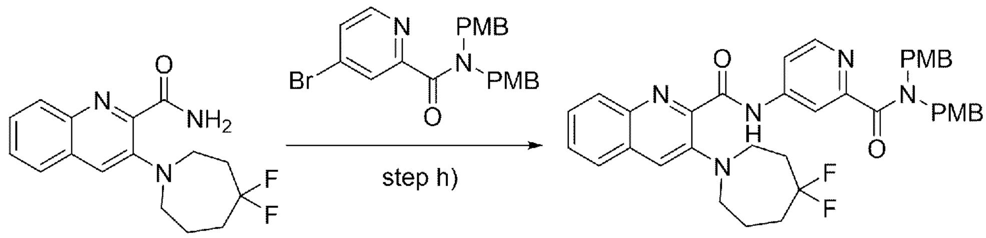


3-(4,4-Difluoroazepan-1-yl)quinoline-2-carboxamide (120 mg, 0.393 mmol), Ruphos-Pd-G2 (30.6 mg, 0.0393 mmol) and cesium carbonate (320 mg, 2.5 mmol) were added to the solution of 4-bromo-N,N-bis(4-methoxybenzyl)pyridine-2-carboxamide (208.7 mg, 0.472 mmol) in 1,4-dioxane (4 mL) and stirred at 105 °C for 16 h under nitrogen protection. After the reaction was completed, the reaction system was quenched with water (10 mL), extracted with ethyl acetate (15 mL × 3), and the combined organic phase was washed with saturated salt water (10 mL × 3), dried with anhydrous sodium sulfate, filtered, and the filtrate was concentrated under reduced pressure. The resulting crude product was purified by silica gel column chromatography (ethyl acetate/petroleum ether (v/v) = 1/1) to obtain yellow solid N-(2-(bis(4-methoxybenzyl)carbamoyl)pyridine-4-yl)-3-(4,4-difluoroazepan-1-yl)quinoline-2-carboxamide (180 mg, yield 65%). ESI-MS(m/z): 666.5 $[M+H]^+$.

step i): Preparation of N-(2-carbamoylpyridine-4-yl)-3-(4,4-difluoroazepan-1-yl) quinoline-2- carboxamide

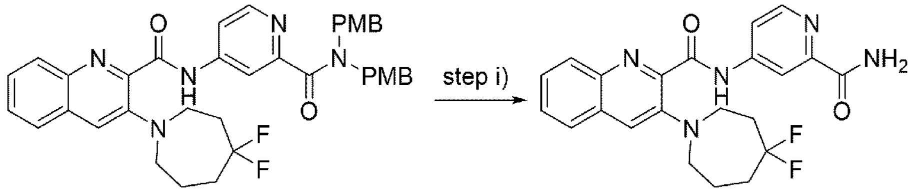


Trifluoroacetic acid solution (5 mL) and a drop of trifluoromethanesulfonic acid were added to the solution of N-(2-(bis(4-methoxybenzyl)carbamoyl)pyridine-4-yl)-3-(4,4-difluoroazepan-1-yl)quinoline-2-carboxamide (170 mg, 0.255 mmol) in 1,2-dichloroethane (3 mL) and reacted at 70 °C for 1 h. After the reaction was completed, the reaction mixture was concentrated under reduced pressure, the pH of the resulting mixture was adjusted to neutral with saturated sodium bicarbonate aqueous solution, then it was extracted with ethyl acetate (15 mL × 3), and the combined organic phase was washed with saturated salt water (10 mL), dried with anhydrous sodium sulfate, filtered, and the filtrate was concentrated under reduced pressure. The resulting crude product was prepared by Prep-HPLC to yellow solid N-(2-carbamoylpyridine-4-yl)-3-(4,4-difluoroazepan-1-yl)quinoline-2-carboxamide (15 mg, yield 14%). $^1$H NMR (400 MHz, DMSO-$d_6$) δ 11.33 (s, 1H), 8.58 (d, *J* = 5.5 Hz, 1H), 8.44 (d, *J* = 1.9 Hz, 1H), 8.12 (s, 1H), 8.00 7.96 (m, 1H), 7.96 (d, *J* = 2.3 Hz, 1H), 7.95-7.93 (m, 1H), 7.92 (dd, *J* = 4.6, 3.3 Hz, 1H), 7.67 (s, 1H), 7.64-7.57 (m, 2H), 3.43-3.38 (m, 4H), 2.26-2.23 (m, 2H), 2.13-2.09 (m, 2H), 1.90-1.81 (m, 2H). ESI-MS(m/z): 426.4 $[M+H]^+$.

Step j): Preparation of 4-bromo-N,N-bis (4-methoxybenzyl) pyridine-2-carboxamide

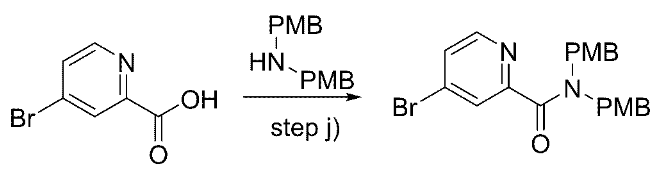

Bis-(4-methoxybenzyl)-amine (636 mg, 2.47 mmol), HATU (1130 mg, 2.97 mmol) and N, N-diisopropylethylamine (638 mg, 4.95 mmol) were added to a solution of 4-bromopyridine carboxylic acid (500 mg, 2.47 mmol) in N,N-dimethylformamide (6 mL) and reacted at room temperature for 16 h. After the reaction was completed, the reaction system was quenched with water (20 mL), extracted with ethyl acetate (20 mL × 3), and the combined organic phase was washed with saturated salt water (20 mL), dried with anhydrous sodium sulfate, filtered, and the filtrate was concentrated under reduced pressure. The crude product was purified by silica gel column chromatography (ethyl acetate/petroleum ether (v/v) =1/4) to obtain transparent oil 4-bromo-N,N-bis (4-methoxybenzyl) pyridine-2-carboxamide (500 mg, yield 45%). $^1$H NMR (400 MHz, DMSO-$d_6$) δ 8.49 (d, *J* = 5.4 Hz, 1H), 7.90 (d, *J* = 1.8 Hz, 1H), 7.77 (dd, *J* = 5.3, 1.9 Hz, 1H), 7.23 (d, *J* = 8.6 Hz, 2H), 7.14 (d, *J* = 8.6 Hz, 2H), 6.94 (2H, t, *J* = 5.7 Hz), 6.89 (d, *J* = 8.7 Hz, 2H), 4.48 (s, 2H), 4.34 (s, 2H), 3.76 (s, 3H), 3.74 (s, 3H). ESI-MS(m/z): 443.1 $[M+H]^+$.

**Example 16**

Preparation of 2-(4,4-difluoroazepan-1-yl)-6,7-difluoro-N-(3-sulfamoylphenyl) quinoline-3- carboxamide (KH16)

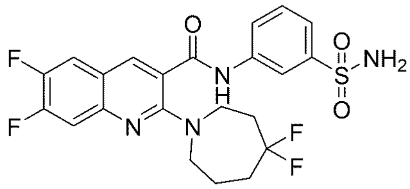

KH16

The preparation of 2-(4,4-difluoroazepan-1-yl)-6,7-difluoro-N-(3-sulfamoylphenyl) quinoline-3-carboxamide is similar to that of Example 10 step a-d and Example 6, except that 3-(4,4-difluoroazepan-1-yl)quinoxaline-2-carboxylic acid in step f of example 6 is changed into 2-(4,4-difluoroazepan-1-yl)-6,7-difluoroquinolin-3-carboxylic acid which can be synthesized by step a-d of example 10, and 4-bromo-2-fluoropyridine in step a of example 6 is replaced by 4-bromo-2-fluorobenzene, and the room temperature at step e of example 6 was changed to 80 °C to obtain a white solid 2-(4, 4-difluoroazepan-1-yl)-6,7-difluoro-N-(3-sulfamoylphenyl)quinoline-3-carboxamide (10.92 mg, yield 17.5%). $^1$H NMR (400 MHz, MeOD) δ 8.36 (s, 1H), 8.26 (s, 1H), 7.86 (d, *J* = 8.1 Hz, 1H), 7.67 (dd, *J* = 19.6, 10.0 Hz, 2H), 7.60-7.43 (m, 2H), 3.87-3.78 (m, 2H), 3.66 (2H, t, *J* = 5.9 Hz), 2.42-2.40 (m, 2H), 2.08-2.00 (m, 2H), 1.99-1.91 (m, 2H). ESI-MS(m/z): 497.1 $[M+H]^+$.

**Example 17**

Preparation of 2-(4,4-difluoroazepan-1-yl)-6,7-difluoro-N-(2-sulfamoylpyridine-4-yl) quinoline-3-carboxamide (KH17)

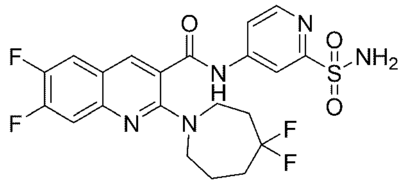

KH17

The synthesis of 2-(4,4-difluoroazepan-1-yl)-6,7-*difluoro-N*-(2-sulfamoylpyridine-4-yl) quinoline-3-carboxamide is similar to step a-d of Example 10 and Example 6, except that 3-(4,4-difluoroazepan-1-yl)quinoxaline-2-carboxylic acid in step f of Example 6 is changed into 2-(4,4-difluoroazepan-1-yl)-6, 7-difluoroquinolin-3-carboxylic acid which can be synthesized by step a-d of example 10, and stirring at room temperature for 2 h in step e of example 6 is changed to stirring at room temperature for 5 h. Yellow solid 2-(4,4-difluoroazepan-1-yl)-6,7-difluoro-N-(2-sulfamoylpyridine-4-yl)quinoline-3-carboxamide (13.9 mg, 24% yield) was obtained. $^1$H NMR (400 MHz, DMSO-$d_6$) δ 11.38 (s, 1H), 8.64 (d, *J* = 5.3 Hz, 1H), 8.41 (s, 1H), 8.32 (s, 1H), 7.92 (t, *J* = 10.0 Hz, 1H), 7.86 (d, *J* = 5.4 Hz, 1H), 7.60 (dd, *J* = 11.8, 7.9 Hz, 1H),

7.47 (s, 2H), 3.73 (s, 2H), 3.54 (s, 2H), 2.40 (s, 2H), 1.99 (s, 2H), 1.89 (s, 2H). ESI-MS(m/z): 498.2 [M+H]$^+$.

**Example 18**

Preparation of N-(2-carbamoylpyridin-4-yl)-7-(4,4-difluoroazepan-1-yl)quinoline-6- carboxamide (KH18)

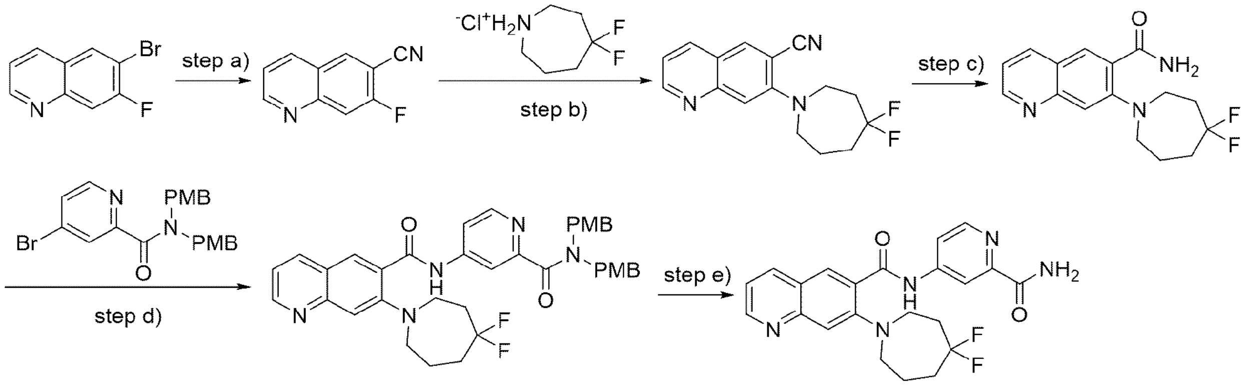


KH18

Step a): Preparation of 7-fluoroquinoline-6-carbonitrile

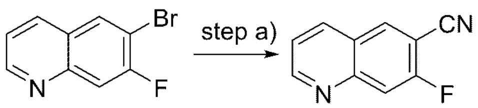


$Zn(CN)_2$ (2.07 g, 17.68 mmol) and Pd $(PPh_3)_4$(2.04 g, 1.77 mmol) were added to a solution of 6-bromo-7-fluoroquinoline (2 g, 8.84 mmol) in NMP (20 mL) and the reaction system was stirred at 120 °C for 16 h under nitrogen protection. After the reaction was completed, it was diluted with water, extracted with ethyl acetate (20 mL × 3), and the combined organic phase was washed with saturated salt water, dried with anhydrous sodium sulfate, filtered, and the filtrate was concentrate under reduced pressure. The resulting crude product was purified by silica gel column chromatography (petroleum ether/ethyl acetate (v/v) = 1/1) to obtain a light yellow solid 7-fluoroquinoline-6-carbonitrile (1.2g, yield 78.8%). $^1$H NMR (400 MHz, $CDCl_3$) δ 9.06 (dd, *J* = 4.3, 1.5 Hz, 1H), 8.24 (dd, *J* = 11.7, 3.8 Hz, 2H), 7.88 (d, *J* = 10.0 Hz, 1H), 7.53 (dd, *J* = 8.4, 4.3 Hz, 1H). ESI-MS(m/z): 173.0 [M+H]$^+$.

Step b): Preparation of 7-(4, 4-difluoroazepan-1-yl) quinoline-6-carbonitrile

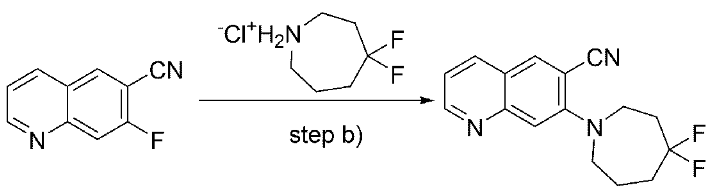


4,4-Difluoroazepane hydrochloride (4.7 g, 34.85 mmol), $K_2CO_3$ (4.8 g, 34.85 mmol) and DIEA (4.5 g, 34.85 mmol) were added to a solution of 7-fluoroquinolin-6-carbonitrile (1.2 g, 6.97 mmol) in DMSO (10 mL) respectively and stirred at 100 ° C for 16 h. After the reaction was completed, the reaction system was diluted with water, extracted with ethyl acetate (20 mL × 3), and the combined organic phase was washed with saturated salt water, dried with anhydrous sodium sulfate, filtered, and the filtrate was concentrated under reduced pressure. The resulting crude product was purified by silica gel column chromatography (petroleum ether/ethyl acetate (v/v) = 3/1) to obtain light yellow solid 7-(4,

4-difluoroazepan-1-1)quinoline-6-carbonitrile (800 mg, yield 24.1%). $^1$H NMR (400 MHz, DMSO - $d_6$) delta 8.99 8.84 (m, 1H), 8.54 (s, 1H), 8.30 (d, *J*= 8.2 Hz, 1H), 7.45 (s, 1H), 7.41 (dd, *J*= 8.2, 4.2 Hz, 1H), 3.65-3.48 (m, 4H), 2.50-2.37 (m, 2H), 2.32-2.14 (m, 2H), 2.06-1.88 (m, 2H). ESI-MS(m/z): 288.0 [M+H]$^+$.

Step c) : Preparation of 7-(4,4-difluoroazepan-1-yl)quinoline-6-carboxamide

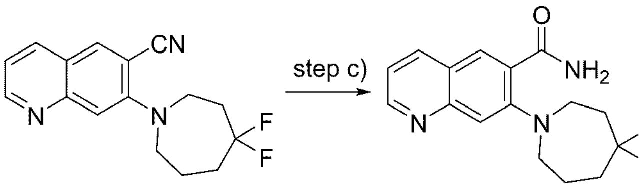


Cesium carbonate (3.16g, 9.72 mmol) was added to a solution of 7-(4, 4-difluoroazepan-1-yl) quinoline-6-carbonitrile (800 mg, 2.77 mmol) in dimethyl sulfoxide (10 mL), and 30% hydrogen peroxide (2.5 mL) was slowly added at 0 °C. Reaction was performed at room temperature for 4 h. After the reaction was completed, the reaction system was quenched with ice water (10 mL), extracted with ethyl acetate (60 mL × 2), and the combined organic phase was washed with saturated salt water (60 mL), dried with anhydrous sodium sulfate, and filtered. The filtrate was concentrated under reduced pressure to obtain white solid 7-(4,4-difluoroazepan-1-yl) quinoline-6-carboxamide (80 mg, yield 95%). ESI-MS(m/z): 306.1 [M+H]$^+$.

step d): Preparation of N-(2-(bis(4-methoxybenzyl)carbamoyl)pyridin-4-yl)-7-(4,4-difluoroazepan-1-yl)quinoline-6-carboxamide

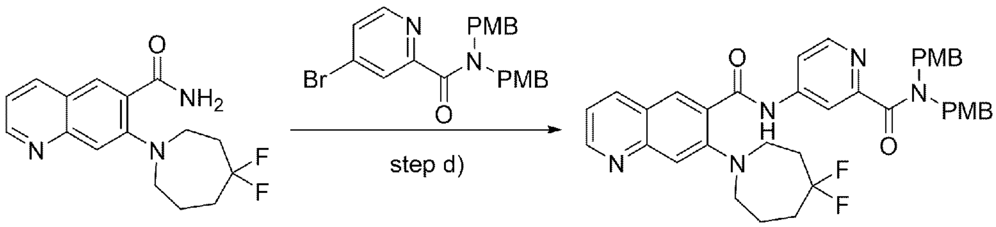


For the synthesis of 4-bromo-N,N-bis(4-methoxybenzyl)pyridine-2-carboxamide, see step j of example 15.

4-Bromo-N,N-bis(4-methoxybenzyl)pyridine-2-carboxamide (530 mg, 1.19 mmol), Brettphos-Pd-G3 (98.7 mg, 0.109 mmol) and cesium carbonate (880 mg, 2.72 mmol) were added to a solution of 7-(4, 4-difluoroazepan-1-yl)quinoline-6-carboxamide (350 mg, 1.06 mmol) in toluene (5 mL) and stirred at 110 °C for 16 h under nitrogen protection. After the reaction was completed, the reaction system was quenched with water (50 mL), extracted with ethyl acetate (15 mL × 3), and the combined organic phase was washed with saturated salt water (10 mL × 3), dried with anhydrous sodium sulfate, filtered, and the filtrate was concentrated under reduced pressure. The crude products was purified by silica gel column chromatography purification (ethyl acetate/petroleum ether (v/v) = 1/1) to obtain yellow solid N-(2-(bis(4-methoxybenzyl)carbamoyl)pyridine-4-yl)-7-(4,4-difluoroazepan-1-yl)quinoline-6-carboxamide (200 mg, yield 26.2%). $^1$H NMR (400 MHz, DMSO-$d_6$) δ 11.13 (s, 1H), 8.86 (dd, *J* = 4.3, 1.7 Hz, 1H), 8.54 (d, *J* = 5.6 Hz, 1H), 8.41-8.26 (m, 1H), 8.14 (s, 1H), 8.01 (d, *J* = 1.7 Hz, 1H), 7.77 (dd, *J* = 5.6, 2.0 Hz, 1H), 7.50 (s, 1H), 7.39 (dd, *J* = 8.2, 4.3 Hz, 1H), 7.20 (dd, *J* = 21.2, 8.6 Hz, 4H), 6.92 (dd, *J* = 18.3, 8.6 Hz, 4H), 4.47 (s, 2H), 4.41 (s, 2H), 3.75 (d, *J* = 10.4 Hz, 6H), 3.42 (t, *J* = 5.8 Hz, 4H), 2.34-2.18 (m, 2H), 2.08 (dd, *J* = 16.0, 10.4 Hz, 2H), 1.83 (d, *J* = 5.3 Hz, 2H). ESI-MS(m/z): 665.9 [M+H]$^+$.

step e): Preparation of N-(2-carbamoylpyridine-4-yl)-7-(4,4-difluoroazepan-1-yl)quinoline-6-carboxamide

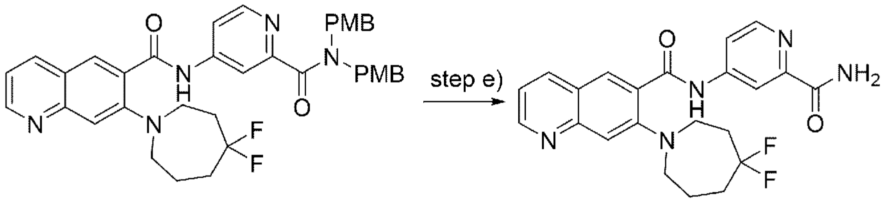


KH18

Trifluoroacetic acid (2 mL) was added to a solution of N-(2-(bis(4-methoxybenzyl)carbamoyl)pyridine-4-yl)-7-(4,4-difluoroazepan-1-yl)quinoline-6-carboxamide (200 mg, 0.30 mmol) in dichloroethane (2 mL). The reaction system was stirred at 80 ° C for 2 H. After the reaction was completed, the reaction mixture was concentrated and the resulting crude product was purified by Prep-HPLC (1% formic acid aqueous solution, methanol) to obtain white solid N-(2-carbamoylpyridine-4-yl)-7-(4,4-difluoroazepan-1-yl)quinoline-6-carboxamide (40 mg, yield 31.2%). $^1$H NMR (400 MHz, DMSO-$d_6$) δ 11.33 (s, 1H), 9.02 (d, *J* = 4.0 Hz, 1H), 8.79 (d, *J* = 7.8 Hz, 1H), 8.58 (d, *J* = 5.5 Hz, 1H), 8.45-8.30 (m, 2H), 8.12 (s, 1H), 7.92 (dd, *J* = 5.4, 2.0 Hz, 1H), 7.69-7.60 (m, 2H), 7.43 (s, 1H), 3.55 (dd, *J* = 10.5, 4.6 Hz, 4H), 2.37-2.35 (m, 2H), 2.13-2.07 (m, 2H), 1.95-1.87 (m, 2H). ESI-MS(m/z): 426.0 [M+H]$^+$.

**Example 19**

Preparation of N-(3-carbamoyl-4-fluorophenyl)-7-(4,4-difluoroazepan-1-yl)quinoline-6-carboxamide (KH19)

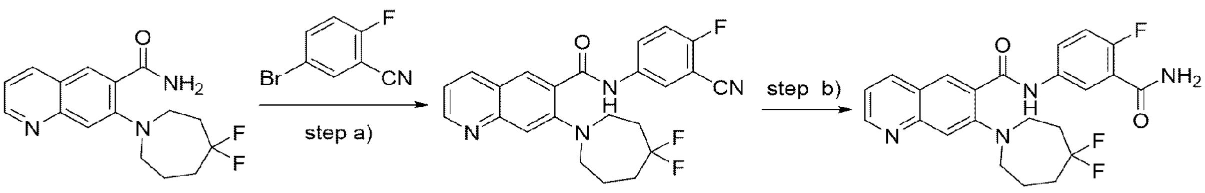


KH19

Step a): Preparation of N-(3-cyano-4-fluorophenyl)-7-(4,4-difluoroazepan-1-yl)quinoline-6-carboxamide

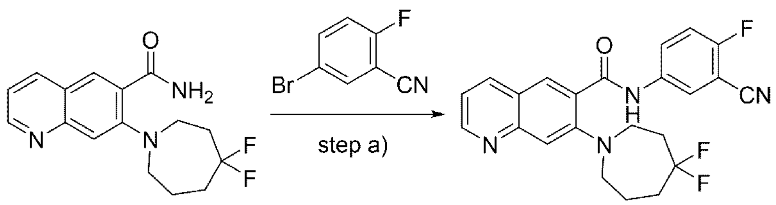


For the synthesis of 7-(4,4-difluoroazepan-1-yl)quinoline-6-carboxamide, see step a-c of Example 18.

5-Bromo-2-fluorobenzonitrile (238 mg, 1.19 mmol), Brettphos-Pd-G3 (98.7 mg, 0.109 mmol) and cesium carbonate (880 mg, 2.72 mmol) were added to a solution of 7-(4,4-difluoroazepan-1-yl) quinoline-6-carboxamide (350 mg, 1.06 mmol) in toluene (5 mL) and stirred at 110 °C for 16 h under nitrogen protection. After the reaction was completed, the reaction system was quenched with water (50 mL), extracted with ethyl acetate (15 mL × 3), and the combined organic phase was washed with saturated salt water (10 mL × 3), dried with anhydrous sodium sulfate, filtered, and the filtrate was concentrated under reduced pressure. The crude product was purified by silica gel column chromatography (ethyl acetate/petroleum ether (v/v) = 1/1) to obtain yellow solid *N*-(3-cyano-4-fluorophenyl)-7-(4,4-difluoroazepan-1-yl)quinoline-6-carboxamide(200 mg, yield 44.3%). ESI-MS(m/z): 424.9 [M+H]$^+$.

Step b): Preparation of N-(3-carbamoyl-4-fluorophenyl)-7-(4,4-difluoroazepan-1-yl)quinoline-6-carboxamide (KH19)

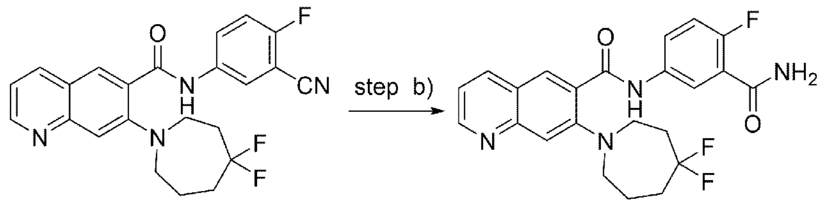


Cesium carbonate (535 mg, 1.64 mmol) was added to a solution of N-(3-cyano-4-fluorophenyl)-7-(4,4-difluoroazepan-1-l)quinoline-6-carboxamide (200 mg, 0.47 mmol) in dimethyl sulfoxide (5 mL), and 30% hydrogen peroxide (0.5mL) was slowly added at 0 °C, and reacted at room temperature for 4 h. After the reaction was completed, the reaction system was quenched with ice water (10 mL), extracted with ethyl acetate (20 mL × 3), and the combined organic phase was washed with saturated salt water (60 mL), and then dried with anhydrous sodium sulfate, filtered, and the filtrate was concentrated under reduced pressure. The resulting crude product was purified by Prep-HPLC (1% formic acid aqueous solution, methanol) to obtain white solid N-(3-carbamoyl-4-fluorophenyl)-7-(4,4-difluoroazepan-1-yl)quinoline-6-carboxamide (22.12 mg, yield 30.56%). $^{1}$H NMR (400 MHz, DMSO-$d_6$) δ 10.91 (s, 1H), 8.99 (d, *J* = 3.9 Hz, 1H), 8.75 (d, *J* = 8.1 Hz, 1H), 8.30 (s, 1H), 8.05 (dd, *J* = 6.4, 2.7 Hz, 1H), 7.92-7.80 (m, 1H), 7.72-7.54 (m, 3H), 7.40 (s, 1H), 7.31 (d, *J* = 9.2 Hz, 1H), 3.67-3.43 (m, 4H), 2.35-2.30 (m, 2H), 2.13-2.09 (m, 2H), 1.91 (br s, 2H). ESI-MS(m/z): 443.3 [M+H]$^+$.

**Example 20**

Preparation of N-(2-carbamoylpyridin-4-yl)-6-chloro-3-(4,4-difluoroazepan-1-yl)quinoline-2-carboxamide (KH20)

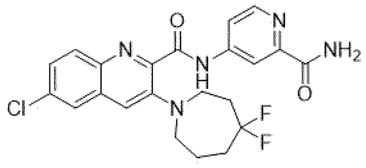

KH20

The synthesis of N-(2-carbamoylpyridin-4-yl)-6-chloro-3-(4,4-difluoroazepan-1-yl) quinoline-2-carboxamide is similar to that of example 15, except that 2-methyl-1H-indole in step a is replaced with 5-chloro-2-methyl-1H-indole. Yellow solid N-(2-carbamoylpyridin-4-yl)-6-chloro-3-(4,4-difluoroazepan-1-yl)quinoline-2-carboxamide(6.67 mg, 18% yield) was obtained. $^{1}$H NMR (400 MHz, DMSO-$d_6$) δ 11.38 (s, 1H), 8.57 (d, *J* = 5.4 Hz, 1H), 8.42 (d, *J* = 1.8 Hz, 1H), 8.12 (s, 1H), 8.02 (d, *J* = 2.3 Hz, 1H), 7.98 (d, *J* = 8.9 Hz, 1H), 7.92 (dd, *J* = 5.5, 2.1 Hz, 1H), 7.88 (s, 1H), 7.67 (s, 1H), 7.57 (dd, *J* = 8.9, 2.3 Hz, 1H), 3.47-3.40 (m, 4H), 2.29-2.24 (m, 2H), 2.16-2.04 (m, 2H), 1.87 (br s, 2H). ESI-MS(m/z): 460.1 [M+H]$^+$.

**Example 21**

Preparation of N-(3-carbamoyl-4-fluorophenyl)-2-(4,4-difluoroazepan-1-yl)-6,7-difluoroquinoline-3-carboxamide (KH21)

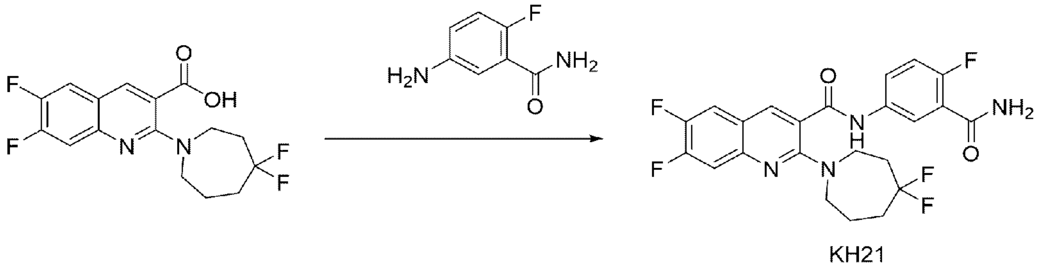

KH21

2-(4,4-Difluoroazepan-1-yl)-6,7-difluoroquinoline-3-carboxylic acid was prepared in the same way as step a-d of Example 10.

HATU (187.7 mg,0.494mmol), DIEA (196.1 mg, 1.520 mmol) and 5-amino-2-fluorobenzamide (58.5 mg, 0.380 mmol) were added to a solution of 2-(4, 4-difluoroazepan-1-yl)-6,7-difluoroquinolin-3-carboxylic acid (130 mg, 0.380 mmol) in dichloromethane (4 mL) and DMF (1 mL) at room temperature and reacted at 40 °C for 48 h. After the reaction was completed, water was added to quench the reaction system, and the organic phase (15 mL) was extracted with ethyl acetate, washed with saturated salt water, dried with anhydrous sodium sulfate and filtered, and the filtrate was concentrated under reduced pressure. The crude product was prepared by Prep-HPLC to obtain white solid N-(3-carbamoyl-4-fluorophenyl)-2-(4,4-difluoroazepan-1-yl)-6,7-difluoroquinoline-3-carboxamide(64.17 mg, yield 35%). $^1H$ NMR (400 MHz, DMSO-$d_6$) δ 10.83 (s, 1H), 8.31 (s, 1H), 8.11-8.00 (m, 1H), 7.95-7.88 (m, 1H), 7.86-7.81 (m, 1H), 7.70 (s, 2H), 7.60-7.57 (m, 1H), 7.30 (t, $J$ = 9.5 Hz, 1H), 3.74 (br s, 2H), 3.59 (t, $J$ = 5.7 Hz, 2H), (2.49 2.39 m, 2H), 2.02-1.90 (m, 2H), 1.89 (brs, 2H). ESI-MS(m/z): 479.2 $[M+H]^+$.

**Example 22**

Preparation of N-(2-carbamoylpyridin-4-yl)-2-(4,4-difluoroazepan-1-yl)-7-methoxyquinoline-3-carboxamide (KH22)

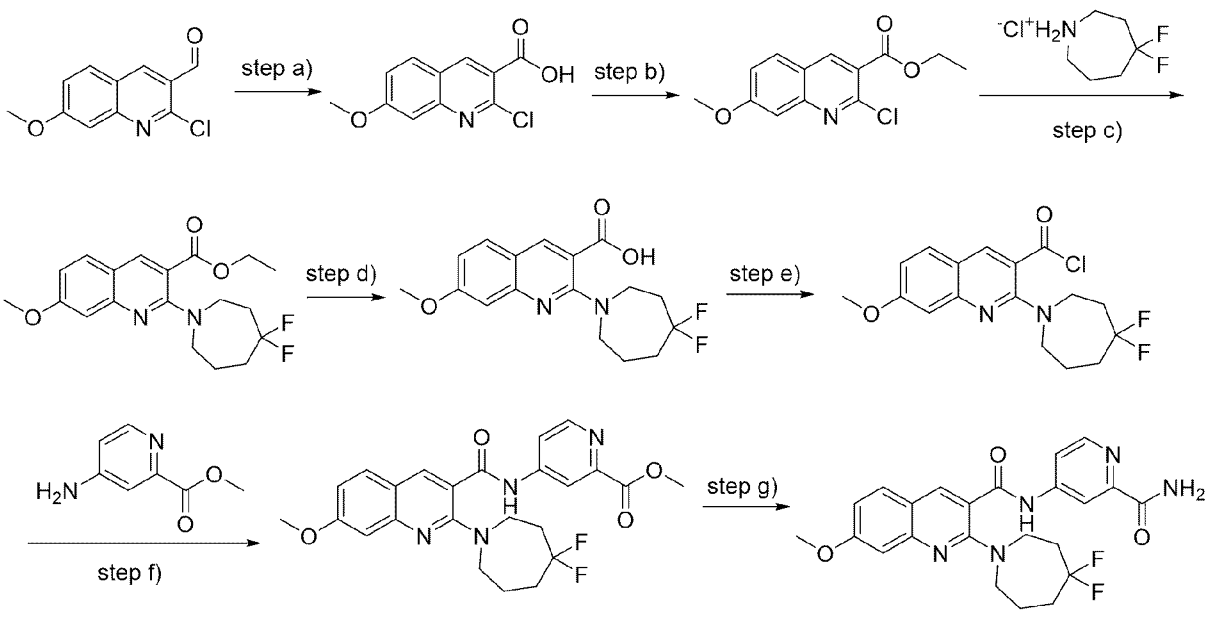


KH22

Step a): Preparation of 2-chloro-7-methoxyquinoline-3-carboxylic acid

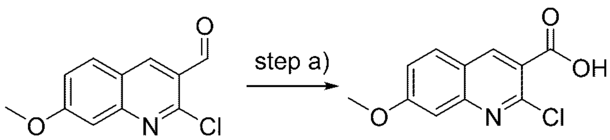


Ethanol/water (v/v = 4/1, 10mL) solution of silver nitrate (2.45g, 14.44mmol) and sodium hydroxide (1.80g, 45.12mmol) were added to the solution of 2-chloro-7-methoxyquinoline-3- carbaldehyde (2.0g, 9.02mmol) in ethanol (20 mL) and stirred at room temperature for 16 h. After the reaction was completed, the reaction system was diluted with 20 mL water, then extracted with ethyl acetate (30 mL × 3), and the combined organic phase was washed with saturated salt water, dried with anhydrous sodium sulfate, filtered, and the filtrate was concentrated under reduced pressure and crude brown solid 2-chloro-7-methoxyquinoline-3-carboxylic acid was obtained (1.0 g, yield 50%). $^1H$NMR

(400 MHz, DMSO-$d_6$) δ 13.58 (brs, 1H), 8.86 (s, 1H), 8.08 (d, $J$ = 9.0 Hz, 1H), 7.41 (d, $J$ = 2.4 Hz, 1H), 7.38-7.32 (m, 1H), 3.95 (s, 3H). ESI-MS(m/z): 237.8 [M+H]$^+$.

Step b): Preparation of ethyl 2-chloro-7-methoxyquinoline-3-carboxylate

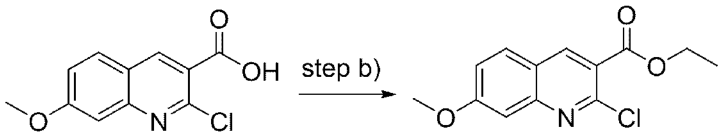


2-Chloro-7-methoxyquinoline-3-carboxylic acid (1.0g, 4.21mmol) was dissolved in DMF (15mL), and $K_2CO_3$ (1.16g, 8.42mmol) was added. The reaction system was placed in a 0 °C ice water bath and then ethane iodide was added (1.31g, 8.42mmol). After that, the ice water bath was removed and the reaction was carried out at room temperature for 2 h. After the reaction was completed, the reaction system was diluted with 20 mL water, extracted by ethyl acetate (30 mL × 3), and the combined organic phase was washed in saturated salt water, dried with anhydrous sodium sulfate, and filtered, and the filtrate was concentrated under reduced pressure. The crude product was purified by silica gel column chromatography (petroleum ether/ethyl acetate (v/v) = 8/1) to obtain white solid ethyl 2-chloro-7-methoxyquinoline-3-carboxylate (600 mg, yield 60%). ESI-MS(m/z) : 265.9 [M+H]$^+$.

Step c): Preparation of ethyl 2-(4, 4-difluoroazepan-1-yl)-7-methoxyquinoline-3-carboxylate

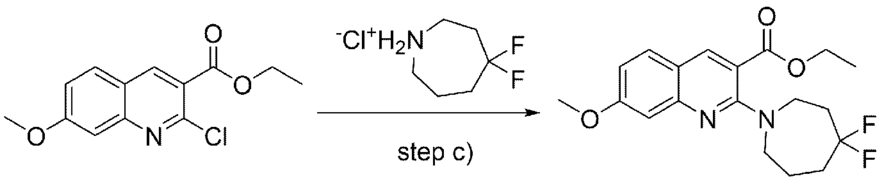


4,4-Difluoroazepane hydrochloride (503.82 mg, 2.94 mmol) and N,N-diisopropylethylamine (875.61 g, 6.77 mmol) were added to a solution of ethyl 2-chloro-7-methoxyquinoline-3-carboxylate(600 mg, 2.26 mmol) in *N-methylpyrrolidone* (10 mL). After the reaction was completed, the reaction system was diluted with water (30 mL), then extracted with ethyl acetate (20 mL × 2), and the combined organic phase was washed with saturated salt water (20 mL), dried with anhydrous sodium sulfate and filtered, and the filtrate was concentrated under reduced pressure. The crude product was purified by silica gel column chromatography (ethyl acetate/petroleum ether (v/v) = 1/8) to obtain colorless oil ethyl 2-(4,4-difluoroazepan-1-yl)-7-methoxyquinoline-3-carboxylate (400 mg, yield 66.7%). ESI-MS(m/z) : 365.5 [M + H]$^+$.

Step d): Preparation of 2-(4, 4-difluoroazepan-1-yl)-7-methoxyquinoline-3-carboxylic acid

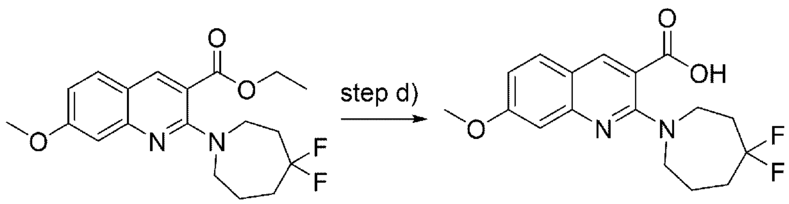


Lithium hydroxide (262.87 mg, 10.98 mmol) was slowly added to the solution of ethyl 2-(4,4-difluoroazepan-1-yl)-7-methoxyquinoline-3-carboxylate (400 mg, 1.1 mmol) in the mixed solvent of water (1 mL)/ methanol (5 mL)/ tetrahydrofuran (1 mL) and reacted at 50 °C for 12 h. After the reaction was completed, water (20 mL) was added to dilute the reaction mixture, and the pH of that was adjusted to about 4 with 1 M of dilute hydrochloric acid, then the organic phase was extracted with ethyl acetate (30 mL × 3), washed with saturated salt water, dried with anhydrous sodium

sulfate and filtered. The filtrate was concentrated under reduced pressure to obtain white solid 2-(4,4-difluoroazepan-1-yl)-7-methoxyquinoline-3-carboxylic acid (320 mg, yield 80%). ESI-MS(m/z) : 336.9 $[M + H]^+$.

Step e): Preparation of 2-(4,4-difluoroazepan-1-yl)-7-methoayquinoline-3-fortnyl chloride

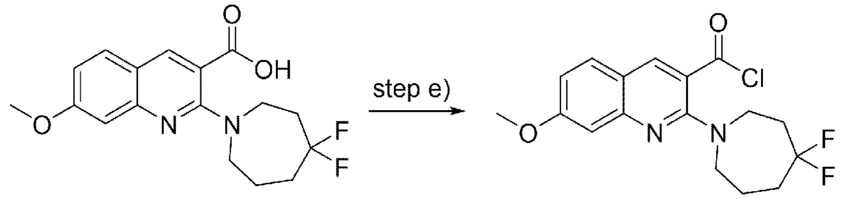


Thionyl chloride (222.36 mg, 1.9 mmol) was added to the solution of 2-(4,4-difluoroazepan-1-yl)-7-methoxyquinoline-3-carboxylic acid(320 mg, 0.951 mmol) in dichloromethane (5 mL) at 0 °C. After the reaction was completed, the reaction mixture was concentrated under reduced pressure and colorless oil 2-(4,4-difluoroazepan-1-yl)-7-methoayquinoline-3-fortnyl chloride (320 mg, yield 100%) was obtained, which did not need purification and was directly used in the next step. ESI-MS(m/z): 335.6 $[M + H]^+$.

step f): Preparation of methyl 4-(2-(4,4-difluoroazepan-1-yl)-7-methoxyquinoline-3-carboxamido) pyridine-2-carboxylate

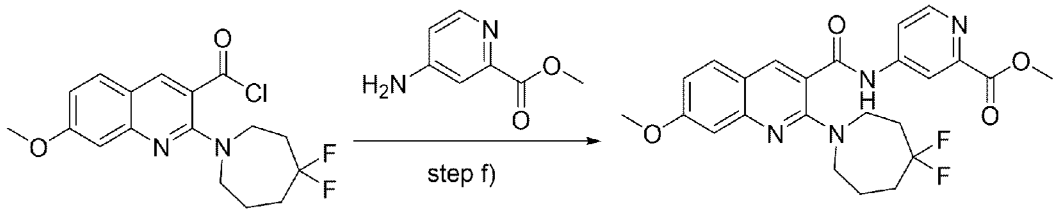


Triethylamine (182.54 mg, 1.8 mmol) and methyl 4-amino-pyridine-2-carboxylate (205.85mg, 1.35 mmol) were added to a solution of 2-(4,4-difluoroazepan-1-yl)-7-methoxyquinoline-3-formyl chloride (320 mg, 0.9 mmol) in dichloromethane (5 mL) at 0 °C. After the reaction was completed, the reaction mixture was diluted with water (20 mL), then extracted with ethyl acetate (20 mL × 2), and the combined organic phase was washed with saturated salt water (20 mL), dried with anhydrous sodium sulfate and filtered, and the filtrate was concentrated under reduced pressure. The crude product was purified by silica gel column chromatography (ethyl acetate/petroleum ether (v/v) = 1/1) to obtain yellow solid methyl 4-(2-(4,4-difluoroazepan-1-yl)-7-methoxyquinoline-3-carboxamido) pyridine-2-carboxylate (140 mg, yield 43.75%). $^1$HNMR (400 MHz, $CDCl_3$) δ 8.71-8.67 (m, 2H), 8.24 (s, 1H), 8.05 (s, 1H), 7.72 (d, $J$ = 8.9 Hz, 1H), 7.23-7.21 (m, 1H), 7.12 (d, $J$ = 9.0 Hz, 1H), 4.12 (d, $J$ = 7.2 Hz, 2H), 4.03 (s, 3H), 3.99-3.97 (m, 3H), 3.77-3.73 (m, 2H), 3.57-3.53 (m, 2H), 2.22 (br s, 2H), 1.97 (br s, 2H). ESI-MS(m/z): 470.8 $[M + H]^+$.

step g): Preparation of N-(2-carbamoylpyridin-4-yl)-2-(4,4-difluoroazepan-1-yl)-7-methoxyquinoline-3-carboxamide

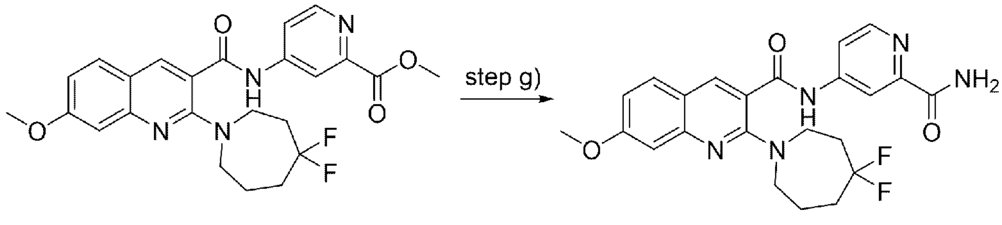


Ammonium chloride (127.33 mg, 2.38 mmol) and ammonia water (417.15 mg, 2.98 mmol) were added to a solution of methyl 4-(2-(4,4-difluoroazepan-1-yl)-7-methoxyquinoline-3-carboxamido)pyridine-2-carboxylate (140 mg,

0.297 mmol) in dimethyl sulfoxide (4 mL), and it was stirred for 24h in a sealed tank at 70 °C. After the reaction was completed, water (15 mL) was added to dilute the reaction mixture, then it was extracted with ethyl acetate (20 mL × 2), and the combined organic phase was washed with saturated salt water (20 mL), dried with anhydrous sodium sulfate and filtered, and the filtrate was concentrated under reduced pressure. The resulting crude product was prepared by C18 reversed columnar formic acid to obtain yellow solid powder N-(2-carbamoylpyridin-4-yl)-2-(4,4-difluoroazepan-1-yl)-7-methoxyquinoline-3-carboxamide(52.93 mg, yield 37.8%).) $^1$HNMR (400 MHz, DMSO-$d_6$) δ 11.14 (s, 1H), 8.54 (d, *J* = 5.5 Hz, 1H), 8.38 (d, *J* = 1.5 Hz, 1H), 8.31 (s, 1H), 8.10 (s, 1H), 7.91 (dd, *J* = 5.3, 1.8 Hz, 1H), 7.75 (d, *J* = 8.8 Hz, 1H), 7.65 (s, 1H), 7.03 (d, *J* = 2.2 Hz, 1H), 6.95 (dd, *J* = 8.8, 2.4 Hz, 1H), 3.89 (s, 3H), 3.77 (br s, 2H), 3.54 (t, *J* = 5.8 Hz, 2H), 2.44-2.38 (m, 2H), 1.99-1.89 (m, 2H), 1.88-1.82 (m, 2H). ESI-MS(m/z) : 456.2 [M +H]$^+$.

**Example 23**

Preparation of N-(2-carbamoylpyridin-4-yl) -2-(4,4-difluoroazepan-1-yl)-7-methylquinoline-3-carboxamide (KH23)

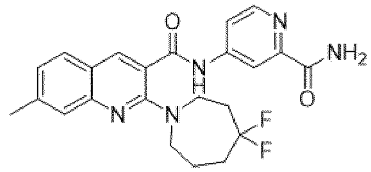

KH23

N-(2-carbamoylpyridin-4-yl)-2-(4,4-difluoroazepan-1-yl)-7-methylquinoline-3-carboxamide was synthesized with the same method as in Example 22, except that 2-chloro-7-methoxyquinoline-3-formaldehyde in step a is replaced by 2-chloro-7-methylquinolin-3-formaldehyde. The yellow solid *N*-(2-carbamoylpyridin-4-yl)-2-(4,4-difluoroazepan-1-yl)-7-methylquinoline-3-carboxamide(30.27 mg, yield 21%) was obtained by replacing the smothered tank 70 °C in step g with smothered tank 80 °C. $^1$H NMR (400 MHz, DMSO-$d_6$) δ 11.16 (s, 1H), 8.55 (d, *J* = 5.5 Hz, 1H), 8.38 (d, *J* = 2.0 Hz, 1H), 8.33 (s, 1H), 8.10 (s, 1H), 7.90 (d, *J* = 5.4, 2.1 Hz, 1H), 7.74 (d, *J* = 8.2 Hz, 1H), 7.64 (s, 1H), 7.46 (s, 1H), 7.16 (d, *J* = 8.3, 1.5 Hz, 1H), 3.76-3.70 (m, 2H), 3.55 (2H, t, *J* = 5.9 Hz), 2.46 (br s, 3H), 2.45-2.31 (m, 2H), 2.04-1.99 (m, 2H), (1.90 1.81 m, 2H). ESI-MS(m/z) : 440.3 [M +H]$^+$.

**Example 24**

Preparation of N-(2-carbamoylpyridin-4-yl)-2-(4,4-difluoroazepan-1-yl)-6,7-dimethoxyquinoline-3-carboxamide (KH24)

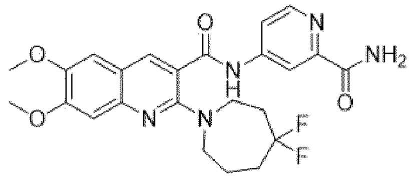

KH24

N-(2-carbamoylpyridin-4-yl)-2-(4,4-difluoroazepan-1-yl)-6,7-dimethoayquinoline-3-carboxamide was synthesized with the same method as that in Example 22, except that 2-chloro-7-methoxyquinoline-3-formaldehyde in step a) is replaced by 2-chloro-6,7-dimethoxyquinoline-3-formaldehyde. The yellow solid *N*-(2-carbamoylpyridin-4-yl)-2-(4,4-difluoroazepan-1-yl)-6,7-dimethoayquinoline-3-carboxamide (110.22 mg, yield 56%) was obtained by replacing tank 70 °C in step g with tank 85 °C. $^1$H NMR (400 MHz, DMSO - $d_6$) δ 11.09 (s, 1H), 8.54 (d, *J* = 5.5 Hz, 1H), 8.39 (s, 1H), 8.22 (s, 1H), 8.10 (s, 1H), 7.90 (d, *J* = 3.3 Hz, 1H), 7.64 (s, 1H), 7.28 (s, 1H), 7.06 (s, 1H), 3.91 (s, 3H), 3.84 (s, 3H), 3.68 (br s, 2H), 3.51 (t, *J* = 5.8 Hz, 2H), 2.40 (br s, 2H), 1.99-1.95 (m, 2H), 1.90 1.83 (m, 2H). ESI-MS(m/z) : 486.2 [M +H]$^+$.

**Example 25**

Preparation of N-(2-carbamoylpyridin-4-yl)-2-(4,4-difluoroazepan-1-yl)-7-methoxyquinoline-3-carboxamide (KH25)

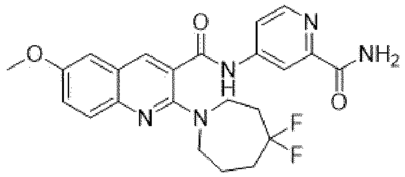

KH25

*N*-(2-carbamoylpyridin-4-yl)-2-(4,4-difluoroazepan-1-yl)-7-methoxyquinoline-3-carboxamide was synthesized with the same method as that in Example 22, except that 2-chloro-7-methoxyquinoline-3-formaldehyde in step a is replaced by 2-chloro-6-methoxyquinoline-3-formaldehyde. The yellow solid N-(2-carbamoylpyridin-4-yl)-2-(4,4-difluoroazepan-1-yl)-7-methoxyquinoline-3-carboxamide (35.20 mg, yield 28%) was obtained by replacing tank 70 °C in step g with tank 85 °C. $^1$H NMR (400 MHz, DMSO-$d_6$) δ 11.16 (s, 1H), 8.55 (d, *J* = 5.4 Hz, 1H), 8.39 (s, 1H), 8.30 (s, 1H), 8.12 (d, *J* = 13.3 Hz, 1H), 7.89 (d, *J* = 1.9 Hz, 1H), 7.64 (d, *J* = 13.8 Hz, 1H), 7.60 (d, *J* = 9.3 Hz, 1H), 7.34 (d, *J* = 2.9 Hz, 1H), 7.31 (s, 1H), 3.84 (s, 3H), 3.68 (m, 2H), 3.53 (2H, t, *J* = 5.9 Hz), 2.35 (m, 2H), 1.99 (m, 2H), 1.86 (m, 2H). ESI-MS(m/z) : 456.1 [M +H]$^+$.

Among them, the synthesis method of 2-chloro-6-methoxyquinoline-3-formaldehyde is as follows.

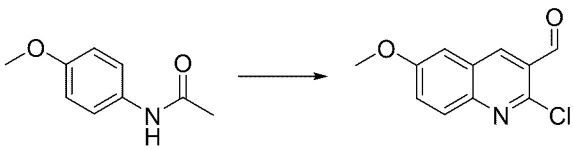

Phosphorus oxytrichloride (41.77 g, 272.41 mmol) was slowly added to DMF (7.96 g, 108.96 mmol) at 0 °C, and it was stirred continually for 1 hour. p-Methoxyacetanilide (4.5g, 27.24mmol) was added into the reaction mixture, and the temperature was raised to 80 °C and the reaction system was continually stirred for 12 hours. After the reaction was completed, the reaction system was quenched with ice water, and then filtered after stirring for 10 minutes, the filter cake was washed with water, and the brown solid 2-chloro-6-methoxyquinoline-3-formaldehyde (1.8g, yield 40%) crude product was obtained after being dried, which was directly used in the next step without purification. $^1$H NMR (400 MHz, DMSO-$d_6$) δ 8.76 (s, 1H), 8.13 (d, *J* = 8.1 Hz, 1H), 7.98 (d, *J* = 8.4 Hz, 1H), 7.88 (t, *J* = 7.6 Hz, 1H), 7.70 (t, *J* = 7.5 Hz, 1H), 3.69 (s, 3H). ESI-MS(m/z) : 221.9 [M+H]$^+$.

**Example 26**

Preparation of 2-(4,4-difluoroazepan-1-yl)-N-(2-(S-methylsulfonimidoyl)pyridin-4-yl) quinoline-3-carboxamide (KH26)

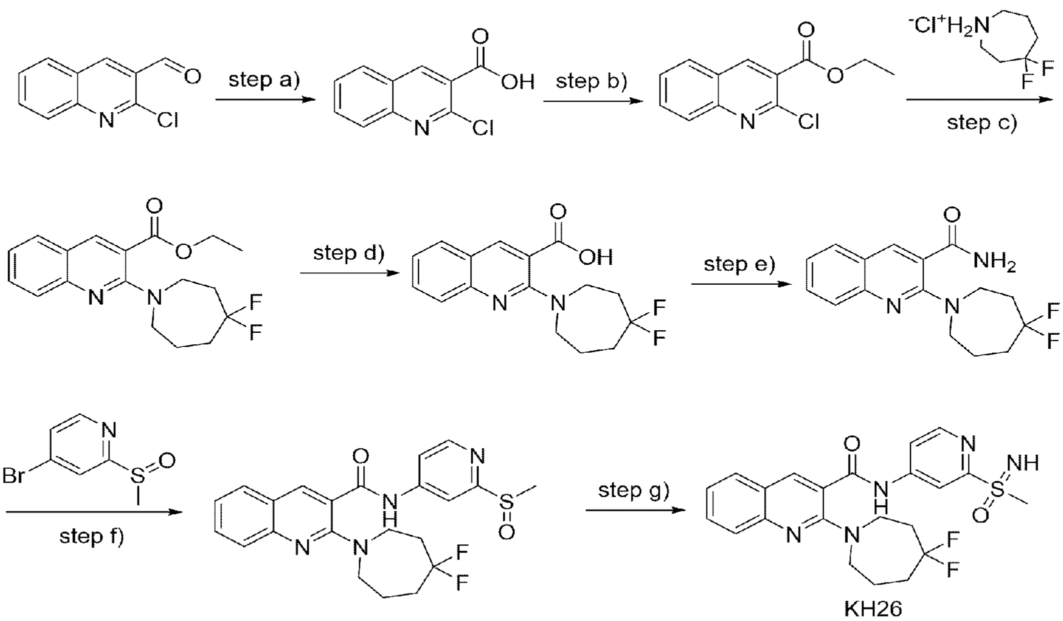


KH26

Step a): Preparation of 2-chloroquinoline-3-carboxylic acid

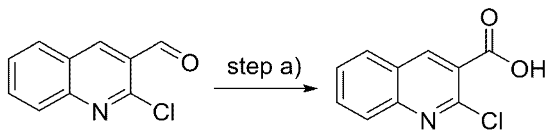


Sodium hydroxide (14.61 g, 365.32 mmol) aqueous solution was added to silver nitrate (29.61 g, 174.31mmol) aqueous solution, followed by 2-chloroquinoline-3-formaldehyde (20 g, 104.38 mmol) and stirred at 0 °C for 3 h. After the reaction was completed, the reaction liquid was filtered, and the filtrate was adjusted to pH = 5 with 3 N HCl, filtered, and yellow filter cake was obtained, that is, 2-chloroquinoline-3-carboxylic acid crude product (15 g, yield 69.2%), which was directly used in the next step. $^1$H NMR (400 MHz, DMSO-$d_6$) δ 10.38 (s, 1H), 8.85 (s, 1H), 7.96 (d, $J$ = 9.2 Hz, 1H), 7.71 (d, $J$ = 2.8 Hz, 1H), 7.63 (dd, $J$ = 9.2, 2.8 Hz, 1H). ESI-MS(m/z) : 207.8 [M+H]$^+$.

Step b): Preparation of ethyl 2-chloroquinoline-3-carboxylate

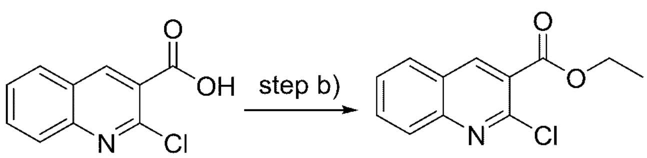


$K_2CO_3$ (30.62 g, 221.57mmol) was added to a solution of 2-chloroquinoline-3-carboxylic acid (23 g, 110.78 mmol) in DMF (200 mL), and ethane iodide (34.55 g, 221.57mmol) was dropwise added at 0 °C, and then the reaction system rose to room temperature and the reaction was continued for 16 h. After the reaction was completed, 200 mL water was added to dilute the reaction mixture, and then it was extracted with ethyl acetate (200 mL × 3), and the combined organic phase was washed with saturated salt water, dried with anhydrous sodium sulfate and filtered. The filtrate was concentrated under reduced pressure, and the crude product was purified by silica gel column chromatography (petroleum ether/ethyl acetate (v/v) = 9/1) to obtain yellow solid ethyl 2-chloroquinoline-3-carboxylate (14 g, yield 53.6%). $^1$H NMR (400 MHz, DMSO-$d_6$) δ 8.96 (s, 1H), 8.22-8.18 (m, 1 H), 8.05-8.01 (m, 1 H), 7.96 (m, 1 H), 7.76 (m, 1 H), (4.48 4.37 m, 2H), 1.41-1.34 (m, 3H). ESI-MS(m/z): 235.9 [M+H]$^+$.

Step c): Preparation of ethyl 2-(4,4-difluoroazepan-1-yl)quinoline-3-carboxylate

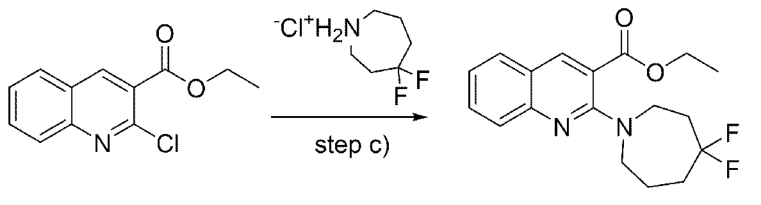


4,4-Difluoroazepane hydrochloride (28.05 g, 165.00 mmol) and DIEA (49.15 g, 381.00 mmol) were added to a solution of ethyl 2-chloroquinolin-3-carboxylate (30 g, 127.00 mmol) in NMP (400 ml) and reacted at 140 °C for 16 h. After the reaction was completed, 500 mL water was added to dilute the reaction system, which was extracted with ethyl acetate (600 mL × 3), and the combined organic phase was washed three times with saturated salt water, dried with anhydrous sodium sulfate and filtered. The filtrate was concentrated under reduced pressure, and the resulting crude product was purified by silica gel column chromatography (petroleum ether/ethyl acetate (v/v) = 3/1) to obtain yellow solid ethyl 2-(4,4-difluoroazepan-1-yl)quinoline-3-carboxylate (35 g, yield 82.23%). $^1H$ NMR (400 MHz, DMSO-$d_6$) δ1 8.42 (d, $J$ = 7.0 Hz, 1H), 7.84 (d, $J$ = 8.0 Hz, 1H), 7.69-7.54 (m, 2H), 7.35-7.19 (m, 1H), 4.35 (q, $J$ = 7.1 Hz, 2H), 3.78-3.63 (m, 2H), 3.44 (2H, t, $J$ = 5.8 Hz), 2.48-2.34 (m, 2H), 2.08-1.85 (m, 4H), 1.34 (t, $J$ = 7.1 Hz, 3H). ESI-MS(m/z): 335.1 [M +H]$^+$.

Step d): Preparation of 2-(4,4-difluoroazepan-1-yl)quinoline-3-carboxylic acid

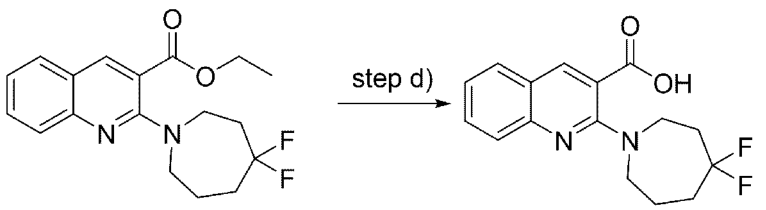


Lithium hydroxide (11.86 g, 495.00 mmol) was added to methanol (400 mL)/ water (80mL) solution of ethyl 2-(4, 4-difluoroazepan-1-yl)quinoline-3-carboxylate (35 g, 105.00 mmol) and it was stirred at room temperature overnight. After the reaction was completed, the methanol of the reaction mixture was removed by vacuum rotary evaporation, and the pH of the resulting mixture was adjusted to 3~4 with diluted hydrochloric acid, then the product was precipitated, filtered, and the filter cake was pulped with petroleum ether to obtain white solid 2-(4,4-difluoroazepan-1-yl)quinoline-3-carboxylic acid (25 g, yield 78%). $^1H$ NMR (400 MHz, DMSO - $d_6$) δ 8.43 (s, 1H), 7.84 (d, $J$ = 7.8 Hz, 1H), 7.65-7.51 (m, 2H), 7.27 (m, 1H), 3.74-3.68 (m, 2H), 3.50 (t, $J$ = 5.9 Hz, 2H), 1.99-1.90 (m, 6H). ESI-MS(m/z) : 307.1 [M +H]$^+$.

Step e): Preparation of 2-(4, 4-difluoroazepan-1-yl)quinoline-3-carboxamide

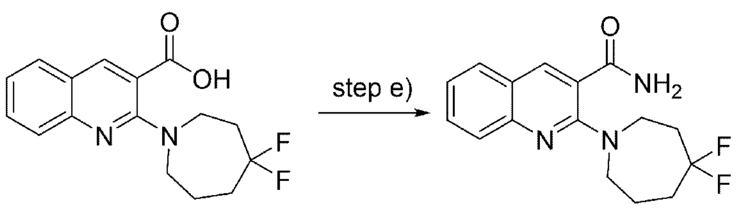


Ammonium chloride (12.05 g, 225.26 mmol), DIEA (29.11 g, 225.26mmol) and HATU (34.26g, 90.10 mmol) were added to a solution of 2-(4,4-difluoroazepan-1-yl)quinoline-3-carboxylic acid (23 g, 75.09 mmol) in DMF (200 ml) in sequence and reacted at room temperature for 16 h. After the reaction was completed, the reaction system was diluted with 200 mL water, extracted with ethyl acetate (200 mL × 3), and the combined organic phase was washed with

saturated salt water, dried with anhydrous sodium sulfate and filtered. The filtrate was concentrated under reduced pressure, and the resulting crude product was purified by silica gel column chromatography (petroleum ether/ethyl acetate (v/v) = 1/1) to obtain yellow solid 2-(4,4-difluoroazepan-1-yl)quinoline-3-carboxamide (9 g, yield 39.26%). $^1$H NMR (400 MHz, DMSO-$d_6$) δ 8.15 (d, *J* = 12.5 Hz, 2H), 7.84 (d, *J* = 8.0 Hz, 1H), 7.64 (d, *J* = 3.9 Hz, 2H), 7.39-7.23 (m, 1H), 3.82-3.75 (m, 2H), 3.68 (2H, t, *J* = 6.0 Hz), 2.49-2.45 (m, 2H), 2.08 (td, *J* = 14.3, 8.2 Hz, 2H), 2.02-1.91 (m, 2H). ESI-MS(m/z) : 305.9 $[M +H]^+$.

Step f): Preparation of 2-(4,4-difluoroazepan-1-yl)-N-(2-(methylsulfmyl)pyridin-4-yl)quinoline-3-carboxamide

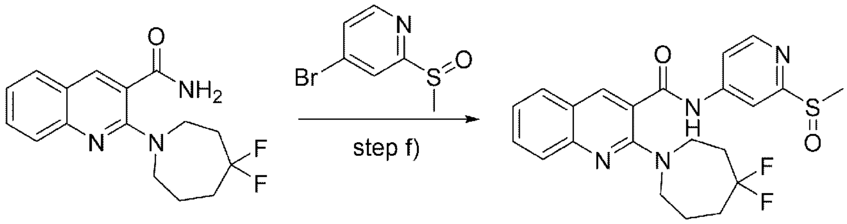


2-(4,4-Difluorazepane-1-yl)quinoline-3-carboxamide(305 mg, 1.00 mmol), 4-bromo-2-(methylsulfinyl)pyridine (220 mg, 1.00 mmol), $Cs_2CO_3$ (1.14 g, 3.50 mmol), Ruphos-Pd-G2 (77 mg, 0.10 mmol) were dissolved in toluene (5 mL), and the reaction system was replaced with nitrogen and stirred at 110 °C for 16 h. After the reaction was completed, 10 mL water was added to the reaction mixture, and then it was extracted with ethyl acetate (10 mL × 2), and the combined organic phase was washed with saturated salt water (10 mL), dried with anhydrous sodium sulfate and filtered, and the filtrate was concentrated under reduced pressure. The crude product was purified by silica gel plate (ethyl acetate/petroleum ether (v/v) = 2/1) to obtain yellow solid 2-(4,4-difluoroazepan-1-yl)-N-(2-(methylsulfmyl)pyridin-4-yl)quinoline-3-carboxamide(386 mg, yield 86.7%). $^1$H NMR (400 MHz, DMSO-$d_6$) δ 11.41 (s, 1H), 8.65 (d, *J* = 5.4 Hz, 1H), 8.47 (s, 1H), 8.36 (s, 1H), 7.94-7.85 (m, 2H), 7.71 (d, *J* = 3.7 Hz, 2H), 7.41-7.35 (m, 1H), 3.86-3.75 (m, 2H), 3.63 (2H, t, *J* = 5.8 Hz), 2.87 (s, 3H), (2.52 2.40 m, 2H), 2.13-2.05 (m, 2H), 1.98-1.90 (m, 2H). ESI-MS(m/z) : 445.1 $[M +H].^+$

Step g): Preparation of 2-(4,4-difluoroazepan-1-yl)-N-(2-(S-methylsulfonimidoyl)pyridin-4-yl)quinoline-3-carboxamide

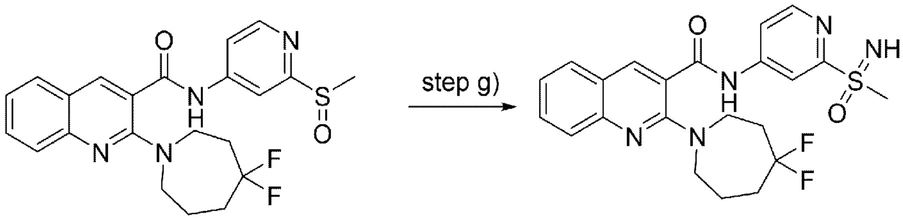


2-(4,4-Difluoroazepan-1-yl)-N-(2-(methylsulfinyl)pyridin-4-yl)quinoline-3-carboxamide (350 mg, 0.786 mmol) was dissolved in Eaton's reagent (5 mL), and $NaN_3$ (102 mg, 1.573 mmol) was added and stirred at 50 °C for 1 H. After the reaction was completed, the reaction system was quenched with water (10 mL), extracted with dichloromethane (10 mL × 2), and the combined organic phase was washed with saturated salt water (10 mL × 2), dried with anhydrous sodium sulfate and filtered, and the filtrate was concentrated under reduced pressure. The resulting crude product was purified by reverse phase preparation to obtain yellow solid 2-(4,4-difluoroazepan-1-yl)-*N*-(2-(S-methylsulfonimidoyl)pyridin-4-yl)quinoline-3-carboxamide (47.31 mg, yield 13.1%). $^1$H NMR (400 MHz, DMSO-$d_6$) δ 11.37 (s, 1H), 8.63 (d, *J* = 5.4 Hz, 1H), 8.44 (d, *J* = 1.5 Hz, 1H), 8.40 (s, 1H), 7.90 (dd, *J* = 5.4, 1.8 Hz, 1H), 7.85 (d, *J* = 8.0 Hz, 1H), 7.65 (d, *J* = 3.7 Hz, 2H), 7.35-7.28 (m, 1H), 4.36 (s, 1H), 3.81-3.70 (m, 2H), 3.56 (2H, t, *J* = 5.7 Hz), 3.16 (s, 3H), 2.47-2.35 (m, 2H), 2.08-1.95 (m, 2H), 1.93-1.83 (m, 2H). ESI-MS(m/z) : 460.2 $[M +H]^+$.

**Example 27**

Preparation of 2-(4,4-difluoroazepan-1-yl)-6,7-difluoro-N-(2-(S-methylsulfonimidoyl) pyridin-4-yl) quinoline-3-carboxamide (KH27)

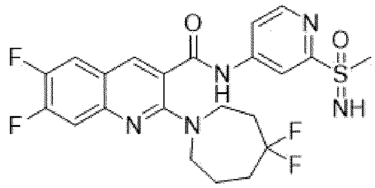

KH27

The synthesis of 2-(4,4-difluoroazepan-1-yl)-6,7-difluoro-N-(2-(S-methylsulfonimidoyl) pyridin-4-yl) quinoline-3-carboxamide is similar to step e-g of Example 26, except that the 2-(4,4-difluoroazepan-1-yl)quinoline-3-carboxylic acid in step e is replaced with 2-(4,4-difluoroazepan-1-yl)-6,7-difluoroquinoline-3-carboxylic acid. The synthesis of 2-(4,4-difluoroazepan-1-yl)-6,7-difluoroquinoline-3-carboxylic acid is described in steps a-d of Example 10.

$^1$H NMR (400 MHz, DMSO-$d_6$) δ 11.46 (s, 1H), 8.67 (d, $J$ = 5.5 Hz, 1H), 8.46 (s, 1H), 8.40 (s, 1H), 7.95-7.90 (m, 2H), 7.62-7.57 (m, 1H), 3.73 (br s, 2H), 3.54 (2H, t, $J$ = 5.6 Hz), 3.28 (s, 3H), 2.45 2.39 (m, 2H), 2.03-1.97 (m, 2H), 1.93-1.88 (m, 2H). ESI-MS(m/z): 496.3 [M +H]$^+$.

**Example 28**

Preparation of 2-(4,4-difluoroazepan-1-yl)-N-(2-(N-methylsulfamoyl)pyridin-4-yl)quinoline-3-carboxamide (KH28)

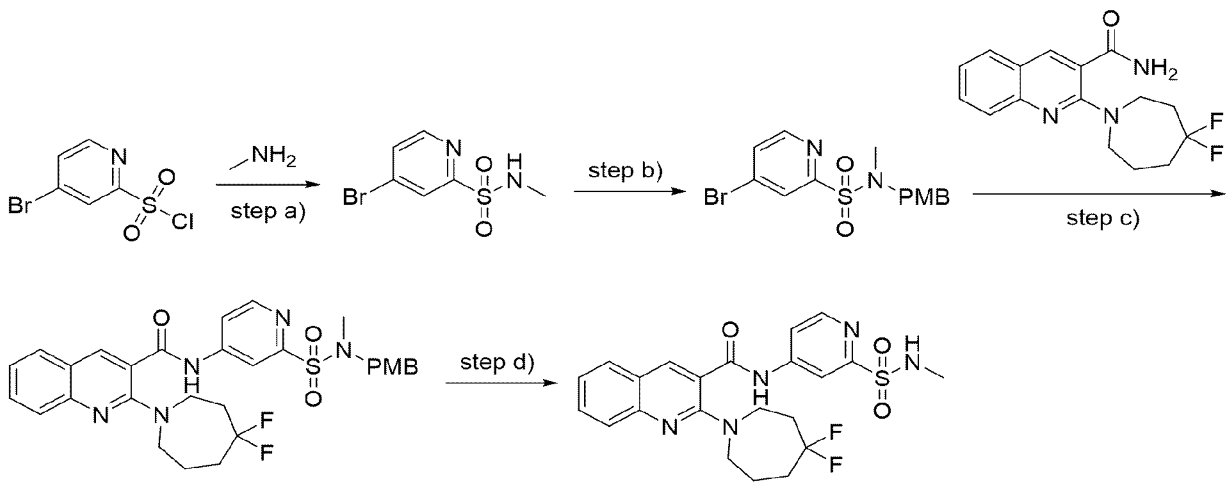


KH28

Step a) Preparation of 4-bromo-N-methylpyridine-2-sulfonamide

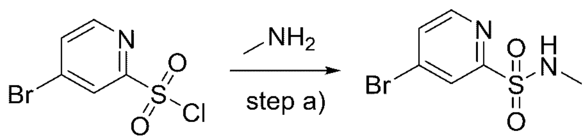

DIEA (5.786 g, 44.85 mmol) and 4-bromopyridine-2-sulfonyl chloride (2.87 g, 11.21 mmol) were added to a solution of methylamine (2.78 g, 25% aqueous solution) in tetrahydrofuran (10 mL) and it was stirred at room temperature for 2 h. After the reaction was completed, 30 mL of water was added, then it was extracted with ethyl acetate (30 mL × 2), and the combined organic phase was washed with saturated salt water (30 mL), dried with anhydrous sodium sulfate and filtered, and the filtrate was concentrated under reduced pressure. The resulting crude product was purified by silica gel column chromatography (ethyl acetate/petroleum ether (v/v) = 1/1). Yellow solid 4-bromo-N-methylpyridine-2-sulfonamide (1.36g, yield 48.3%) was obtained. $^1H$ NMR (400 MHz, DMSO-$d_6$) δ 8.64 (d, *J* = 5.2 Hz, 1H), 8.10 (d, *J* = 1.8 Hz, 1H), 7.99 (dd, *J* = 5.2, 1.8 Hz, 1H), 7.87-7.81 (m, 1H), 2.57 (d, *J* = 4.9 Hz, 3H). ESI-MS(m/z) : 250.9 $[M + H]^+$.

Step b) Preparation of 4-bromo-N -(4-methoxybenzyl) -N-methylpyridine-2-sulfonamide

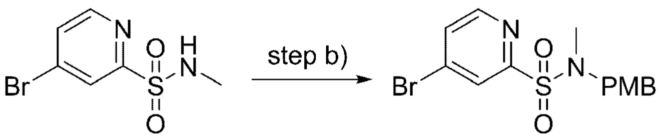


4-bromo-N-methylpyridine-2-sulfonamide (400 mg, 1.59 mmol), PMBCl (500 mg, 3.19 mmol) and $K_2CO_3$ (440 mg, 3.19 mmol) were dissolved in DMF (4 mL) and stirred at room temperature for 6 h. After the reaction was completed, 10 mL of water was added, then it was extracted with ethyl acetate (10 mL × 3), and the combined organic phase was washed with saturated salt water (10 mL × 3), dried with anhydrous sodium sulfate and filtered, and the filtrate was concentrated under reduced pressure. The resulting crude product was purified and prepared by silica gel plate (ethyl acetate/petroleum ether (v/v) = 1/4) to obtain yellow solid 4-bromo-N-(4-methoxybenzyl)-N-methylpyridine-2-sulfonamide (500 mg, yield 84.5%) was obtained. $^1H$ NMR (400 MHz, $CDCl_3$) δ 8.45 (d, *J* = 5.1 Hz, 1H), 8.06 (d, *J* = 1.7 Hz, 1H), 7.58 (dd, *J* = 5.1, 1.7 Hz, 1H), 7.23 (d, *J* = 8.5 Hz, 1H), 6.85-6.76 (m, 3H), 4.30 (s, 2H), 3.74 (s, 3H), 2.74 (s, 3H). ESI-MS(m/z): 371.0 $[M +H]^+$.

Step c) Preparation of 2-(4,4-difluoroazepan-1-yl)-N-(2-(N-(4-methoxybenzyl)-N-methyl sulfamoyl)pyridin-4-yl) quinoline-3-carboxamide

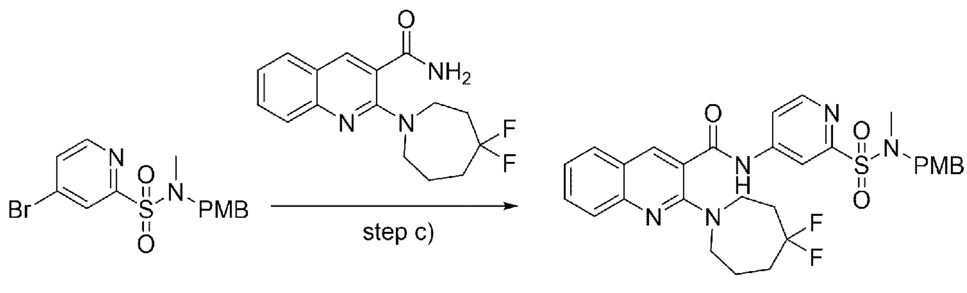


For the synthesis of 2-(4,4-difluoroazepan-1-yl) quinoline-3-carboxamide, see step a-e of Example 26.

4-Bromo-N -(4-methoxybenzyl)-N-methylpyridine-2-sulfonamide (250 mg, 0.67 mmol), 2-(4,4-difluoroazepan-1-yl) quinoline-3-carboxamide (205 mg, 0.67 mmol), Ruphos-Pd-G2 (52 mg, 0.067 mmol) and $Cs_2CO_3$ (768 mg, 2.36 mmol) were dissolved in toluene (5 mL) and stirred at 110 °C for 12 H under nitrogen protection. After the reaction was completed, 10 mL of water was added, then it was extracted with ethyl acetate (10 mL × 2), and the combined organic phase was washed with saturated salt water (10 mL), dried with anhydrous sodium sulfate and filtered, and the filtrate was concentrated under reduced pressure. The crude product was purified by silica gel plate (petroleum ether/ethyl acetate (v/v) = 2/3) and yellow solid 2-(4,4-difluoroazepan-1-yl)-N-(2-(N-(4-methoxybenzyl)- N-methylsulfamoyl)pyridin-4-yl) quinoline-3-carboxamide (370 mg, yield 92.1%) was obtained. $^1H$ NMR (400 MHz, DMSO-$d_6$) δ *11.46* (s, 1H), 8.75 (d, *J* = 5.5 Hz, 1H), 8.50 (s, 1H), 8.40 (s, 1H), 8.03 (d, *J* = 3.6 Hz, 1H), 7.91 (d, *J* = 7.9 Hz, 1H), 7.74-7.69 (m, 2H), 7.42-7.36 (m, 1H), 7.34-7.29 (m, 2H), 7.01-6.97 (m, 2H), 4.36 (s, 2H), 3.85-3.76 (m, 6H), 3.66-3.58 (m, 2H), 2.78 (s, 2H), 2.51-2.40 (m, 2H), 2.12-2.01 (m, 2H), 2.00-1.91 (m, 2H). ESI-MS(m/z) : 596.2 $[M +H]^+$.

Step d) Preparation of 2-(4,4-difluoroazepan-1-yl)-N-(2-(N-methylsulfamoyl)pyridin-4-yl) quinoline-3 -carboxamide

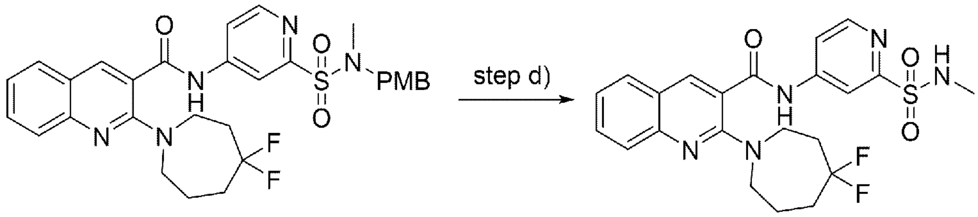


2-(4,4-Difluoroazepan-1-yl)-N-(2-(N-(4-methoxybenzyl)-N-methylsulfamoyl)pyridin-4-yl) quinoline-3-carboxamide (370 mg, 0.62 mmol) was dissolved in trifluoroacetic acid (5 mL) and trifluoromethanesulfonic acid (0.5 mL) was added and stirred at 70 °C for 2 h. After the reaction was completed, the reaction system was restored to room temperature, and it was then poured into ice water (10 mL), and extracted with 2-methyltetrahydrofuran (10 mL × 2). The combined organic phase was washed with saturated salt water (10 mL), dried with anhydrous sodium sulfate and filtered, and the filtrate was concentrated under reduced pressure. The resulting crude product was prepared and purified to obtain light yellow solid powder 2-(4,4-difluoroazepan-1-yl)-N-(2-(N-methylsulfamoyl)pyridin-4-yl) quinoline-3-carboxamide (105.74 mg, yield 35.8%). $^1$H NMR (400 MHz, DMSO - $d_6$) δ 11.36 (s, 1H), 8.65 (d, $J$ = 5.5 Hz, 1H), 8.44 (s, 1H), 8.32 (s, 1H), 7.95-7.90 (m, 1H), 7.85 (d, $J$ = 7.9 Hz, 1H), 7.75-7.69 (m, 1H), 7.67 (d, $J$ = 3.8 Hz, 2H), 7.37-7.30 (m, 1H), 3.78-3.71 (m, 2H), 3.56 (t, $J$ = 5.8 Hz, 2H), 2.57 (3H, d, $J$ = 4.8 Hz), 2.46-2.35 (m, 2H), 2.06-1.94 (m, 2H), 1.93-1.85 (m, 2H). ESI-MS(m/z): 476.1 [M +H]$^+$.

## Example 29

Preparation of 2-(4,4-difluoroazepan-1-yl)-6,7-difluoro-N-(2-(N-methylsulfamoyl)pyridin-4-yl) quinoline-3-carboxamide (KH29)

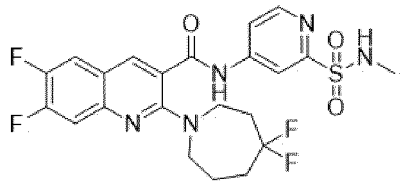

KH29

The synthesis of 2-(4,4-difluoroazepan-1-yl)-6,7-difluoro-N-(2-(N-methylsulfamoyl)pyridin-4-yl)quinoline-3-carboxamide is similar to example 28, except that 2-(4,4-difluoroazepan-1-yl)quinoline-3-carboxamide is replaced by 2-(4,4-difluoroazepan-1-yl)-6,7-difluoroquinoline-3-carboxamide (for the synthesis see step a-d of Example 10 and step e of Example 26), obtaining a quick light yellow solid 2-(4,4-difluoroazepan-1-yl)-6,7-difluoro-N-(2-(N-methylsulfamoyl)pyridin-4-yl)quinoline-3-carboxamide(61. 82 mg, 23.13% yield). $^1$H NMR (400 MHz, DMSO-$d_6$) δ 11.40 (s, 1H), 8.66 (d, $J$ = 5.5 Hz, 1H), 8.42 (s, 1H), 8.30 (d, $J$ = 1.9 Hz, 1H), 7.95-7.88 (m, 2H), 7.73 (q, $J$ = 4.9 Hz, 1H), 7.63-7.58 (m, 1H), 3.76-3.67 (m, 2H), 3.54 (2H, t, $J$ = 5.8 Hz), 2.56 (d, $J$ = 4.9 Hz, 3H), 2.47-2.33 (m, 2H), 2.07-1.93 (m, 2H), 1.90-1.84 (m, 2H). ESI-MS(m/z) : 512.1 [M +H]$^+$.

## Example 30

Preparation of 2-(2-chloro-4-fluorophenoxy)-N-(3-sulfamoylphenyl)quinoline-3-carboxamide (compound in Example 66 of Patent WO2014120815A1)

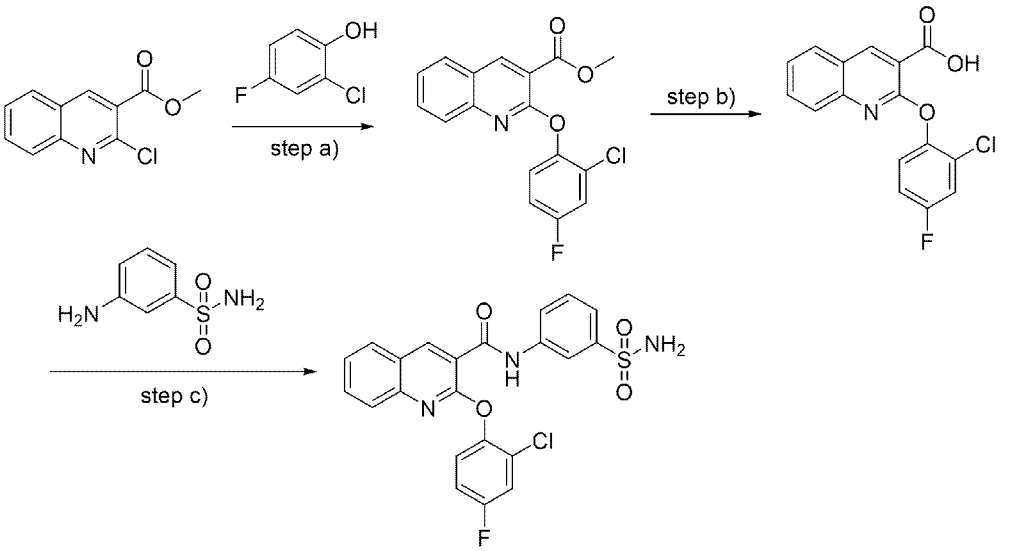


Step a): Preparation of methyl 2-(2-chloro-4-fluorophenoxy)quinoline-3-carboxylate

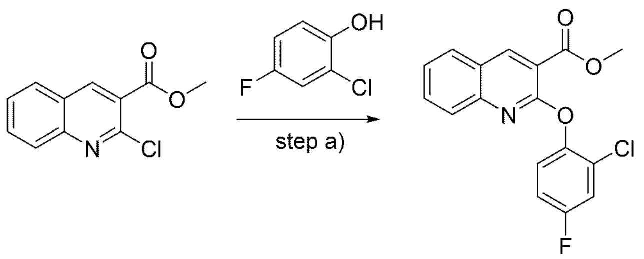


Methyl 2-chloroquinolin-3-carboxylate (0.117g, 0.53mmol), cesium carbonate (0.525g, 1.6mmol), 10 mL DMF and 2-chloro-4-fluorophenol (0.159g, 1.1mmol) were added into a single-neck bottle of 100 mL, and the reaction system was replaced with nitrogen for three times, and temperature was raised to 100 °C to continue the reaction. After the reaction was confirmed by TLC point plate, the system was cooled to room temperature. Then 50mL pure water was added, and ethyl acetate (30 mL × 3) was used for extraction, and the combined organic phase was washed with saturated sodium chloride, dried with anhydrous sodium sulfate and filtered, and the filtrate was concentrated to be dry with vacuum rotary evaporation. The crude product was purified by thin layer chromatography silica gel preparation plate (development agent: petroleum ether/ethyl acetate (v/v) = 4/1; Eluent: dichloromethane/methanol (v/v) = 10/1) and yellow solid methyl 2-(2-chloro-4-fluorophenoxy)quinoline-3-carboxylate (0.132g, yield 75.5%) was obtained. ESI-MS(m/z) : 332.4 $[M+H]^+$.

Step b): Preparation of 2-(2-chloro-4-fluorophenoxy)quinoline-3-carboxylic acid

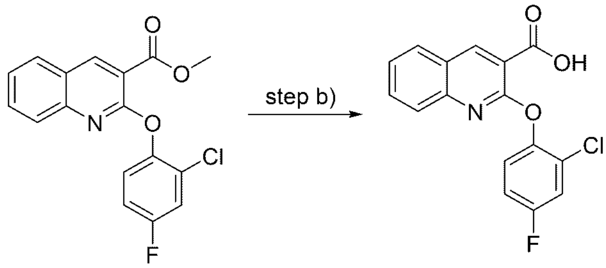

Methyl 2-(2-chloro-4-fluorophenoxy)quinoline-3-carboxylate (0.122g, 0.37mmol) and 4 mL methanol were added into a 100 mL single-neck bottle, and stirred for 10 minutes, then 20 mL pure water and lithium hydroxide (36mg, 1.5mmol) were added, and the system continued to react at 45 °C for 10 hours. The reaction mixture was concentrated to be dry under reduced pressure, then 20 mL pure water was added, and ethyl acetate (20 mL × 2) was used for extraction. The water phase was adjusted pH4~5 with 1M hydrochloric acid, then extracted with ethyl acetate (20 mL × 3), and the combined organic phase was washed with saturated sodium chloride solution, dried with anhydrous sodium sulfate and filtered, and the filtrate was concentrated under reduced pressure to obtain white solid 2-(2-chloro-4-fluorophenoxy)quinoline-3-carboxylic acid (0.102 g, yield 87.3%). ESI-MS(m/z): 316.0 $[M-H]^-$.

Step c): Preparation of 2-(2-chloro-4-fluorophenoxy)-*N*-(3-sulfamoylphenyl)quinoline-3-carboxamide

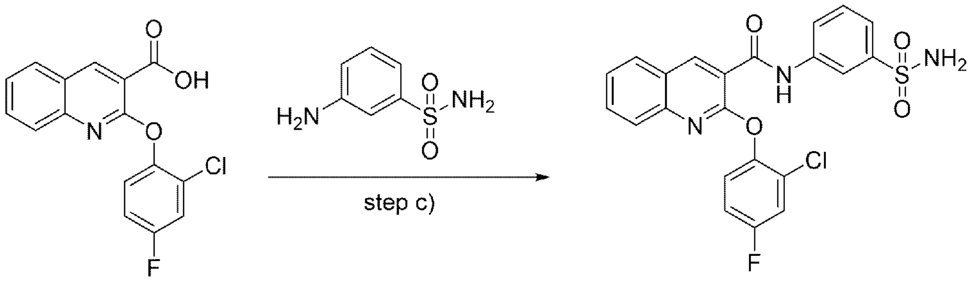


2-(2-Chloro-4-fluorophenoxy)quinoline-3-carboxylic acid (60mg, 0.189mnol), 3-aminobenzenesulfonamide (39mg, 0.226mmol) and 4mL DMF were added into a 250 mL three-neck bottle, and the reaction system was replaced with nitrogen for three times to protect it. After cooling in ice water bath for 20 minutes, DIPEA (53mg, 0.41mmol) was added, and then the solution of propylphosphonic anhydride (0.331g, 0.52mmol) was added by drops, and the reaction was continued for 1h after dropping, and then it was raised to room temperature and stirred overnight. After the reaction was completed, 30 mL of pure water was added, and then it was extracted with ethyl acetate (30 mL × 3), and the combined organic phase was washed with saturated sodium chloride aqueous solution, dried with anhydrous sodium sulfate and filtered, and the filtrate was concentrated to be dry under reduced pressure. The crude product was purified by thin layer chromatography silica gel preparation plate (development agent: dichloromethane/methanol (v/v) = 10/1); Eluent: dichloromethane/methanol (v/v) = 10/1) and white solid 2-(2-chloro-4-fluorophenoxy)-*N*-(3-sulfamoylphenyl)quinoline-3-carboxamide (17mg, yield 19.06%) was obtained. $^1H$ NMR (400 MHz, DMSO-$d_6$) δ 10.94 (s, 1H), 8.79 (s, 1H), 8.39 (s, 1H), 8.09 (d, $J$ = 8.0 Hz, 1H), 7.95-7.82 (m, 1H), 7.79-7.71 (m, 1H), 7.68-7.54 (m, 6H), 7.48-7.32 (m, 3H). ESI-MS(m/z) : 472.2 $[M+H]^+$.

**Example 31**

Preparation of 5-chloro-2 -(4,4-difluoroazepan-1-yl)-N-(2-sulfamoylpyridin-4-yl)-4-trifluoromethylbenzamide (compound A6 in patent CN112457294B)

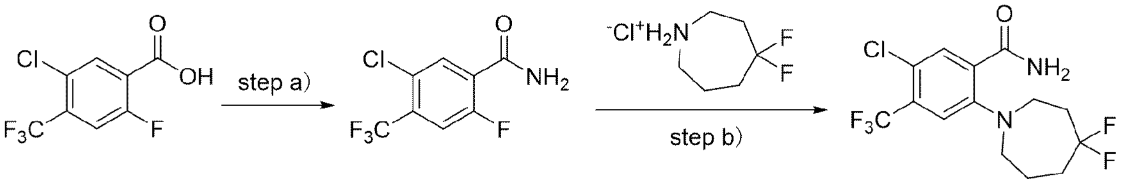


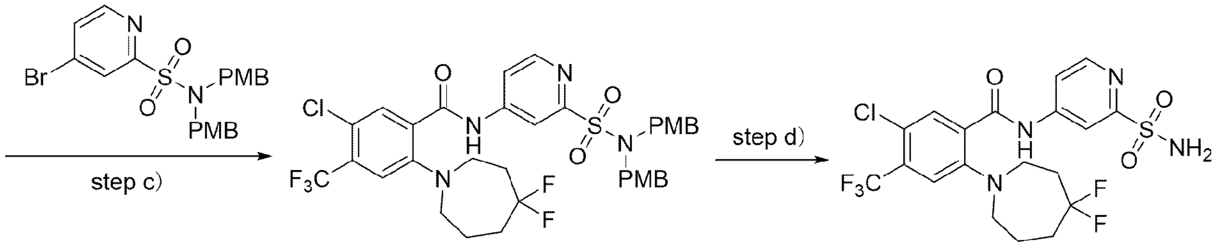


Step a): Preparation of 5-chloro-2-fluoro-4-trifluoromethylbenzamide

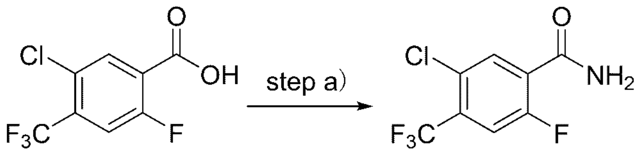


Oxalyl chloride (25 mL) and DMF (400 mg, 5.47mmol) were added to a solution of 5-chloro-2-fluoro-4-trifluoromethylbenzoic acid (22 g, 90 mol) in dichloromethane (100 mL) at 0 °C and it was continually stirred for 0.5 h. After the reaction was completed, the reaction solution was concentrated under reduced pressure to obtain yellow oil, and the dichloromethane was added (100 mL), and the system was placed in an ice water bath, then ammonia water (50 mL) was slowly added, and stirred for 1 H. After the reaction was completed, the reaction system was diluted with water (100 mL), extracted with dichloromethane (100 mL × 2), and the combined organic phase was washed with saturated salt water (50 mL), dried with anhydrous sulfuric acid and filtered. The filtrate was concentrated under reduced pressure to obtain white solid 5-chloro-2-fluoro-4-trifluoromethylbenzamide (21 g, yield 95%). $^1$H NMR (400 MHz, DMSO-$d_6$) δ 8.06 (s, 1H), 7.96 (s, 1H), 7.93 (s, 1H), 7.91 (d, $J$ = 2.5 Hz, 1H). ESI-MS(m/z): 239.7 [M-H]$^-$.

Step b): Preparation of 5-chloro-2-(4,4-difluoroazepan-1-yl) -4-trifluoromethylbenzamide

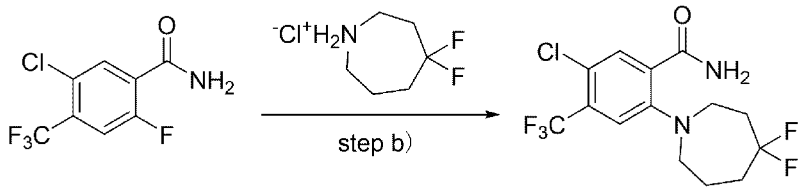


4,4-Difluoroazepane hydrochloride (30.6 g, 0.18 mol) and N,N-diisopropylethylamine (34.8 g, 0.27 mol) were added to a solution of 5-chloro-2-fluoro-4-trifluoromethylbenzamide (21.7 g, 0.09 mol) in N-methylpyrrolidone (200 mL) and stirred at 100 °C for 16 h. After the reaction was completed, the reaction system was quenched with water (600 mL), then extracted with ethyl acetate (400 mL × 2), and the combined organic phase was washed with saturated salt water (200 mL), dried with anhydrous sulfuric acid and filtered. The filtrate was concentrated under reduced pressure, and the crude product was purified by silica gel column chromatography (ethyl acetate/petroleum ether (v/v) = 1/3) to obtain yellow solid 5-chloro-2-(4,4-difluoroazepan-1-yl)-4-trifluoromethylbenzamide (27 g, yield 84%). $^1$H NMR (400 MHz, DMSO-$d_6$) δ 8.06 (s, 1 H), 7.69 (s, 1 H), 7.49 (s, 1 H), 7.27 (s, 1 H), 3.44-3.38 (m, 2 H), (3.35-3.30 m, 2 H), 2.31-2.22 (m, 2 H), 2.17-2.05 (m, 2 H), 1.89-1.80 (m, 2 H). ESI-MS(m/z): 357.0 [M+H]$^+$.

Step c): Preparation of N-(2-(N,N-bis(4-methoxybenzyl)sulfamoyl)pyridin-4-yl)-5-chloro-2-(4,4-difluoroazepan-1-yl)-4-trifluorometh ylbenzamide

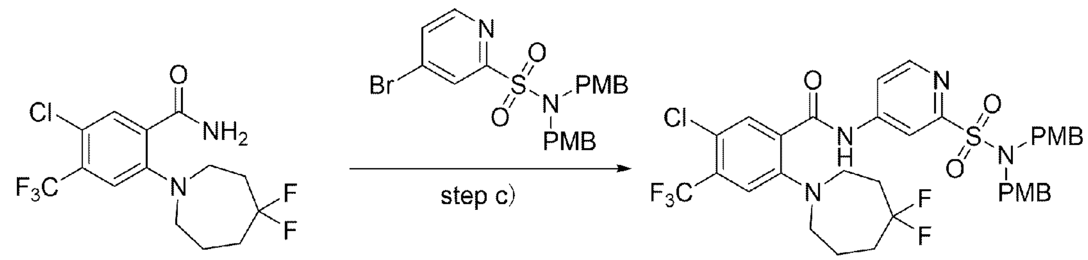


*4-Bromo-N*,N-bis(4-methoxybenzyl)pyridine-2-sulfonamide(16 g, 33.5 mmol), $Pd_2(dba)_3$ (2.3 g, 2.5 mmol), BINAP (3.14 g, 5.0 mmol) and cesium carbonate (27.3 g, 84.0 mmol) were added to a solution of 5-chloro-2 -(4,4-difluoroazepan-1-yl) -4-trifluoromethylbenzamide (12 g, 33.5 mmol) in toluene (100 mL) and stirred at 85 °C for 16 h. After the reaction was completed, the reaction system was diluted with water (300 mL), extracted with ethyl acetate (300 mL × 2), and the combined organic phase was washed with saturated salt water (100 mL), dried with anhydrous sodium sulfate and filtered. The filtrate was concentrated under reduced pressure. The crude product was purified by silica gel column chromatography (ethyl acetate/petroleum ether (v/v)=1/5) to obtain a yellow solid N-(2-(N,N-bis(4-methoxybenzyl)sulfamoyl)pyridin-4-yl)-5-chloro-2-(4,4-difluoroazepan-1-yl)-4-trifluorometh ylbenzamide (20 g, yield: 79%). ESI-MS(m/z): 753.2 $[M+H]^+$.

step d): Preparation of 5-chloro-2-(4,4-difluoroazepan-1-yl)-N-(2-sulfamoylpyridin-4-yl)-4-trifluoromethylbenzamide

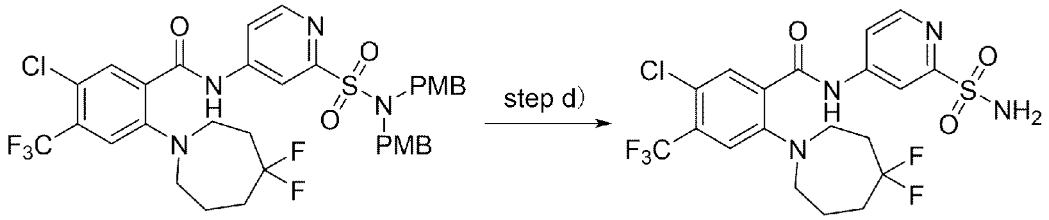


Trifluoromethane sulfonic acid (1 mL, 7.1 mmol) was slowly added to a trifluoroacetic acid (13 mL) solution of

*N*-(2-(N,N-bis(4-methoxybenzyl)sulfamoyl)pyridin-4-yl)-5-chloro-2-(4,4-difluoroazepan-1-yl)-4-trifluorometh ylbenzamide (7 g, 9.29 mmol) and reacted at 50 °C for 0.5 h. After the reaction was completed, the reaction solution was slowly added to ice water (100 mL), and stirred for 0.5 h, and filtered to obtain crude product. The crude product was purified by C18 reverse column (trifluoroacetic acid system) to obtain white solid (2.3 g, yield 48%). $^1H$ NMR (400 MHz, DMSO - $d_6$) δ 11.21 (s, 1H), 8.63 (d, *J* = 5.2 Hz, 1H), 8.31 (s, 1H), 7.88-7.80 (m, 1H), 7.76 (s, 1H), 7.47 (s, 2H), 7.36 (s, 1H), 3.41-3.39 (m, 1H), 3.36-3.34 (m, 1H), 2.29-2.14 (m, 2H), 2.12-1.98 (m, 2H), 1.83-1.74 (m, 2H). ESI-MS(m/z): 513.2 $[M+H]^+$.

**Example 32**

Preparation of 2-(4-fluoro-2-methylphenoxy)-N-(2-carbonyl-1,2-dihydropyridin-4-yl)-4-(trifluoromethyl)benzamide (parent drug of VX150)

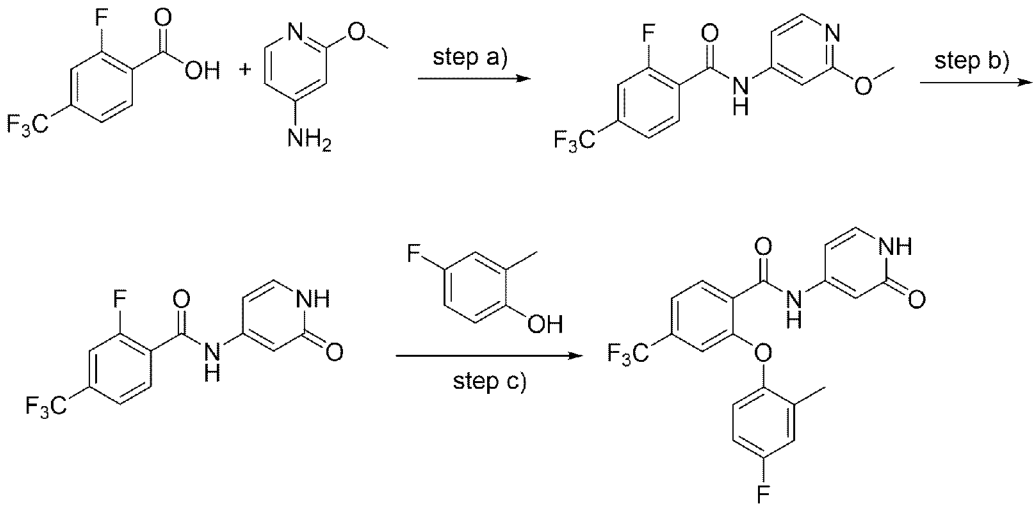


Step a): Preparation of 2-fluoro-N-(2-methoxypyridin-4-yl)-4-(trifluoromethyl)benzamide

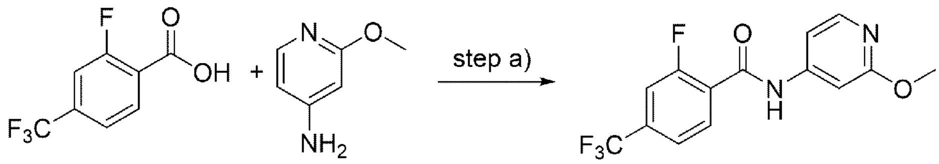


Triethylamine (1.7 g, 16.8 mmol) was added to dichloromethane (30 mL) solution of 2-fluoro-4-trifluoromethyl benzoic acid (1 g, 4.8 mmol), 2-methoxy-4-aminopyridine (0.626 g, 5 mmol) and HATU (2.741 g, 7.2 mmol) under nitrogen protection and stirred at room temperature for 16 h. After the reaction was completed, 50 mL pure water was added and stirred evenly, the liquid was divided and the lower organic phase was kept for use, and the upper aqueous phase was extracted with dichloromethane (30 mL × 3), all organic phases were combined, and then washed with saturated NaCl solution (30 mL × 2), dried with anhydrous sodium sulfate and filtered, and the filtrate was concentrated to be dry with vacuum rotary evaporation. The crude product was purified by silica gel column chromatography (PE/EA (v/v) = 8/1-3/1) to obtain white solid 2-fluoro-N-(2-methoxypyridin-4-yl)-4-(trifluoromethyl) benzamide (1.179 g, yield 78.1%).

Step b): Preparation of 2-fluoro-N-(2-carbonyl-1,2-dihydropyridin-4-yl)-4-(trifluoromethyl) benzamide

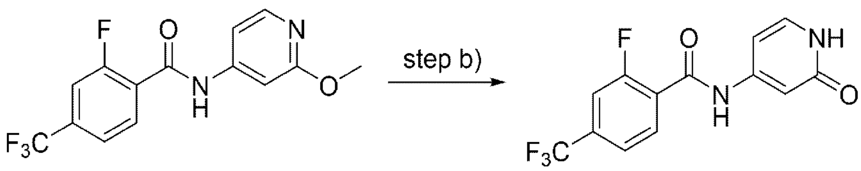


Hydrobromic acid/acetic acid solution (10 mL, 33%) was added to *2-fluoro-N* -(2-methoxy-pyridin-4-yl)-4-(trifluoromethyl) benzamide (0.5 g, 1.6 mmol) under the protection of nitrogen. The solution was reacted at 100 °C for 5 h, and then at room temperature for 12 h. 40 mL of pure water was added and stirred for 30 min, then filtered, and the filter cake was washed with pure water (10 mL × 2), and dried at 50 °C under reduced pressure to obtain gray solid *2-fluoro-N* -(2-carbonyl-1,2-dihydropyridin-4-yl)-4-(trifluoromethyl) benzamide (0.215 g, yield 45%).

Step c): Preparation of 2-(4-fluoro-2-methylphenoxy)-N-(2-carbonyl-1,2-dihydropyridin-4-yl)-4-(trifluoromethyl) benzamide

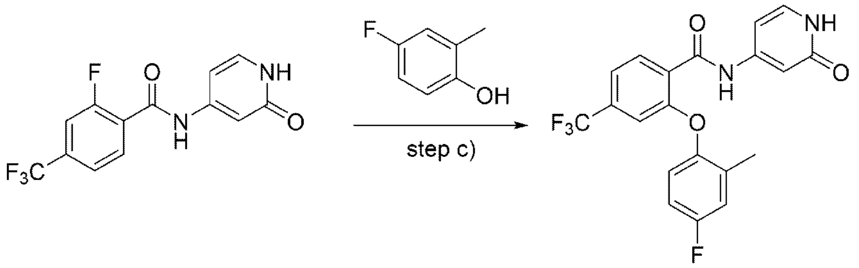


DMF (6 mL) was added to the mixture of 2-*fluoro*-*N*-(2-carbonyl-1,2-dihydropyridin-4-yl)-4-(trifluoromethyl) benzamide (0.21g, 0.7 mmol), 4-fluoro-2-methylphenol (0.261 g, 2 mmol) and cesium carbonate (0.684 g, 2 mmol) under nitrogen protection and reacted at 80 °C for 2 H. After the reaction was completed, the system was cooled to room temperature, and 50 mL of pure water was added, and stirred for 30 min, then it was filtered, and the filter cake was washed with pure water (20 mL × 2) and petroleum ether (20 mL × 2) in turn, and then dried under reduced pressure to obtain 0.278 g crude product. The crude product was completely dissolved with ethyl acetate (about 5 mL) and stirred for 5 min, then petroleum ether (about 15 mL) was slowly added,and a large amount of precipitation was precipitated, and it continued to be stirred for 2 h and filtered. The filter cake was washed with petroleum ether (10 mL × 2) and dried under reduced pressure to obtain white solid 2-(4-fluoro-2-methylphenoxy)-*N*-(2-carbonyl-1,2-dihydropyridin-4-yl)-4-(trifluoromethyl) benzamide (0.212 g, yield 75.6%). $^1$H NMR (400 MHz, DMSO-$d_6$) δ 11.29 (s, 1H), 10.64 (s, 1H), 7.84 (d, *J* = 7.9 Hz, 1H), 7.60 (d, *J* = 8.5 Hz, 1H), 7.31 (d, *J* = 7.2 Hz, 1H), 7.27 7.20 (m, 1H), 7.10 (dd, *J* = 6.7, 3.1 Hz, 1H), 6.97 (s, 1H), 6.75 (s, 1H), 6.38 (dd, *J* = 7.2, 2.0 Hz, 1H), 2.16 (s, 3H). ESI-MS(m/z): 407.2 $[M+H]^+$.

**Example 33: Electrophysiological assay**

The patch clamp technique is known as the "gold standard" for studying ion channels, and it uses glass microelectrodes to seal the cell membrane to measure the currents of various membrane channel. Navs are a class of transmembrane proteins, which contain three different states, the resting state, the active state and the inactive state. Patch clamp technology can evaluate the inhibition strength of different compounds on different states of Navs by measuring the changes of membrane channel currents after different compounds bind to different states of Navs.

The representative compounds of the present invention used stable CHO or HEK293 cell lines transfected with specific ion channels to measure the effect of the compounds on Nav1.8 currents by manual patch clamp test, to evaluate their inhibition strength.

Method 1:

The assay protocol of manual patch clamp is as follows:

1) Cell culture

CHO cell lines with stable expression of hNav1.8 were cultured in F12 medium containing 10% fetal bovine serum at a constant temperature of 37 °C, and the carbon dioxide concentration was maintained at 5%. The old culture medium was removed and washed once with PBS, then 1 mL 0.25%-Trypsin-EDTA solution was added and incubated at 37 °C for 1 min. When the cells were detached from the bottom of the dish, 5 mL of the complete medium preheated at 37 °C was added, and the aggregated cells were separated by gently blowing with a straw. The cell suspension was transferred into a sterile centrifuge tube and centrifuged at 1000 rpm for 5 min to collect cells. The cells were then inoculated into a cell culture dish with a diameter of 6 cm ($2.5\times10^5$ cells/petri dish, 5 mL medium) for expansion or maintenance.

In order to maintain the electrophysiological activity of the cells, the cell density must be less than 80%.

The cells were separated with 0.25%-Trypsin-EDTA before the assay, and $5\times10^3$ cells were seeded on a cover slip and cultured in a 24-well plate (final volume: 500μL) for 18 h. Then the assay was conducted.

2) Preparation of compound samples

The compounds prepared in the examples of the present invention were dissolved in dimethyl sulfoxide (DMSO) and prepared into DMSO stock solution with a concentration of 10 mM for assay. And it was diluted to various concentrations with extracellular solution (140mM NaCl, 3.5mM KCl, 1mM $MgCl_2$, 2mM $CaCl_2$, 10mM Glucose, 10mM HEPES, 1.25mM $NaH_2PO_4$, pH=7.4 regulated by NaOH), and the final concentration of DMSO in each compound sample was 0.1% or less.

3) Patch clamp measures sodium channel blocking effect

A capillary glass pipette (BF150-86-10, Sutter Instruments) was pulled into a recording electrode with a micropipette puller (P97, Sutter Instruments). The micromanipulator (MP285, Sutter Instruments) was manipulated under an inverted microscope (IX71, Olympus) to make the recording electrode contact the cell and negative pressure suction was applied to achieve a GΩ seal. Then, rapid capacitance compensation was performed, and negative pressure was continued, cell membrane was broken by suction, and whole cell recording mode was formed. Then the slow capacitance was compensated and the membrane capacitance and series resistance were recorded without leakage compensation. When the whole cell recording current was stabilized, dosing was initiated, and the next concentration was detected after acting 5 minutes of each drug concentration. Multiple cells were independently repeated during the recording period. All electrophysiological assays were performed at room temperature. Specifically, 6 concentrations (measuring $IC_{50}$) or 2 concentrations (preliminary screening) were set for each compound, and the percentage of inhibition of sodium channels was determined by calculating the relative percentage of peak current generated before and after each concentration compound treated cells, and the $IC_{50}$ value or percentage of inhibition at a specific concentration was calculated using IGOR pro software.

The voltage stimulation protocol for recording hNav1.8 sodium channel current using whole cell patch clamp is as follows: the membrane potential is clamped at -120 mV, the command voltage starts from -130 mV, and is maintained in steps of 10 mV for 8 seconds, then depolarized to 0 mV (or current size is 0 pA), maintained for 30 ms, and the half inactivation voltage is measured. After the formation of whole cell sealing, the cell membrane voltage is clamped at -120 mV, and the clamp voltage is depolarized to 0 mV for 50 ms, then the voltage is restored to the measured half inactivation voltage for 8 s. The cell membrane potential then recovers to -120 mV for 20 ms, then depolarized to 0 mV for 50 ms, and finally restored to the clamp voltage of -120 mV for 30 ms. The data were collected repeatedly every 20 s to observe the effect of the drug on the peak current of hNav1.8 sodium channel.

The inhibitory activity (resting state) of some examples on hNav1.8 was calculated as shown in Table 2 below.

Table 2 Inhibitory activity of the examples against hNav1.8

| No. | $IC_{50}$ (nM) |
|---|---|
| Example 01 | > 100 |
| Example 02 | 5.3 |
| Example 03 | > 10 |
| Example 04 | 66.7 |
| Example 05 | 8.9 |
| Example 06 | 3.1 |
| Example 07 | 14.7 |
| Example 08 | 3.3 |
| Example 09 | 1 |
| Example 10 | 0.784 |
| Example 14 | 0.34 |
| Example 16 | 0.084 |
| Example 17 | 0.095 |
| Example 21 | 0.36 |

(continued)

| No. | $IC_{50}$ (nM) |
|---|---|
| Example 32 | 16.0 |

Table 3 Inhibition rates of hNav1.8 for example compounds

| No. | 10 nM | 100 nM |
|---|---|---|
| Example 01 | 0.36% | 41.79% |
| Example 02 | 66.12% | 96.07% |
| Example 03 | 19.72% | 70.03% |
| Example 04 | 3.33% | 45.95% |
| Example 05 | 46.68% | 90.46% |
| Example 06 | 77.57% | NT |
| Example 07 | 48.30% | NT |
| Example 08 | 75.39% | 96.61% |
| Example 09 | 91.28% | NT |
| Example 10 | 95.46% | 99.08% |
| Example 11 | 15.27% | 72.75% |
| Example 12 | 95.79% | 99.96% |
| Example 13 | 95.64% | 99.65% |
| Example 14 | 98.02% | 99.63% |
| Example 15 | 15.38% | 49.93% |
| Example 16 | 99.83% | 99.98% |
| Example 17 | 98.28% | 100% |
| Example 18 | - 3.14% | 21.54% |
| Example 19 | 0.44% | 16.56% |
| Example 20 | 19.48% | 81.11% |
| Example 22 | / | 90.7% |
| Example 23 | / | 97.18% |
| Example 24 | / | 50.96% |
| Example 25 | / | 91.55% |
| Example 26 | / | 91.49% |
| Example 27 | / | 97.02% |
| Example 28 | / | 94.24% |
| Example 29 | / | 83.67% |
| Example 30 | / | 94.00% |
| Example 31 | / | 97.07% |
| Example 32 | 38.12% | 84.71% |

Method 2:

Human Nav1.8 ion channel is stably expressed on HEK293 cells. After the Nav1.8 current is stabilized, the effect of the compounds on Nav1.8 ion channel can be obtained by comparing the NaV1.8 current before and after the compounds are applied.

1) Formulation

Reagents except NaOH and KOH which are used for acid-base titration, are all purchased from Sigma (St. Louis, MO) Company. The test compounds were accurately weighed and then prepared into a solution in DMSO with a concentration of 9 mM. The final concentrations were all prepared on the day of the assay and then dissolved in the extracellular solution. Extracellular solution (mM) was: NaCl, 137; KCl, 4; CaCl2, 1.8; MgCl2, 1; HEPES, 10; glucose 10; pH 7.4 (NaOH titration). All test compounds and control compound solutions contained 1μM TTX.

2) Assay method

All assays were conducted at room temperature, with a control of each cell itself . The compounds were all irrigated by a perfusion system that utilized its own gravity. At least two cells were tested for each concentration. After the current had been stabilized (or 5 min), the strength of the current change before and after applying compounds were compared and used to calculate the blocking effect of the compound.

a) Assay instruments

Patch clamp amplifier: Patch Clamp PC-505B(WARNER instruments)/MultiClamp 700A (Axon instrument)

Digital-to-analog Converter: Digidata 1440A (Axon CNS)/Digidata 1550A (Axon instruments)

Micromanipulator: MP-225 (SUTTER instrument)

Inverted microscope: TL4 (Olympus)

Glass micropipette puller: PC-10 (NARISHIGE)

Microelectrode glass capillary: B12024F (Wuhan Microtanyi Scientific Instrument Co., LTD.)

b) Electrophysiology

The cells were transferred to a perfusion tank for perfusion with extracellular fluid. Intracellular fluid (mM): Aspartic acid, 140; $MgCl_2$, 2; EGTA 11; HEPES, 10; pH 7.2 (CsOH titration). Intracellular solution was stored at -80 °C refrigerator in small batches and melt on test day. The electrodes were drawn with PC-10 (Narishige, Japan). During the whole-cell patch clamp recording, noise was filtered at one-fifth of the sampling frequency.

c) Assay protocol (resting state)

Clamped the cell at -80 mV and then depolarized to 10mV with a square wave lasting 10 ms to get the Nav1.8 current (see Figure 1 attached). This procedure was repeated every 5 seconds. The maximum current triggered by the square wave was detected, and when it stabilized, the test compounds were perfused, and when the reaction stabilized, the strength of the block was calculated.

The inhibitory activity of some examples on hNav1.8 was calculated as shown in Table 4 below.

Table 4. The inhibitory activity of the examples against hNav1.8

| No. | $IC_{50}$ (nM) |
|---|---|
| Example 02 | 2.82 |
| Example 05 | 21.3 |

(continued)

| No. | $IC_{50}$ (nM) |
|---|---|
| Example 08 | 1.74 |
| Example 09 | 0.918 |
| Example 10 | 0.46 |
| Example 14 | 0.725 |
| Example 16 | 0.071 |
| Example 23 | 1.5 |
| Example 24 | 97.11 |
| Example 25 | 9.23 |
| Example 26 | 8 |
| Example 28 | 11.42 |
| Example 30 | 9.5 |
| Example 32 | 35.1 |

**Example 34: Assay of kinetic solubility**

1) Preparation of stock solutions

The stock solutions of the test compound and the standard progesterone were prepared in DMSO at the concentration of 10 mM.

2) Assay procedures for solubility

30μL of stock solutions at 10 mM of the test compounds and standard substance were placed in order into their proper 96-well plate. 970 μL phosphate buffer at the specified pH was added to the proper vial of the sample plate. The assay was performed in duplicate. A stirring stick was added to each vial and the teflon/silicone plug was capped. The sample tray was then placed in the Eppendorf Thermomixer Comfort and shaken at 1100 RPM at 25 °C for 2 hours. After 2 hours, plugs were removed, a big magnet was used to remove the stirring sticks, and samples were transferred from the sample plate to the filter plate. Vacuum pump was used to create negative pressure and filtered the samples. Transferred 5 μL of the filtrate to the new sample plate, then added 5 μL DMSO and 490 μL water/acetonitrile (V/V=1/1) to dilute the solvent. The dilution factor may be adjusted according to the solubility values of the substances to be tested or the strength of its LC-MS signal response.

3) Preparation of standard solution

From the 10mM DMSO stock solution, 15μL of that was transferred into the empty plate and 485μL DMSO was added to prepare a 300μM standard solution. From the 300 μM standard solution plate, 5 μL of that was transferred to another empty plate and 5 μL phosphate buffer at specified pH and 490 μL water/acetonitrile (V/V=1/1) dilution solvent were added to the standard solution to get a final concentration of 3 μM. The concentration of the standard solution may be adjusted according to the strength of its LC-MS signal response.

4) Sample analysis steps

The sample plate was placed into the sample tray of the automatic sampler and the samples were evaluated by LC-MS/MS analysis.

5) Data analysis

All calculations were carried out by Microsoft Excel.
The sample filtrate was analyzed and quantified against a standard of known concentrations using LC -MS

spectral peaks to identify and quantify. The solubility values of the control drug and the compounds to be tested were calculated as the following formula:

[Sample]: concentration of the sample
$AREA_{Sample}$: peak area of the sample
INJ $VOL_{Std}$: standard sample injection volume
$DF_{Sample}$: sample dilution factor
[STD]: standard substance concentration
$AREA_{Std}$: peak area of standard substance
INJ $VOL_{Sample}$: sample injection volume

Table 5. Kinetic solubility of example compounds (unit: μM) (compounds with comparable activity)

| No. | pH3.5 |
|---|---|
| Example 02 | 2.80 |
| Example 05 | 0.39 |
| Example 06 | 0.39 |
| Example 07 | 52.7 |
| Example 08 | 175.0 |
| Example 09 | 157.21 |
| Example 13 | 3.38 |
| Example 14 | 270.67 |
| Example 24 | 9.88 |
| Example 25 | 87.93 |
| Example 26 | 127 |
| Example 27 | 78.8 |
| Example 28 | 131 |
| Example 29 | < 1.56 |
| Example 30 | < 0.18 |
| Example 31 | 0.69 |
| Example 32 | < 1.56 |

**Example 35: Stability assay method for human liver microsomes in vitro**

1. Materials

Human liver microsomes, purchased from Corning or Xenotech, stored in a -80°C refrigerator. Nicotinamide adenine dinucleotide phosphate (NADPH), Chem-impex international, 00616 Control compounds: testosterone, diclofenac, propafenone

2. Instruments

Water bath (HH-60, Guohua, Changzhou, China)
Pipette workstation (PP-550DS, Apricot, Covina, USA)
Centrifuge (5810R, Eppendorf, Hamburg, Germany)
Mass Spectrometer (Sciex API 4000, Singapore)

High Performance Liquid Chromatography (LC 20-AD, Shimadzu, Kyoto, Japan)
Sampler (ADDA, Apricot Designs, California, USA)

3. Assay procedures

3.1 Preparation of working solution

Stock solution: 10mM in DMSO

Working solution concentration preparation: 10mM stock solution was diluted to 100μM with 100% acetonitrile (organic phase content: 99%ACN, 1%DMSO)

3.2 Assay Procedure

Two 96-well incubation plates were named T60 incubation plate and NCF60 incubation plate.

445μL microsomal working solution (liver microsomal protein concentration: 0.56 mg/mL) was added to the T60 and NCF60 incubation plates respectively, then the incubation plates above were placed in a water bath at 37°C for pre-incubation approximately 10 minutes.

After the pre-incubation finished, 5μL of the test compound working solution was added to the T60 and NCF60 incubation plates respectively, and mixed well. 50 μL potassium phosphate buffer was added to each well on the NCF60 incubation plate to start the reaction. 180 μL termination solution (acetonitrile solution containing 200 ng/mL tolbutamide and 200 ng/mL labetalol) and 6 uL working solution of NADPH regeneration system were added to the T0 termination plate, and 54 μL samples were removed from the T60 incubation plate to the T0 termination plate (T0 sample generated). The reaction was initiated by adding 44 μL of NADPH regenerating system working solution per well on the T60 incubation plate. Only 54 μL microsomal working solution, 6 uL NADPH regeneration system working solution, and 180 μL termination solution were added to the Blank plate. Therefore, in the samples of the test or control compounds, the final reaction concentration of the compounds, testosterone, diclofenac and propafenone was 1 μM, the concentration of the liver microsomes was 0.5 mg/mL, and the final concentration of DMSO and acetonitrile in the reaction system was 0.01% (v/v) and 0.99% (v/v), respectively.

After incubation for an appropriate time (e.g. 5, 10, 20, 30, and 60 minutes), 180 μL of termination solution (acetonitrile solution containing 200 ng/mL tolbutamide and 200 ng/mL labetalol) was added to the sample hole of each termination plate, respectively. After that, 60 μL sample was removed from the T60 incubation plate to terminate the reaction.

All sample plates were shaken evenly and centrifuged at 3220 × g for 20 min. Then 80 μL supernatant was taken from each well and diluted to 240 μL pure water for LC-MS/MS analysis.

4. Liquid chromatographic tandem mass spectrometry analysis

All samples were injected for analysis. The liver microsomal stability results were shown in Table 6.

Table 6 Human liver microsome stability ($T_{1/2}$) test results are as follows:

| No. | $T_{1/2}$ (min) |
|---|---|
| Example 1 | > 145 |
| Example 2 | 58.8 |
| Example 3 | 79.3 |
| Example 4 | 79.9 |
| Example 5 | 93.7 |
| Example 6 | > 145 |
| Example 7 | 63.9 |
| Example 8 | 23 |

(continued)

| No. | $T_{1/2}$ (min) |
|---|---|
| Example 9 | 22.3 |
| Example 10 | 92.7 |
| Example 14 | > 145 |
| Example 16 | 41.3 |
| Example 17 | > 145 |
| Example 21 | 56.6 |
| Example 24 | > 145 |
| Example 26 | 70 |
| Example 27 | 135.7 |

**Example 36 Efficacy test in postoperative pain model in rats**

Experimental Animals

Sprague-Dawley male rats, purchased from Zhejiang Vital River Laboratory Animal Technology Co., LTD Sample solution preparation

Compounds of the example 10, 14, 16, 31 of the invention were weighted and prepared into solutions of 3 mg/mL using 5% DMSO (Anhui Senrise Technologies Co.,Ltd)+30% Solutol HS15 (Beijing Ouhe Technology Co., LTD.)+65% water for injection, and administered at a volume of 10 mL/kg by gavage. The dosage was 30 mg/kg.

Experimental procedure

Animals were anesthetized with Zoletil 50+ Xylazine Hydrochloride (20 mg/kg+8 mg/kg, intraperitoneally injected). The surgical area on the plantar skin of the left hind paw was disinfected three times with iodophor and 70% ethanol, and the surgery was started after the skin was dry.

A longitudinal incision about 1cm was made towards the toes starting from 0.5cm away from the heel of the left hind paw. After the skin was incised, the flexor digitorum brevis was raised and longitudinal blunt injury was caused. After hemostasis by pressing, the wound was sutured. After surgery, animals were placed on an electric warm pad and 5 mL of physiological saline was injected subcutaneously to prevent dehydration. 24 h after modeling, all animals (including the normal control group) were tested for base mechanical allodynia (PWT). The modeling animals without mechanical hypersensitivity (PWT greater than 5 g) were excluded and 50 animals were selected and randomly divided into 5 groups, with 9 animals in each group except 5 in the normal group (negative control group).

The animals were weighed and administered orally at a volume of 10 mL/kg. Except normal control animals, other animals were given a single dose of compounds, and mechanical allodynia was performed by test fibers on all groups of animals at 1 h, 2 h and 4 h after dosing. The rats were placed individually in a plexiglass box with a grid at the bottom to ensure that the paws of the rats could be tested. The rats would acclimate for 15 minutes before the test. After the acclimation was completed, the test fibers were used to test on the plantar center of the rat's left hind paw. Pressed the test fiber vertically against the skin and applied force to bend the fiber for 6 to 8 seconds, with 5 second intervals between each test. The rapid withdrawal of the animal's paws during the test was recorded as a pain response. The animal's paw withdrawal when the fibers left the animal's skin was also recorded as a pain response. If the animal moves or walks around, it's not recorded as a pain response, and the test should be repeated.

Method of calculation and data processing

Mechanical hyperalgesia was expressed as the paw withdrawal threshold (PWT) in the rat behavioral test, which was calculated according to the following formula:

$$50\%\ \text{response threshold (g)} = (10^{(X_f + k\delta)})/10{,}000$$

*Xf* = final test fiber value used in the test

k = table value (Chaplan et al. 1994, page 62)

δ = mean deviation

Data were collected with excel software and analyzed with Prism 7.0 (Graph pad software, Inc.) software by two-way ANOVA followed by Dunnett multiple comparison test.

RESULTS

The result is shown in Figure 2. In this experiment, example compound 31 inhibited mechanical hyperalgesia induced by postoperative pain models in rats at 2 h and 4 h after a single oral administration of 30 mg/kg. Example compound 10, 14, 16 inhibited mechanical hyperalgesia induced by postoperative pain models in rats at 1 h, 2 h, and 4 h after a single oral administration of 30 mg/kg. The efficacy of compound 14 was significantly stronger than that of example compound 31 at 1 h and 4 h after administration; and example compound 16 was significantly more effective than example compound 31 after 1 h of administration.
Note: *$p<0.05$, **$p<0.01$, ***$p<0.001$ *compared with normal group;* # $p<0.05$, ### $p<0.001$ compared with example compound 31.

**Claims**

1. A compound of formula (I) or a pharmaceutically acceptable salt thereof:

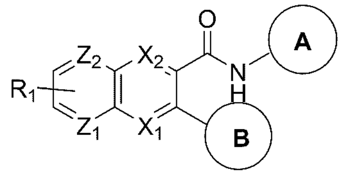

Formula I

wherein:

$X_1$, $X_2$, $Z_1$, $Z_2$ are independently selected from substituted or unsubstituted C or N;
$R_1$ is independently selected from hydrogen, halogen, $-NH_2$, -CN, OH, $-C_1$-$C_6$ alkyl, $-C_1$-$C_6$ haloalkyl, $-C_1$-$C_6$ alkoxy, $-C_1$-$C_6$ haloalkoxy, $-C_1$-$C_6$ alkylamino, $-SO_2R_2$, $-S(O)(NH)R_2$, $-COR_2$, $-CONR_2R_3$; wherein $R_2$ and $R_3$ are independently selected from hydrogen, $-NH_2$, and $-C_1$-$C_3$ alkyl;
ring A is substituted or unsubstituted benzene ring or six-membered heteroaryl;
ring B is the substituted or unsubstituted three- to ten- membered aliphatic ring or aliphatic heterocyclic ring.

2. The compound or the pharmaceutically acceptable salt of claim 1, wherein at least one of $X_1$ and $X_2$ is N.

3. The compound or the pharmaceutically acceptable salt of claim 1, wherein ring A is selected from a six-membered aryl or heteroaryl containing 0-3 nitrogen atoms, wherein the aryl or heteroaryl is optionally substituted by hydrogen, halogen, $-NH_2$, -CN, -OH, $C_1$-$C_6$ alkyl, carbonyl, $-C_1$-$C_6$ haloalkyl, $-C_1$-$C_6$ alkoxy, $-C_1$-$C_6$ haloalkoxy, $-C_1$-$C_6$ alkylamino, $-SO_2R_2$, $-S(O)(NH)R_2$, $-COR_2$, $-CONR_2R_3$ or $-POR_2R_3$; wherein $R_1$ and $R_2$ are independently selected from hydrogen, $-NH_2$, $-NHCH_3$, and $-C_1$-$C_3$ alkyl.

4. The compound or the pharmaceutically acceptable salt of claim 1, wherein ring B is selected from a 3 to 10 membered aliphatic ring or aliphatic heterocyclic ring with 0-3 heteroatoms which are selected from N, O, and S, and optionally, the aliphatic ring or aliphatic heterocyclic ring is substituted by a halogen, carbonyl group, $-NH_2$, -CN, -OH, $-C_1$-$C_6$ haloalkyl, $-C_1$-$C_6$ alkoxy, $-C_1$-$C_6$ haloalkoxy, $-C_1$-$C_6$ alkylamino, $-C_3$-$C_6$ cycloalkyl or $-C_3$-$C_6$ cycloheteroalkyl.

5. The compound or the pharmaceutically acceptable salt of claim 1, wherein the compound is described as Formula II:

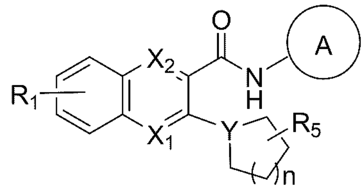

Formula II

wherein $X_1$, $X_2$ are independently selected from C or N, and at least $X_1$ is N;
$R_1$ is independently selected from hydrogen, halogen, $-NH_2$, -CN, -OH, $-C_1-C_6$ alkyl, $-C_1-C_6$ haloalkyl, $-C_1-C_6$ alkoxy, $-C_1-C_6$ haloalkoxy, $-C_1-C_6$ alkylamino, $-SO_2R_2$, $-S(O)(NH)R_2$, $-COR_2$, $-CONR_2R_3$; wherein $R_2$ and $R_3$ are independently selected from hydrogen, $-NH_2$, and $-C_1-C_3$ alkyl;
Y is selected from CH or N;
$R_5$ is selected from hydrogen, halogen, $-NH_2$, -CN, OH, $-C_1-C_6$ alkyl, $-C_1-C_6$ haloalkyl, $-C_1-C_6$ alkoxy, $-C_1-C_6$ haloalkoxy, $-C_1-C_6$ alkylamino;
n is an integer from 0 to 6;
ring A is selected from a six-membered aryl or heteroaryl containing 0-3 nitrogen atoms, wherein the aryl or heteroaryl is optionally substituted by hydrogen, halogen, $-NH_2$, -CN, -OH, $-C_1-C_6$ alkyl, carbonyl, $-C_1-C_6$ haloalkyl, $-C_1-C_6$ alkoxy, $-C_1-C_6$ haloalkoxy, $-C_1-C_6$ alkylamino, $-SO_2R_2$, $-S(O)(NH)R_2$, $-COR_2$, $-CONR_2R_3$ or $-POR_2R_3$; wherein $R_2$ and $R_3$ are independently selected from hydrogen, $-NH_2$, $-NHCH_3$, and $-C_1-C_3$ alkyl.

**6.** The compound or the pharmaceutically acceptable salt of claim 5, wherein the ring A is

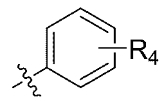

or

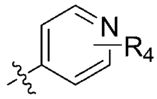

;

wherein $R_4$ is hydrogen, halogen, $-NH_2$, -CN, OH, $-C_1-C_6$ alkyl, $-C_1-C_6$ haloalkyl, $-C_1-C_6$ alkoxy, $-C_1-C_6$ haloalkoxy, $-C_1-C_6$ alkylamino, $-SO_2R_2$, $-S(O)(NH)R_2$, $-COR_2$, $-CONR_2R_3$, or $-POR_2R_3$;
$R_2$ and $R_3$ are independently selected from hydrogen, $-NH_2$, $-NHCH_3$, $-C_1-C_3$ alkyl, or $R_2$ and $R_3$ together with phosphorus form a three- to eight- membered ring.

**7.** The compound or the pharmaceutically acceptable salt of claim 1, wherein the compound is described as Formula III:

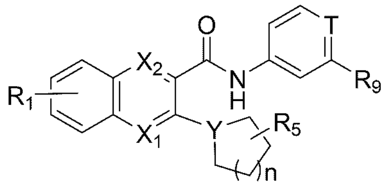

Formula III

wherein $X_1$ and $X_2$ are independently selected from C or N, and at least $X_1$ is N;
$R_1$ is independently selected from hydrogen, halogen, $-NH_2$, -CN, -OH, $-C_1-C_6$ alkyl, $-C_1-C_6$ haloalkyl, $-C_1-C_6$ alkoxy, $-C_1-C_6$ haloalkoxy, $-C_1-C_6$ alkylamino, $-SO_2R_2$, $-S(O)(NH)R_2$, $-COR_2$, $-CONR_2R_3$; wherein $R_2$ and $R_3$ are independently selected from hydrogen, $-NH_2$, and -Ci-Cs alkyl;

T is $CR_6$ or N;
$R_6$ is hydrogen, halogen or $-C_1-C_6$ alkyl;
$R_9$ is hydrogen, halogen, $-NH_2$, -CN, OH, $-C_1-C_6$ alkyl, $-C_1-C_6$ haloalkyl, $-C_1-C_6$ alkoxy, $-C_1-C_6$ haloalkoxy, $-C_1-C_6$ alkylamino, $-SO_2R_2$, $-S(O)(NH)R_2$, $-COR_2$, $-CONR_2R_3$, or $-POR_2R_3$;
$R_2$ and $R_3$ are independently selected from hydrogen, $-NH_2$, $-NHCH_3$, $-C_1-C_3$ alkyl, or $R_2$ and $R_3$ together with phosphorus form a three- to eight-membered ring;
$R_5$ is selected from hydrogen, halogen, $-NH_2$, -CN, -OH, $-C_1-C_6$ alkyl, $-C_1-C_6$ haloalkyl, $-C_1-C_6$ alkoxy, $-C_1-C_6$ haloalkoxy, $-C_1-C_6$ alkylamino;
n is an integer from 0 to 6.

8. The compound or the pharmaceutically acceptable salt of claim 7, wherein Y is a nitrogen and n is an integer from 1 to 4.

9. The compounds or the pharmaceutically acceptable salt of claim 8, wherein n is 3.

10. The compound or the pharmaceutically acceptable salt of claim 7, wherein the compound is described as Formula IV:

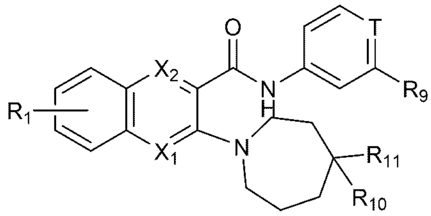

Formula IV

wherein $R_{10}$ and $R_{11}$ are independently selected from hydrogen or halogen;
other substituents are defined as described above.

11. The compound or the pharmaceutically acceptable salt of any of claims 7-10 , wherein $R_1$ is independently selected from hydrogen, halogen, $-C_1-C_6$ alkyl, $-C_1-C_6$ haloalkyl, $-C_1-C_6$ alkoxy or $-C_1-C_6$ haloalkoxy.

12. The compound or the pharmaceutically acceptable salt of claim 11, wherein $R_1$ is independently selected from hydrogen, halogen, $-CH_3$, $-OCH_3$.

13. The compound or the pharmaceutically acceptable salt of claim 12, wherein $R_1$ is independently selected from hydrogen or halogen.

14. The compound or the pharmaceutically acceptable salt of claim 7, wherein $X_1$ is N and $X_2$ is C.

15. The compound or the pharmaceutically acceptable salt of claim 7, wherein $X_1$ is N and $X_2$ is N.

16. The compound or the pharmaceutically acceptable salt of claim 7, wherein $R_6$ is hydrogen or halogen.

17. The compound or the pharmaceutically acceptable salt of claim 7, wherein $R_9$ is $-SO_2R_2$, $-S(O)(NH)R_2$, $-COR_2$ or $-CONR_2R_3$, wherein $R_2$ and $R_3$ are independently selected from hydrogen, $-NH_2$, $-NHCH_3$, and $-C_1-C_3$ alkyl.

18. The compound or the pharmaceutically acceptable salt of claim 7, wherein the compound is described as Formula V:

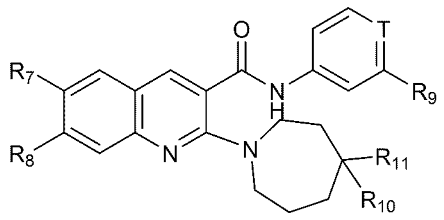

Formula V

wherein $R_7$, $R_8$ are independently selected from hydrogen, halogen, $-CH_3$, $-OCH_3$;
$R_9$ is selected from $-SO_2R_2$, $-S(O)(NH)R_2$, $-COR_2$, $-CONR_2R_3$;
$R_2$ and $R_3$ are independently selected from hydrogen, $-NH_2$, $-NHCH_3$, $-C_1$-$C_3$ alkyl;
T is $CR_6$ or N;
$R_6$ is hydrogen, halogen or $-C_{1-6}$ alkyl;
$R_{10}$ and $R_{11}$ are independently selected from hydrogen or halogen.

**19.** The compound or the pharmaceutically acceptable salt of claim 18, wherein $R_9$ is selected from $-SO_2NH_2$, $-CONH_2$.

**20.** The compound or the pharmaceutically acceptable salt of claim 19, wherein $R_9$ is $-SO_2NH_2$.

**21.** The compound or the pharmaceutically acceptable salt of claim 18, wherein $R_7$ and $R_8$ are independently selected from hydrogen or halogen.

**22.** The compound or the pharmaceutically acceptable salt of claim 18, wherein $R_6$ is H.

**23.** The compound or the pharmaceutically acceptable salt of claim 18, wherein $R_{10}$ and $R_{11}$ are halogen.

**24.** The compound or the pharmaceutically acceptable salt of any of claims 1-23 is selected from:

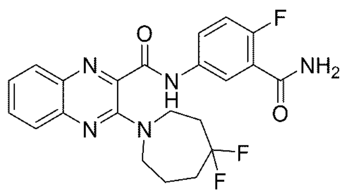 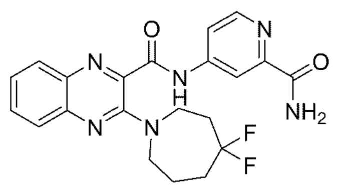 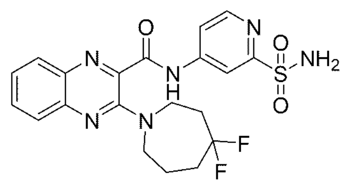

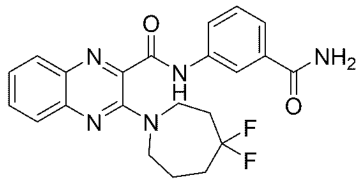 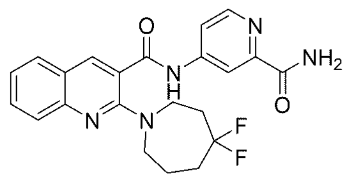 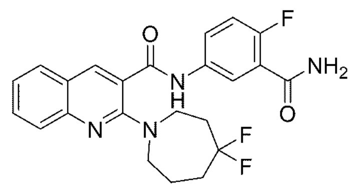

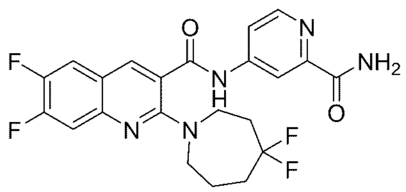 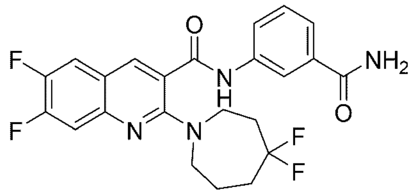 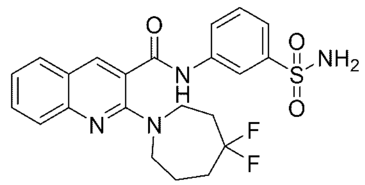

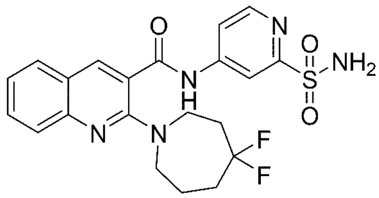 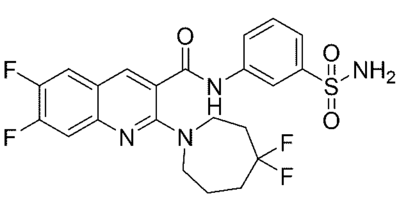 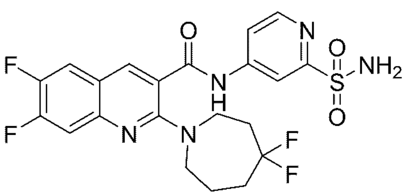

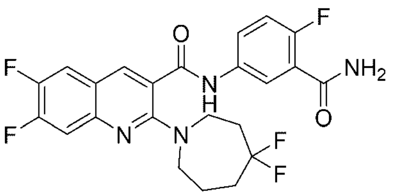 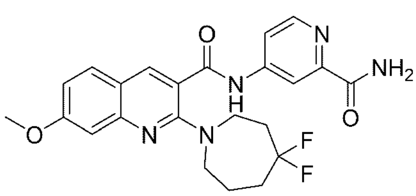 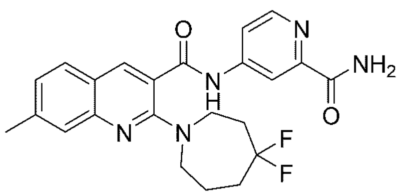

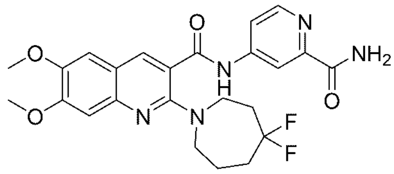 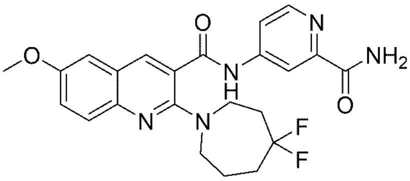 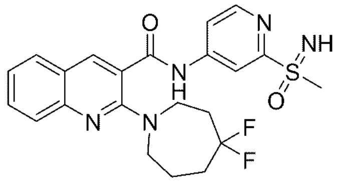

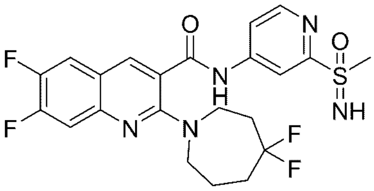 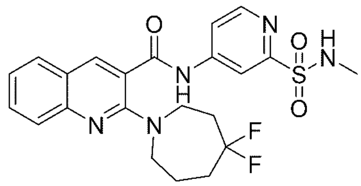 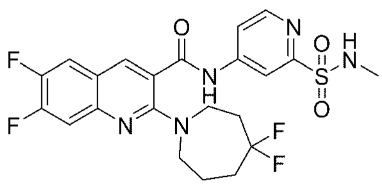

.

**25.** The compound or the pharmaceutically acceptable salt of claim 24 is selected from:

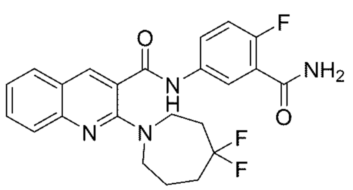 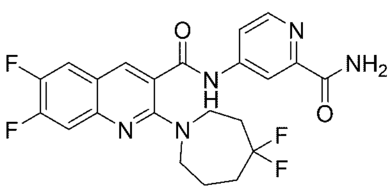 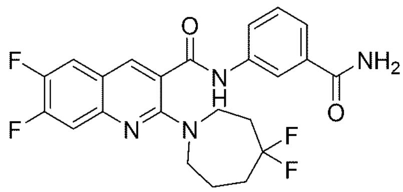

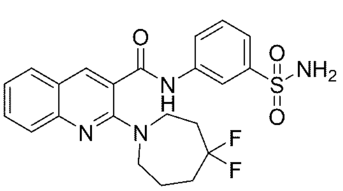 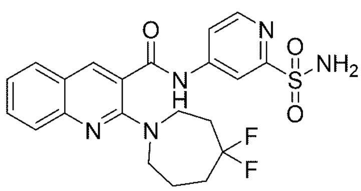 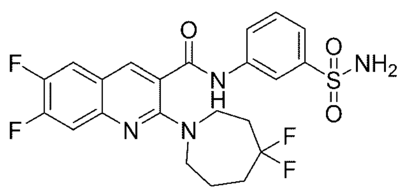

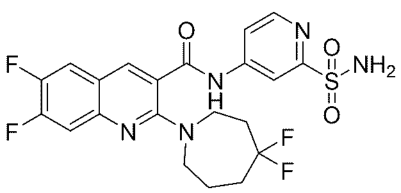 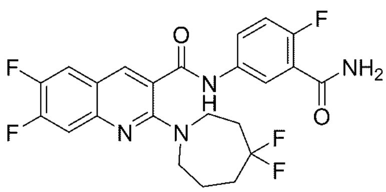 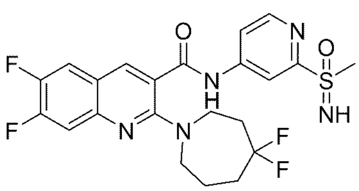

.

**26.** A pharmaceutical composition is **characterized by** comprising the compound or the pharmaceutically acceptable

salt of any of claims 1-25, and pharmaceutically acceptable excipients.

**27.** A use of the compound or the pharmaceutically acceptable salt of any one of claims 1-25 in the preparation of a medicament for treating pain.

**28.** A method for preventing or treating pain, comprising the administration of the compound or the pharmaceutically acceptable salt of any one of claims 1-25 to a patient.

**29.** The use according to claim 28 or the method according to claim 27, wherein the pain is chronic pain, intestinal pain, neuropathic pain, musculoskeletal pain, acute pain, inflammatory pain, cancer pain, primary pain, post-operative pain, visceral pain, multiple sclerosis, Charcot-Marie-Tooth syndrome, incontinence or arrhythmia.

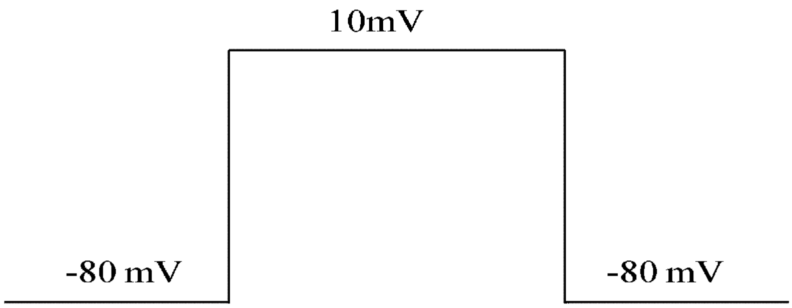
10mV
-80 mV
-80 mV

Fig. 1

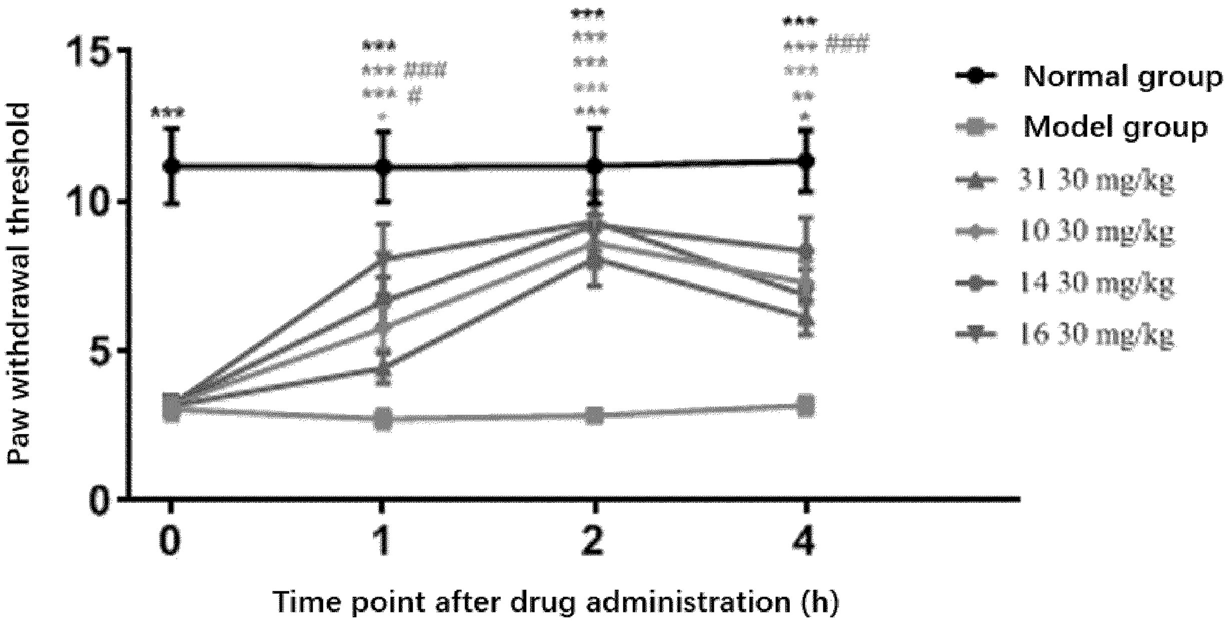
Paw withdrawal threshold
Time point after drug administration (h)
Normal group
Model group
31 30 mg/kg
10 30 mg/kg
14 30 mg/kg
16 30 mg/kg

Fig. 2

**INTERNATIONAL SEARCH REPORT**

International application No.
**PCT/CN2023/072791**

**A. CLASSIFICATION OF SUBJECT MATTER**

C07D401/04(2006.01)i;C07D401/12(2006.01)i;C07D401/14(2006.01)i;C07D471/00(2006.01)i;A61K31/47(2006.01)i;A61K31/498(2006.01)i;A61P25/04(2006.01)i;A61P25/06(2006.01)i;A61P29/00(2006.01)i

According to International Patent Classification (IPC) or to both national classification and IPC

**B. FIELDS SEARCHED**

Minimum documentation searched (classification system followed by classification symbols)

C07D 401/-, C07D 471/-, A61K 31/-, A61P 25/-, A61P 29/-

Documentation searched other than minimum documentation to the extent that such documents are included in the fields searched

Electronic data base consulted during the international search (name of data base and, where practicable, search terms used)

CNTXT, WPABS, CNKI, ISI Web of Knowledge, STN (CAPLUS, REGISTRY, MARPAT): 成都康弘药业集团股份有限公司, 刘婷, 方群, 吴帆, 柯尊洪, 钠离子通道, 疼痛, sodium w channel, Nav, pain, 结构式检索, structural formula search

**C. DOCUMENTS CONSIDERED TO BE RELEVANT**

| Category* | Citation of document, with indication, where appropriate, of the relevant passages | Relevant to claim No. |
|---|---|---|
| A | CN 105073738 A (VERTEX PHARMACEUTICALS INCORPORATED) 18 November 2015 (2015-11-18)<br>description, paragraphs [0938]-[0950], [0977]-[0978], [0981] | 1-29 |
| A | WO 2010056865 A1 (GILEAD PALO ALTO, INC. et al.) 20 May 2010 (2010-05-20)<br>description, paragraphs [0005]-[0006], [0008], [0107] | 1-29 |
| A | CN 106458985 A (ABBVIE INC.) 22 February 2017 (2017-02-22)<br>description, paragraphs [0010]-[0012], [0087] | 1-29 |
| A | WO 2008113006 A1 (XENON PHARMACEUTICALS INC. et al.) 18 September 2008 (2008-09-18)<br>description page 8 line 22 to page 11 line 18, pages 84-86 table 2 | 1-29 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **10.05月2023（10.05.2023）** | **16 May 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

## INTERNATIONAL SEARCH REPORT

International application No.
**PCT/CN2023/072791**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **28-29**
because they relate to subject matter not required to be searched by this Authority, namely:

Claims 28-29 relate to a method for treating and/or preventing diseases. The present search report is provided on the basis of the use of a compound in the preparation of a drug for treating diseases.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**INTERNATIONAL SEARCH REPORT**
**Information on patent family members**

International application No.
**PCT/CN2023/072791**

| Patent document cited in search report | Publication date (day/month/year) | Patent family member(s) | Publication date (day/month/year) |
|---|---|---|---|
| CN 105073738 A | 18 November 2015 | TW 201443022 A | 16 November 2014 |
| | | TWI 631105 B | 01 August 2018 |
| | | AR 095192 A1 | 30 September 2015 |
| | | ES 2620379 T3 | 28 June 2017 |
| | | SG 11201505952 WA | 28 August 2015 |
| | | CA 2898650 A1 | 07 August 2014 |
| | | CA 2898650 C | 07 September 2021 |
| | | EP 2951168 A1 | 09 December 2015 |
| | | EP 2951168 B1 | 04 January 2017 |
| | | WO 2014120815 A1 | 07 August 2014 |
| | | WO 2014120815 A9 | 25 June 2015 |
| | | KR 20150114536 A | 12 October 2015 |
| | | KR 102226587 B1 | 11 March 2021 |
| | | BR 112015017997 A2 | 11 July 2017 |
| | | BR 112015017997 A8 | 05 November 2019 |
| | | BR 112015017997 B1 | 07 March 2023 |
| | | HK 1217691 A1 | 20 January 2017 |
| | | RU 2015136770 A | 07 March 2017 |
| | | RU 2683788 C2 | 02 April 2019 |
| | | AU 2014212426 A1 | 06 August 2015 |
| | | AU 2014212426 B2 | 26 April 2018 |
| | | AU 2014212426 B8 | 10 May 2018 |
| | | ZA 201505326 B | 29 November 2017 |
| | | IL 240091 A0 | 24 September 2015 |
| | | IL 240091 B | 31 March 2019 |
| | | MX 2015009592 A | 25 November 2015 |
| | | MX 368833 B | 18 October 2019 |
| | | US 2014228371 A1 | 14 August 2014 |
| | | US 9139529 B2 | 22 September 2015 |
| | | JP 2016506964 A | 07 March 2016 |
| | | JP 6362623 B2 | 25 July 2018 |
| | | US 2015336945 A1 | 26 November 2015 |
| | | US 9783501 B2 | 10 October 2017 |
| | | HUE 032169 T2 | 28 September 2017 |
| | | NZ 710111 A | 28 August 2020 |
| WO 2010056865 A1 | 20 May 2010 | US 2014107155 A1 | 17 April 2014 |
| | | US 8865739 B2 | 21 October 2014 |
| | | US 2013102632 A1 | 25 April 2013 |
| | | US 8664399 B2 | 04 March 2014 |
| | | TW 201029654 A | 16 August 2010 |
| | | AR 074359 A1 | 12 January 2011 |
| | | US 2010125091 A1 | 20 May 2010 |
| CN 106458985 A | 22 February 2017 | JP 2017505327 A | 16 February 2017 |
| | | JP 6532474 B2 | 19 June 2019 |
| | | US 2018334435 A1 | 22 November 2018 |
| | | US 2015218102 A1 | 06 August 2015 |
| | | US 9452986 B2 | 27 September 2016 |
| | | MX 2016010056 A | 15 November 2016 |
| | | KR 20160118345 A | 11 October 2016 |
| | | WO 2015119998 A1 | 13 August 2015 |

Form PCT/ISA/210 (patent family annex) (July 2022)

**INTERNATIONAL SEARCH REPORT**
**Information on patent family members**

International application No.
**PCT/CN2023/072791**

| Patent document cited in search report | Publication date (day/month/year) | Patent family member(s) | Publication date (day/month/year) |
|---|---|---|---|
| | | CA 2938719 A1 | 13 August 2015 |
| | | SG 11201606367 YA | 29 September 2016 |
| | | AU 2015214366 A1 | 18 August 2016 |
| | | AU 2015214366 B2 | 18 April 2019 |
| | | BR 112016017996 A2 | 08 August 2017 |
| | | EP 3102569 A1 | 14 December 2016 |
| | | US 2016355482 A1 | 08 December 2016 |
| | | US 2017226062 A9 | 10 August 2017 |
| | | US 9969693 B2 | 15 May 2018 |
| | | IL 247014 A0 | 29 September 2016 |
| | | HK 1231471 A1 | 22 December 2017 |
| | | RU 2016135637 A | 12 March 2018 |
| | | RU 2016135637 A3 | 27 August 2018 |
| WO 2008113006 A1 | 18 September 2008 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2013114250 A1
- AU 2015224425 A1
- WO 2015008861 A1
- WO 2016149169 A1
- WO 2020138271 A1
- WO 2020092187 A1
- WO 2020014243 A1
- WO 2019014352 A1
- WO 2020151728 A1
- CN 111808019 A


### Non-patent literature cited in the description


- **NORA D. ; VOLKOW, A. ; THOMAS MCLELLAN.** Opioid Abuse in Chronic Pain-Misconceptions and Mitigation Strategies. *N Engl J Med,* 2016, vol. 374 (13), 1253-63
- **SHENG H G ; SHAO J Y ; KIR KLAND S C et al.** Inhibition of human colon Cancer cell growth by selective inhibition of cyclooxygenase-2. *J Chm Invest,* 1997, vol. 99, 2254
- **JANETTE BROHAN ; BASAVANA G. GOUDRA.** The Role of GABA Receptor Agonists in Anesthesia and Sedation. *CNS Drugs,* 2017
- **BENNETT DL ; CLARK AJ ; HUANG J et al.** The Role of Voltage-Gated Sodium Channels in Pain Signaling. *Physiol Rev,* 2019, vol. 99, 1079-1151
- **MARK D. BAKER ; JOHN N.WOOD.** Involvement of Na+ channels in pain pathways. *TRENDS in Pharmacological Sciences,* 2001, vol. 22 (1), 27-31
- **ALAN L GOLDIN.** RESURGENCE OF SODIUM CHANNEL RESEARCH. *Annu. Rev. Physiol.,* 2001, vol. 63, 871-894
- **ALAN L GOLDIN.** RESURGENCE OF SODIUM CHANNEL RESEARCH. *Annu. Rev. Physiol.,* 2001, vol. 63, 871-94
- **LAURA SOLE ; MICHAEL M. TAMKUN.** Trafficking mechanisms underlying Nav channel subcellular localization in neurons. *Channels,* 2020, vol. 14 (1), 1-17
- **MANUEL DE LERA RUIZ ; RICHARD L. KRAUS.** Voltage-Gated Sodium Channels: Structure, Function, Pharmacology and Clinical Indications. *J. Med. Chem.,* 2015, vol. 58 (18), 7093-7118
- **BLAIR NT ; BEAN BP.** Roles of tetrodotoxin (TTX)-sensitive Na+ current, TTX-resistant Na+ current, and Ca2+ current in the action potentials of nociceptive sensory neurons. *J Neurosci,* 2002, vol. 22, 10277-10290
- **MICHAEL F. JARVIS ; PRISCA HONORE et al.** A-803467, a potent and selective Nav1.8 sodium channel blocker, attenuates neuropathic and inflammatory pain in the rat. *PNAS,* 2007, vol. 104 (20), 8520-8525
- **MERT, GUNES Y.** Antinociceptive activities of lidocaine and the nav1.8 blocker a803467 in diabetic rats. *J Am Assoc Lab Anim Sci,* 2012, vol. 51 (5), 579-585
- **PAYNE CE ; BROWN AR ; THEILE JW et al.** A novel selective and orally bioavailable Nav 1.8 channel blocker, PF-01247324, attenuates nociception and sensory neuron excitability. *Br J Pharmacol.,* 2015, vol. 172 (10), 2654-2670